# EUROPEAN PATENT APPLICATION

(11) **EP 2 305 652 A2**
(43) Date of publication of application: **06.04.2011**
(21) Application number: 10178992.3
(22) Date of filing: 06.12.2006
(51) Int. Cl.: C07D 239/95, C07D 239/91, C07D 401/04, C07D 403/04, A61K 31/517, A61P 29/00

(54) **Trisubstituted quinazolinone derivatives as vanilloid antagonists**

(30) Priority: 08.12.2005 GB 0525068
(62) Divisional of application: 06829349.7
(71) Applicant: Novartis AG, 4056 Basel (CH)
(72) Inventor: Bhalay, Gurdip, Horsham, Sussex RH12 5AB (GB); Culshaw, Andrew James, Horsham, Sussex RH12 5AB (GB); Dziadulewicz, Edward Karol, Frimley/Camberley, Surrey GU16 7SR (GB); Brain, Christopher Thomas, Cambridge, MA MA 02139 (US); Ritchie, Timothy John, Stevenage, Hertfordshire SG1 2NY (GB); Beattie, David, Horsham, Sussex RH12 5AB (GB); Bala, Kamlesh Jagdis, Horsham, Sussex RH12 5AB (GB); Brewer, Alice, Horsham, Sussex RH12 5AB (GB); Hughes, Glyn, Horsham, Sussex RH12 5AB (GB)
(74) Representative: von Sprecher, Georg

(57) **Abstract**

The present invention relates to quinazolinone compounds of the formula 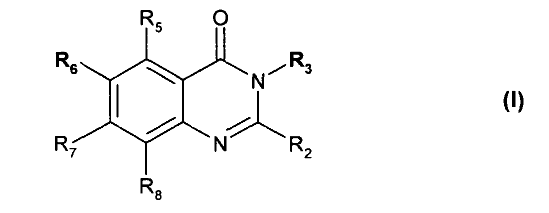 wherein
R₂, R₃, R₅, R₆ R₇ and R₈ are as defined in the specification and in the claims, in free form or in salt form , as vanilloid antagonists.

## Description

The present invention relates to quinazolinone derivatives as vanilloid antagonists, to processes for preparing them, to their use as pharmaceuticals and to pharmaceutical compositions containing them.

In a first aspect, the present invention provides a quinazolinone compound of the formula 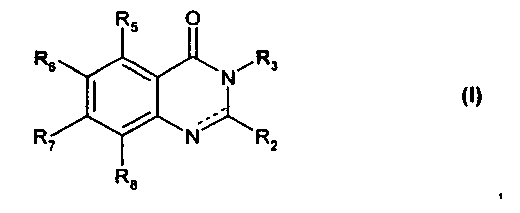 wherein
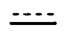 is a single bond or a double bond;
R₂ is selected from
(a) C₁-C₈alkyl, C₃-C₆cycloalkyl, (C₁-C₆alkyl)amino or di-(C₁-C₆alkyl)amino; or
(b) NH₂, hydroxyC₁-C₆alkylamino-, aminoC₁-C₆alkylamino, C₂-C₆alkenyl, di(trifluoromethyl)C₁-C₆alkyl, R₉-O-(C₁-C₆alkyl)- in which the alkyl chain is optionally substituted by trifluoromethyl, (NC)-C, -C₆alkyl-, (R₁₀R₁₁N-)C₁ - C₆alkyl-, (C₁-C₆alkyl)-SO₂-(C₁-C₆alkyl)-, wherein R₉, R₁₀ and R₁₁ are each independently H or C₁-C₆ alkyl; phenyl optionally substituted by one, two or three substituents each independently selected from the group consisting of halogen, C₁-C₆alkyl, halogen-substituted C₁-C₆alkyl, hydroxy C₁-C₆alkyl, cyano or a group -(C=O)-R₂ₐ, where R₂ₐ is C₁-C₆alkyl; or 5, 6, or 7-membered, saturated or unsaturated, heterocyclic ring, directly attached to the quinazolinone ring or attached through -C₁-C₆ alkyl-, containing one, two, or three heteroatoms selected from N, O and S, and optionally substituted with one, two or three substitutents selected from C₁-C₆alkyl, C₁-C₆alkoxy, hydroxy, cyano, halo, R₁₀R₁₁N-, R₉-O-(C=O)-, -(C=O)-N-R₁₀R₁₁, =O and phenyl ;
R₃ is selected from
(a'):
phenyl substituted by one, two or three substituents each independently selected from the group consisting of halogen, C₁-C₆alkyl, halogen-substituted C₁-C₆alkyl, hydroxyC₁-C₆alkyl, cyano or a group -C(=O)-R₃ₐ, where R₃ₐ is C₁-C₆alkyl; or
(b'):
C₁-C₆alkyl, (NC)-C₁-C₆alkyl-, Rg-O-(C₁-C₆alkyl)-, R₉-O-(C₁-C₆alkyl)-O-(C₁-Csalkyl-,R₁₀R₁₁N-(C₁-C₆Csalkyl)-, R₁₀R₁₁N-(C=O)-(C₁-C₆alkyl)-, or (C₁-C₆alkyl)-SO₂-(C₁-C₆alky)-, wherein R₉, R₁₀ and R₁₁ are each independently H or C₁-C₆ alkyl; or
unsubstituted phenyl, phenyl substituted with one or two substituents selected from -(C₁-C₆alkoxy)-, R₁₀R₁₁N-, R₁₀R₁₁N-(C₁-C₆alkyl)-, -SO₂-(C₁-C₆-Cealkyl), R₉-O-(C=O)-, wherein R₉, R₁₀ and R₁₁ are as defined above, or with halo-substituted phenyl or a 5- or 6-membered saturated or unsaturated heterocyclic ring having one, two or three heteroatoms selected from N, O and S and optionally including a further substituent selected from halo, or phenyl substituted with three or four substituents selected from halo, hydroxyl, and C₁-C₆alkyl; or
a cycloalkyl ring having 3, 4, 5 or 6 carbon atoms, directly attached to the quinazolinone ring or attached through -C₁-C₆alkyl-, and which is optionally substituted with one or two substituents selected from C₁-C₆alkyl, C₁-C₆alkoxy, hydroxy, cyano, halo, R₁₀R₁₁N-, R₉-O-(C=O)-, -(C=O)-N-R₁₀R₁₁, and phenyl; or
benzyl, or phenyl(C₁-C₆alkyl)-, phenoxy-(C₁-C₆alkyl)- or phenyl(C=O)-(C₁-C₆alkyl)-, optionally substituted with one, two, or three substituents selected from C₁-C₆alkyl, C₁-C₆alkoxy, hydroxy, cyano, halo, R₁₀R,₁₁N-, R₉-O-(C=O)-, -(C=O)-N-R₁₀R₁₁, and phenyl; or
a 5, 6, or 7- membered, saturated or unsaturated, heterocyclic ring, directly attached to the quinazolinone ring or attached through -C₁-C₆ alkyl-, containing one, two, or three heteroatoms selected from N, O and S, and optionally substituted with one, two or three substitutents selected from C₁-C₆alkyl, C₁-C₆alkoxy, hydroxy, cyano, halo, R₁₀R₁₁N-, R₉-O-(C=O)-, -(C=O)-N-R₁₀R₁₁, =O and phenyl; or
a 9- or 10- membered aromatic or heterocyclic fused ring, directly attached to the quinazolinone ring or attached through -C₁-C₆alkyl-, containing zero, one, two or three heteroatoms selected from N, O and S, and optionally substituted with one, two, three or four substitutents selected from C₁-C₆alkyl, C₁-C₆alkoxy, hydroxy, cyano, halo, R₁₀R₁₁N-, R₉-O-(C=O)-, -(C=O)-N-R₁₀R₁₁, and phenyl;
R₇ is hydroxy, esterified hydroxy, etherified hydroxy, amino, (C₁-C₆alkyl)amino, a group 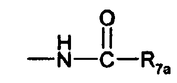 or a group 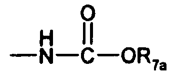 where R₇ₐ is C₁-C₆alkyl or halogen-substituted C₁-C₆alkyl, or a group 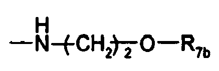 , where R_{7b} is benzyl or phenylethyl; and
R₅, R₆ and R₈ are each independently hydrogen, halogen, C₁-C₆alkyl, C₂-C₆alkenyl, C₂-C₆alkynyl, hydroxy, hydroxy-substituted C₁-C₆alkyl, C₁-C₆alkoxy, C₃-C₆Cycloalkyl, cyano, -C(=O)H, phenyl, (C₃-C₆cycloalkyl)C₁-C₆alkyl, (C₃-C₆cycloalkyl)C₁-C₆alkoxy, (C₁-C₆alkoxycarbonylamino)C₁-C₆alkoxy or (C₁-C₆alkylcarbonylamino)C₁-C₆alkoxy, (amino) C₁-C₆alkoxy, (dimethylamino)C₁-C₆alkoxy, or (C₁-C₆alkoxycarbonyl) C₁-C₆alkoxy, and R₈ is further suitably hydroxy-substituted (C₃-C₆cycloalkyl)C₁-C₆alkyl, hydroxy-substituted phenylC₁-C₆alkyl, hydroxy-substituted heteroarylC₁-C₆alkyl, C₁-C₆alkylcarbonyl, C₁-C₆alkoxyC₁-C₆alkoxy or heteroarylC₁-C₆alkyl,
in free form or in salt form , provided that, in formula (I), when R₂ is selected from group (a), R₃ is selected from group (b') and when R₃ is selected from group (b), R₃ is selected from group (a'), and excluding the compounds in which R₇ is hydroxyl and R₅, R₆ and R₈ are each independently hydrogen and R₂ is isopropyl and R₃ is pyridin-5-yl substituted in the 2-position by Cl or CN.

In a further aspect of the present invention, there is also provided a compound formula (I) where R₂ is phenylC₁-C₆alkyloxycarbonylaminoC₁-C₆alkyl.

If at least one asymmetrical carbon atom is present in a compound of the formula I, such a compound may exist in optically active form or in the form of mixtures of optical isomers, e. g. in the form of racemic mixtures. All optical isomers and their mixtures, including the racemic mixtures, are part of the present invention.

Compounds of formula I are useful as vanilloid antagonists, that is, they exhibit human vanilloid antagonist activity, and, more particularly, they demonstrate antagonism at the TRPVI receptor. As such they are indicated in the treatment of diseases and conditions in which vanilloid receptor activity plays a role or is indicated.

In the compounds of formula I, certain substituents may be preferred, independently, collectively, or in any combination or sub-combination, subject to the above proviso.
---- is preferably a double bond;

In certain embodiments, in the compound of formula I, R₂ may preferably be C₁-C₈alkyl or cycloalkyl, more preferably C₁-C₆ alkyl, for example C₁-C₄ alkyl. One particularly preferred value for R₂ is isopropyl. In other embodiments, R₂ may preferably be NH₂ or C₂-C₆alkenyl, for example C₂-C₄alkenyl, such as isopropenyl. When R₂ is a heterocyclic ring as described above it is preferably 5- or 6- membered with one or two heteroatoms selected from N, O and S; a preferred subsituent for the heterocyclic ring is C₁-C₆alkyl, for example C₁-C₆alkyl such as methyl; where the heterocyclic ring is attached to the quinazolinone ring via C₁-C₆alkyl, C₁-C₆alkyl such as propyl, ethyl, and, most preferably methyl, is preferred. Examples of suitable heterocyclic rings include pyridine, furanyl, isoxazole, pyrrolidone, imidazole, thiophene, morpholine, pyrazine, pyrrole, piperidine and thiazole.

Where R₂ is phenylC₁-C₆alkyloxycarbonylaminoC₁-C₆alkyl, this is suitably 1-benzyloxycarbonylaminoethyl.

Preferably, R₂ is isopropyl, ethyl, tert-butyl, hydroxyisopropyl, dimethylamino or 2-isopropenyl, especially isopropyl.

When R₃ is C₁-C₆alkyl, (NC)-C₁-C₆alkyl-, R₉-O-(C₁-C₆alkyl)-, R₉-O-(C₁-C₆alkyl)-O-(C₁-C₆alkyl)-,R₁₀R₁₁N-(C₁-C₆alkyl)-, R₁₀R₁₁N-(C=O)-(C₁-C₆alkyl)-, or (C₁-C₆alkyl)-SO₂-(C₁-C₆alkyl)-, wherein R₉, R₁₀ and R₁₁ are each independently H or C₁-C₆ alkyl, it may preferably be one of the following:
C₁-C₆alkyl, for example C₁-C₄alkyl, such as isopropyl, propyl, methylbutyl;
(NC)-C₁-C₆alkyl-, for example (NC)-C₁-C₄alkyl, such as acetonitrile;
R₉-O-(C₁-C₆alkyl), for example R₉-O-(C₁-C₄alkyl), such as hydroxyethyl, methoxyethyl;
R₁₀R₁₁N-(C₁-C₆alkyl)-, for example R₁₀R₁₁N-(C₁-C₄)alkyl)-, such as dimethylaminoethyl, methylaminoethyl;
R₁₀R₁₁N-(C=O)-(C₁-C₆alkyl)-, such as R₁₀R₁₁N-(C=O)-(C₁-C₄alkyl), such as dimethylacetamide;
R₉-O-(C₁-C₆alkyl)-O-(C₁-C₆alkyl)-, such as R₉-O-(C₁-C₄alkyl)-O-(C₁-C₄alkyl)-, such as hydroxyethoxyethyl;
(C₁-C₆alkyl)-SO₂-(C₁-C₆alkyl)-, such as (C₁-C₄alkyl)-SO₂-(C₁-C₄alkyl)-, such as methylsulfonylethyl;
when R₃ is unsubstituted phenyl or phenyl substituted according to the above, it may preferably be one of the following:
unsubstituted phenyl;
C₁-C₆ alkoxy phenyl, for example C₁-C₆ alkoxy phenyl, such as methoxyphenyl; or Phenyl substituted by halogen according to the above; such as phenyl substituted with halogen, for example chlorine, and with R₁₀R₁₁N-(C₁-C₆alkyl)-, for example R₁₀R₁₁N-(C₁-C₆alkyl)-, such as dimethylaminomethyl, or phenyl substituted three or four times wherein the substituents are selected from halo, for example chloro and fluoro, hydroxyl, methoxy, trifluoromethyl and methyl;
Phenyl substituted with a 5- or 6-membered saturated or unsaturated heterocyclic ring having one, two or three heteroatoms selected from N, O and S, for example oxazole, or
Phenyl substituted with halo-substituted phenyl, for example fluoro-biphenyl;
when R₃ is cycloalkyl as defined above it may preferably be one of the following: C₃-C₆ cycloalkyl directly attached to the quinzolinone ring, for example cyclopropyl, cyclobutyl, cyclopentyl or cyclohexyl;
C₃-C₆ cycloalkyl attached to the quinzolinone ring via C₁-C₆alkyl, for example C₁-C₄alkyl, such as propyl, isopropyl, ethyl or, particularly, methyl;
Substituted C₃-C₆ cycloalkyl having for example a single substitutent selected from - (C=O)OR₉, for example -(C=O)OC₁-C₆alkyl such as -(C=O)OC₁-C₄alkyl, for example -(C=O)OMe or, particularly, -(C=O)OEt;
when R₃ is benzyl, or phenyl(C₁-C₆alkyl)-, phenoxy-(C₁-C₆alkyl)- or phenyl(C=O)-(C₁-C₆alkyl)-, each as defined above, it may preferably be one of the following:
benzyl;
benzyl substituted by one or two substituents selected from CC₁-C₆alkyl, for example C₁-C₆ alkyl such as methyl, C₁-C₆alkoxy, for example C₁-C₄ alkoxy such as methoxy, phenylethyl;
phenylpropyl;
phenyl(C=O)-(C₁-C₆alkyl)-, for example phenyl(C=O)-(C₁-C₄alkyl)-, such as -CH₂-(C=O)-Ph;
when R₃ is a 5, 6, or 7- membered, saturated or unsaturated, heterocyclic ring, as defined above, it may preferably be one of the following:
i) a 5-or 6-membered, saturated or unsaturated, heterocyclic ring directly attached to the quinazolinone ring;
ii) a 5-or 6-membered, saturated or unsaturated, heterocyclic ring attached to the quinazolinone ring via a methyl or ethyl linker;
iii) a 5-or 6-membered, saturated or unsaturated, heterocyclic ring directly attached to the quinazolinone ring or attached to the quinazolinone ring via a methyl or ethyl linker, containing one or two heteroatoms selected from N, O and S;
iv) any of i)-iii) above substituted with a substituent selected from cyano, C₁-C₆alkyl, for example C₁-C₄alkyl, such as ethyl or, particularly, methyl, halo, for example fluoro or, particularly, chloro, halo phenyl, for example fluoro- or, particularly, chlorophenyl; R₉-O-(C=O)-, for example C(O)OMe or, particularly, C(O)OEt, or =O;
v) any of i)-iv) above wherein the 5-or 6-membered, saturated or unsaturated, heterocyclic ring is selected from pyridine, furanyl, isoxazole, pyrrolidone, imidazole, thiophene, morpholine, pyrazine, pyrrole, piperidine and thiazole;
when R₃ is a 9- or 10- membered aromatic or heterocyclic fused ring as described above, it may preferably be one of the following:
i) a 9- or 10- membered aromatic or heterocyclic fused ring having zero, one or two heteroatons selected from N, O and S;
ii) a 9- or 10- membered aromatic or heterocyclic fused ring according to i) directly attached to the quinazolinone ring;
iii) a 9- or 10- membered aromatic or heterocyclic fused ring according to i) attached to the quinazolinone ring via a methyl or ethyl linker;
iv) a 9- or 10- membered aromatic or heterocyclic fused ring according to ii) or iii) optionally substituted with a substituent selected from halo, for example fluoro or, preferably, chloro, or hydroxyl;
v) a 9- or 10- membered aromatic or heterocyclic fused ring according to ii), iii) or iv), selected from naphthalene, benzothiazole, benzodioxole and quinoline;
and when R₃ is selected from group (a'), it is preferably phenyl substituted by chloro, bromo, C₁-C₄alkyl, hydroxy, C₁-C₄alkoxy or (C₃-C₆cycloalkyl)C₁-C₄alkoxy;

Where R₃ is substituted phenyl, the substituents is / are preferably, 4-chloro, 4-chloro-3-fluoro, 4-methyl, 4-methylcarbonyl, 4-iodo, 4-ethyl, 4-chloro-2-fluoro, 4-cyano-3-methoxy, 4-chloro-3-hydroxy, 4-chloro-3-propoxy, 4-chloro-3-methoxymethyl, 4-chloro-3-hydroxymethyl or 4-cyano.

Where R₃ is substituted pyridyl, the pyridyl is preferably 3-substituted and substituents is / are preferably 2-chloro, 2-bromo, 2-trifluoromethyl, 2-cyano, 2-chloro-3-methyl, 2-chloro-3-hydroxy, 2-cyano-3-methoxy, 2,3-dichloro, 2-trifluromethyl-3-methyl, 2-trifluoromethyl-3-methoxy, 2-cyano-3-methyl, 2-chloro-3-iodo or 2-methyl.

Preferably, R₃ is phenyl, pyridyl or pyrimidyl, where each ring is substituted by one or two halo, trifluoromethyl, C₁-C₆alkyl, C₁-C₆alkoxy, C₁-C₆alkoxyC₁-C₆alkyl, C₁-C₆hydroxyalkyl, C₁-C₆alkylcarbonyl, cyano or hydroxyl, or R₃ is indazolyl or 1-oxo-indan-5-yl.

R₅ is preferably hydrogen or hydroxyl, most preferably hydrogen.

R₆ is preferably hydrogen or hydroxyl, most preferably hydrogen.

R₇ is most hydroxyl or amino, most preferably hydoxyl.

R₈ is suitably hydrogen, halogen, C₁-C₆alkyl, C₂-C₆alkenyl, C₂-C₆alkynyl, hydroxy, hydroxy-substituted C₁-C₆alkyl, C₁-C₆alkoxy, C₁-C₆cycloalkyl, cyano, -C(=O)H, phenyl, (C₃-C₆cydoalkyl)C₁-C₆alkyl, (C₃-C₆cycloalkyl)C₁-Caalkoxy, (C₁-C₆alkoxycarbonylamino)C₁-C₆alkoxy or (C₁-C₆alkylcarbonylamino)C₁-C₆alkoxy, (amino) C₁-C₆alkoxy, (dimethylamino)C₁-C₆alkoxy, or (C₁-C₆alkoxycarbonyl) C₁-C₆alkoxy.

R₈ is also suitably
- hydrogen, - phenyl,
- hydroxy-substituted C₁-C₆alkyl, e.g. 1-hydroxypropyl, 1-hydroxyethyl, 1-hydroxy-2-methyl-propyl, 1-hydroxybutyl, 1-hydroxy-2-methyl-propyl, 1-hydroxy-2,2dimethylpropyl, hydroxymethyl or 1-hydroxy-1-methyl-ethyl,
- hydroxy-substituted (C₃-C₆cyc)oalkyl)C₁-C₆alkyl, where the cycloalkyl is selected from cyclopropyl, cyclobutyl and cyclohexyl, and where the hydroxyl-substituted alkyl is suitably 1-hydroxymethyl,
- hydroxy-substituted phenylC₁-C₆alkyl, e.g. 1-hydroxyphenylethyl or 1-hydroxybenzyl,
- hydroxy-substituted heteroarylC₁-C₆alkyl, e.g. 1-hydroxy - 2- or 3-pyridylmethyl,
- C₁-C₆alkylcarbonyl, e.g. ethylcarbonyl, propyl,arbonyl, isopropylcarbonyl or methylcarbonyl,
- C₁-C₆alkoxyC₁-C₆alkoxy, e.g. methoxyethoxy, or
- heteroarylC₁-C₆alkyl, e.g. 2-pyridylmethyl.

R₈ is most preferably hydrogen, or hydroxy-substituted C₁-C₆ alkyl, for example hydroxymethyl, 1-hydroxyethyl, or 1-hydroxypropyl.

"C₁-C₆alkyl" denotes straight-chain or branched C₁ to C₆-alkyl; "C₁-C₆alkyl" denotes straight-chain or branched C₁ to C₆-alkyl; and "C₁-C₄alkyl" denotes straight-chain or branched C₁ to C₆-alkyl;e.g., methyl ethyl, *n*-propyl, isopropyl, *n*-butyl, isobutyl, sec-butyl or *tert*-butyl.

"C₂-C₆alkenyl" denotes straight-chain or branched C₂ to C₆-alkenyl, e.g., ethenyl, *n*-propenyl or isopropenyl.

"C₁-C₆alkoxy" denotes straight-chain or branched C₁ to C₆-alkyl-oxy, e.g., methoxy, ethoxy, *n*-propoxy or isopropoxy.

"Halo" denotes halogen which may be I, Br, Cl or F.

"Esterified hydroxy" denotes acyloxy, preferably C₁-C₆alkanoyloxy, more preferably C₁-C₄alkanoyloxy.

"Etherified hydroxy" denotes C₁-C₆alkoxy, preferably C₁-C₄alkoxy.

"Heteroaryl" denotes an aromatic ring 5-6 membered aromatic ring comprising one or more nitrogen, oxygen and sulfur atoms as appropriate, e.g. pyridyl or pyrmidiyl.

The quinazolinone compounds of the invention exist in free or salt form. The invention is to be understood as including the compounds of formula (I) in free or salt form.

According to an alternative aspect, there is provided a process for preparing an intermediate compound of formula (II), where R₁ is H or a suitable protecting group and where R₂ is described hereinabove.

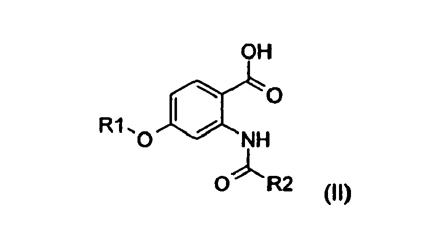

Compounds of formula (11) are useful in the preparation of compounds of the present invention or of compounds described in WO2005120510, those compounds being incorporated herein for the benefit of this invention in their entirety.

A compound of formula (II) may be prepared from a compound of formula (III) 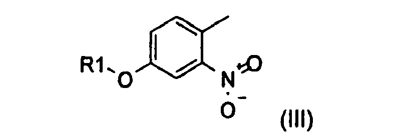 where R₁ is a suitable protecting group by a series of oxidation and reduction / acylation steps illustrated in Scheme 1.

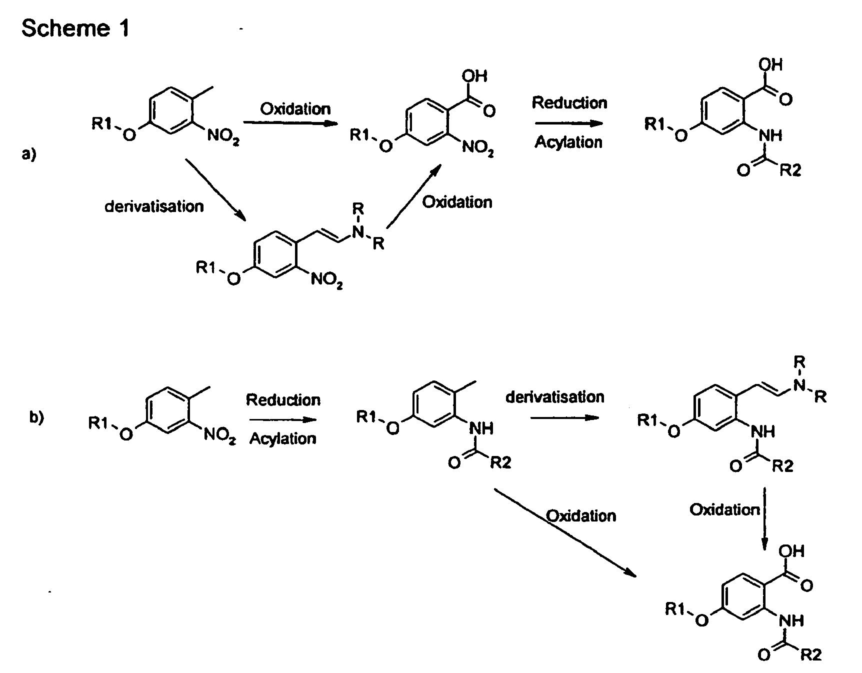

WO2005120510 describes the synthesis of compounds of formula (II) where R₁ is H from compounds of formula (III) where R₁ is H by the methods described above. The inventors have now found that the overall yield of production of compound (II) is improved where the compound of formula (II) is protected at the OH position by a suitable protecting group.

Suitable protecting groups include those selected from C₁₋₆alkyl, e.g. methyl, C₁-ₐ aralky, e.g. benzyl, C₁₋₆alkoxyC₁₋₆alkyl, e.g. methoxymethyl or methoxyethoxymethyl, C₁₋₆aralkoxyC₁₋₆alkyl, e.g. benzyloxymethyl, C₁-₆trialkylsitylatkoxyC₁₋₆alkyl, tetrahydropyranyl, C₁₋₆trialkylsilyl, e.g. triisopropylsilyl or t-butyldimethylslilyl, or diarylC₁₋₆alkylsilyl, e.g. t-butyldiphenylsilyl.

A compound of formula (III) may be prepared from the corresponding compound where R₁ is H by standard protection methods.

Referring to scheme , the oxidation step may take place directly on the methyl group or on a derivatised dialkyaminovinyl derivative. Oxidation may be effected by any suitable oxidation reagent, e.g. KMnO₄, under standard conditions. The derivatised dialkylaminovinyl derivative may be prepared from the corresponding methyl derivative by treatment with Bredereck's reagent (t-butoxy bis(dimethylamino) methane under standard conditions. The reduction step may be effected by any suitable reducing agent, e.g. a metal such as iron, zinc or tin, under standard conditions and the acylation may be effected by any suitable acylating agent, e.g. R₂COCl under standard conditions.

A compound of formula (II) where R₁ is H may be prepared from a protected form of a compound of formula (II) by standard deprotection methods well-known to those skilled in the art. Alternatively, the protected form of a compound of formula (II) may be taken forward to the next step to prepare compounds of the invention or similar without deprotection, followed by deprotection as a final step.

Thus, there is provided a process for the manufacture of a compound of formula (II) where R¹ is H or a suitable protecting group and R₂ is selected from
(a) C₁-C₈alkyl, C₃-C₆cycloalkyl, (C₁-C₆alkyl)amino or di-(C₁-C₆alkyl)amino;
   or
(b) NH₂, hydroxyC₁-C₆alkylamino-, aminoC₁-C₆alkylamino, C₂-C₆alkenyl, di(t(ifluoromethyl)C₁-C₆alkyl, R₉-O-(C₁-C₆alkyl)- in which the alkyl chain is optionally substituted by trifluoromethyl, (NC)-C₁ -C₆alkyl-, (R₁₀R₁₁N-)C₁-C₆alkyl-, (C₁-C₆alkyl)-SO₂(C₁-C₆alkyl)-, wherein R₉, R₁₀ and R₁₁ are each independently H or C₁-C₆ alkyl; phenyl optionally substituted by one, two or three substituents each independently selected from the group consisting of halogen, C₁-C₆alkyl, halogen-substituted C₁-C₆alkyl, hydroxy C₁-C₆alkyl, cyano or a group -(C=O)-R₂ₐ, where R₂ₐ is C₁-C₆alkyl; or 5, 6, or 7-membered, saturated or unsaturated, heterocyclic ring, directly attached to the quinazolinone ring or attached through -C₁-C₆ alkyl-, containing one, two, or three heteroatoms selected from N, O and S, and optionally substituted with one, two or three substitutents selected from C₁-C₆alkyl, C₁-C₆alkoxy, hydroxy, cyano, halo, R₁₀R₁₁N-, R₉-O-(C=O)-, -(C=O)-N-R₁₀R₁₁, =O and phenyl ;
   from a compound of formula (III) by one of the following sequential steps:
   a) oxidation using a suitable oxidizing agent, reduction using a suitable reducing agent and acylation with a suitable acylating agent; or
   b) reduction using a suitable reducing agent, acylation with a suitable acylating agent and oxidation using a suitable oxidizing agent; or
   c) conversion of the methyl group to a dialkylaminovinyl group using a suitable agent, oxidation using a suitable oxidizing agent, reduction using a suitable reducing agent and acylation with a suitable acylating agent; or
   d) reduction using a suitable reducing agent, acylation with a suitable acylating agent., conversion of the methyl group to a dialkylaminovinyl group using a suitable agent, and oxidation using a suitable oxidizing agent, followed by optional deprotection of the protecting group under standard conditions.

The present invention also provides processes for preparing compounds of formula (I), as defined above, as depicted in the following reaction schemes.

The following Scheme 2 is applicable to the manufacture of a broad range of compounds of the invention but is exemplified with compounds where R₂ is isopropyl, R₅, R₆, and R₈ are each hydrogen and R₇ is hydroxyl.

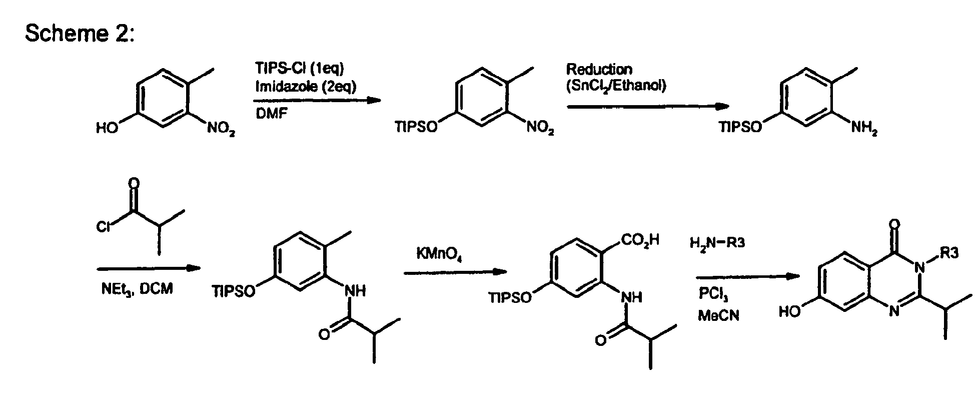

The following Scheme 3 is applicable to the manufacture of a broad range of compounds of the invention but is exemplified with compounds where R₃ is chlorophenyl, R₅, R₆ and R₈ are each hydrogen and R₇ is hydroxyl.

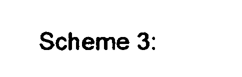

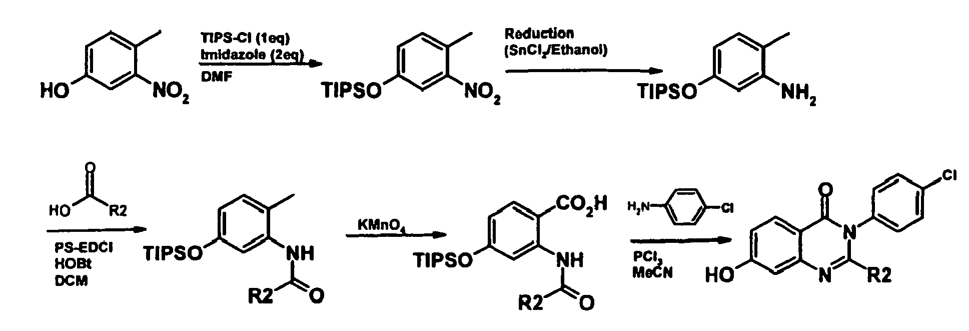

Other compounds of formula I may be prepared analogously to the above.

The following Scheme 4 is applicable to the manufacture of a broad range of compounds but is exemplified where R₃ is phenyl substituted by R, R₅, R₆ and R₈ are each hydrogen and R₇ is hydroxyl.

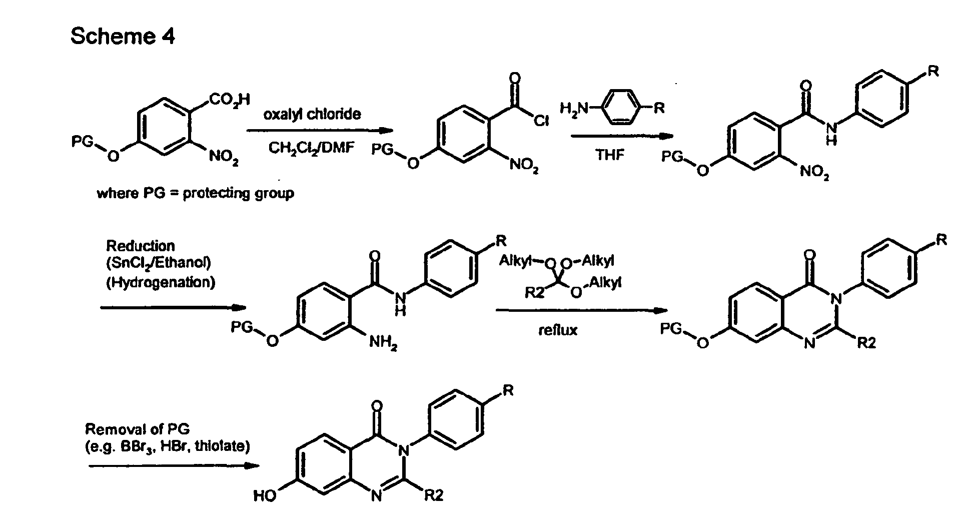

The following Scheme 5 is applicable to the manufacture of a broad range of compounds of the invention but is exemplified where R₃ is phenyl substituted by R, R₅, R₆ and R₈ are each hydrogen and R₇ is hydroxyl.
R₂ N-substituted and R₃ substituted quinazolinones.

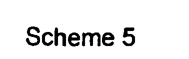

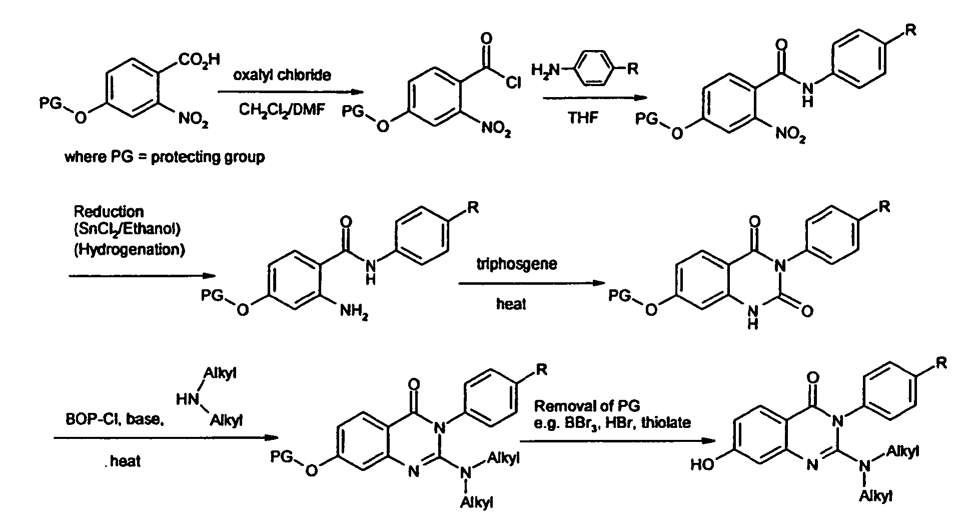

Scheme 6 is applicable to a broad range of substituents but is exemplified where R₃ is phenyl substituted by R, R₅, R₆ and R₈ are each hydrogen and R₇ is hydroxyl, to illustrate the synthesis of R₂ and R₃ substituted quinazolinones:

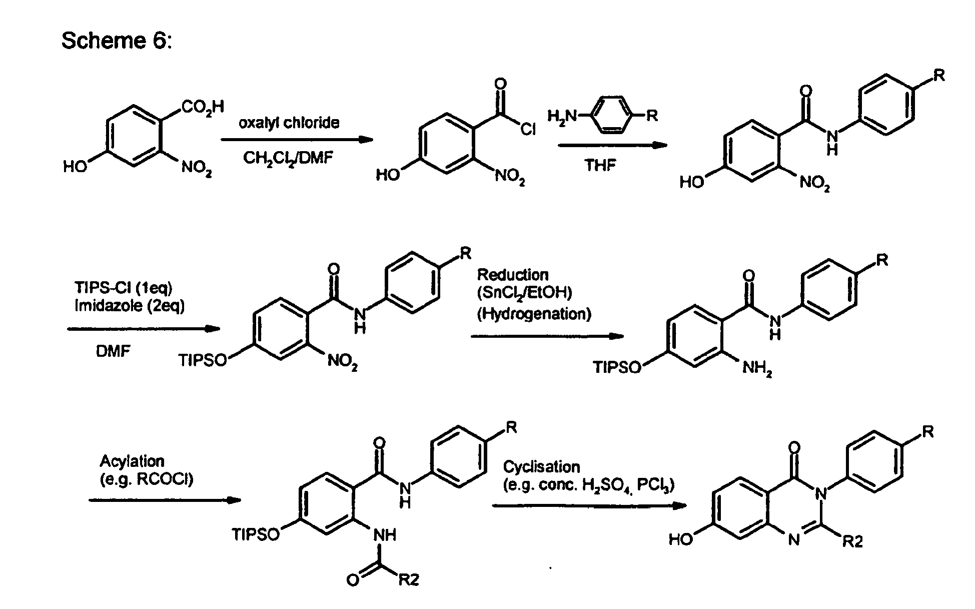

Scheme 7 is applicable to a broad range of substituents but is exemplified where R₃ is phenyl substituted by R, R₅, R₆ and R₈ are each hydrogen and R₇ is hydroxyl, to illustrate the synthesis of R₂ and R₃ substituted quinazolinones:

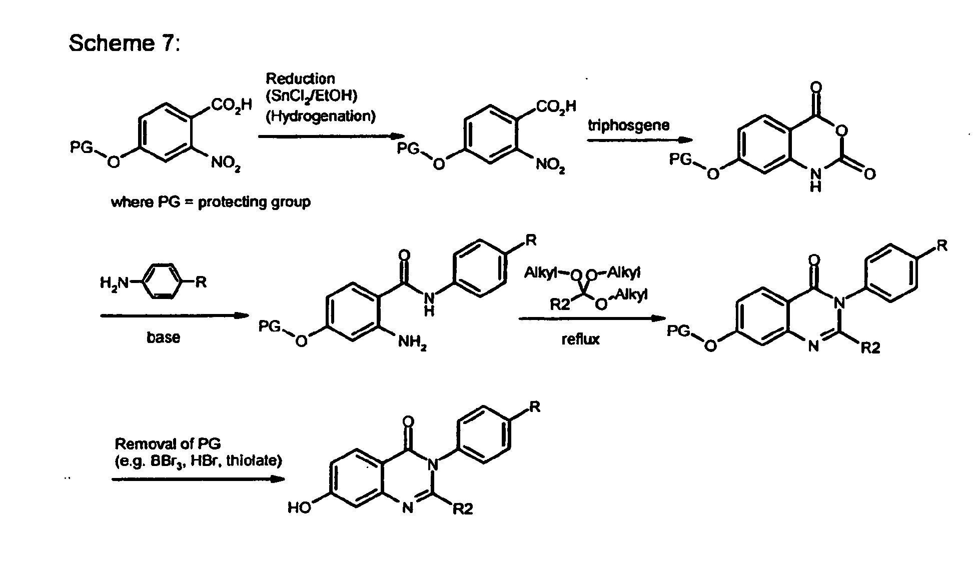

Scheme 8 is applicable to a broad range of substituents but is exemplified where R₃ is phenyl substituted by R, R₅ and R₆ are each hydrogen and R₇ is hydroxyl, to illustrate the synthesis of R₂, R₃ and R₈ substituted quinazolinones:

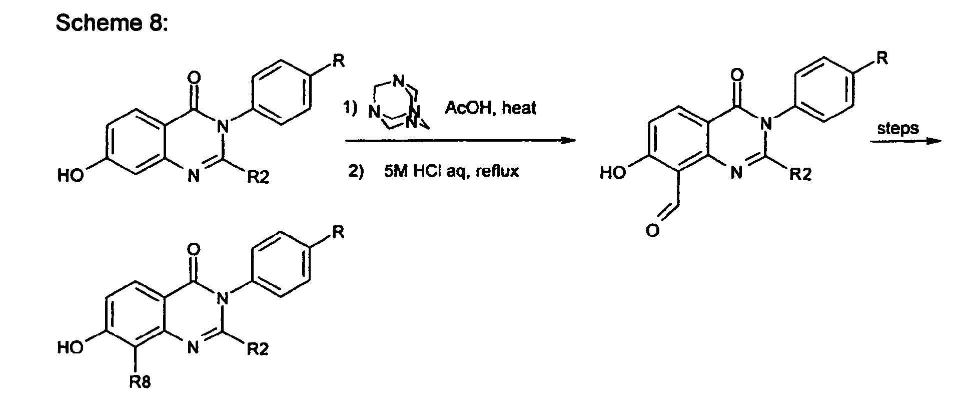

Subsequent reaction steps include reduction of the aldehyde or reaction with an organometallic reagent. The hydroxyalkyl- or hydroxyaryl- compound can then be oxidized or reduced.

Scheme 9 is applicable to a broad range of substituents but is exemplified where R₃ is phenyl substituted by R, R₅ and R₆ are each hydrogen and R₇ is hydroxyl, to illustrate the synthesis of R₂, R₃ and R₈ substituted quinazolinones:

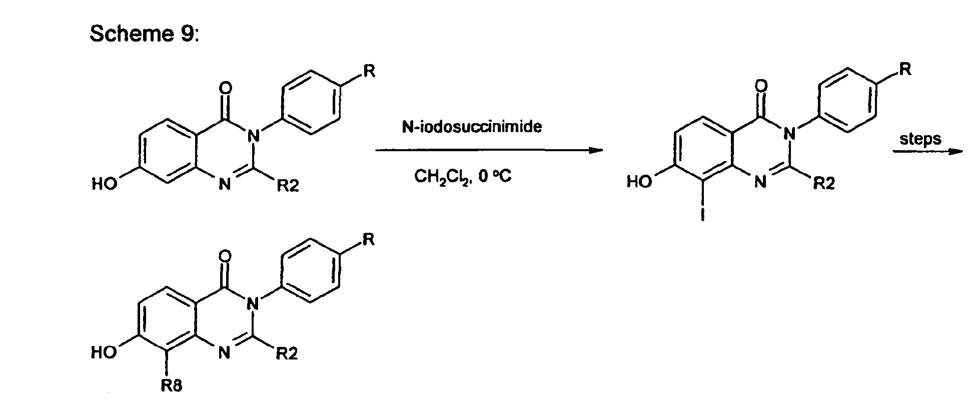

Subsequent reaction steps using the 8-iodo- compound include palladium mediated cross coupling reactions.

Working up the reaction mixtures according to the above processes and purification of the compounds thus obtained may be carried out in accordance with known procedures.

Acid addition salts may be produced from the free bases in known manner, and vice-versa.

Compounds of formula (I) in optically pure form can be obtained from the corresponding racemates according to well-known procedures, e.g., HPLC with chiral matrix. Alternatively, optically pure starting materials can be used.

Stereoisomeric mixtures, e.g., mixtures of diastereomers, can be separated into their corresponding isomers in a manner known per se by means of suitable separation methods. Diastereomeric mixtures, e.g., may be separated into their individual diastereomers by means of fractionated crystallisation, chromatography, solvent distribution and similar procedures. This separation may take place either at the level of a starting compound or in a compound of formula (I) itself. Enantiomers may be separated through the formation of diastereomeric salts, e.g., by salt formation with an enantiomer-pure chiral acid, or by means of chromatography, e.g., by HPLC, using chromatographic substrates with chiral ligands.

In any additional process steps, carried out as desired, functional groups of the starting compounds which should not take part in the reaction may be present in unprotected form or may be protected, e.g., by one or more of the protecting groups mentioned below. The protecting groups are then wholly- or partly-removed according to one of the methods described there.

The protecting groups may already be present in precursors and should protect the functional groups concerned against unwanted secondary reactions. It is a characteristic of protecting groups that they lend themselves readily, i.e., without undesired secondary reactions, to removal, typically by solvolysis, reduction, photolysis or also by enzyme activity, e.g., under conditions analogous to physiological conditions, and that they are not present in the end-products. The skilled artisan knows, or can easily establish, which protecting groups are suitable with the reactions mentioned hereinabove and hereinafter.

The protection of such functional groups by protecting groups, the protecting groups themselves, and their removal reactions are described, e.g., in standard reference works, such as J.F.W. McOmie, Protective Groups in Organic Chemistry, Plenum Press, London and NY (1973); T.W. Greene, Protective Groups in Organic Synthesis, Wiley, NY (1981); The Peptides; Volume 3, E. Gross and J. Meienhofer, Eds., Academic Press, London and NY (1981); Methoden der organischen Chemie (Methods of organic chemistry), Houben Weyl, 4th Edition, Volume 15/1, Georg Thieme Verlag, Stuttgart (1974); H.D. Jakubke and H. Jescheit, Aminosauren, Peptide, Proteine (Amino acids, peptides, proteins), Verlag Chemie, Weinheim, Deerfield Beach, and Basel (1982); and Jochen Lehmann, Chemie der Kohlenhydrate: Monosaccharide und Derivate (Chemistry of carbohydrates: monosaccharides and derivatives), Georg Thieme Verlag., Stuttgart (1974).

All process steps described herein can be carried out under known reaction conditions, preferably under those specifically mentioned, in the absence of or usually in the presence of solvents or diluents, preferably such as are inert to the reagents used and able to dissolve these, in the absence or presence of catalysts, condensing agents or neutralizing agents, e.g., ion exchangers, typically cation exchangers, e.g., in the H⁺ form, depending on the type of reaction and/or reactants at reduced, normal or elevated temperature, e.g., in the range from -100°C to about 190°C, preferably from about -80°C to about 150°C, e.g., at -80°C to 60°C, at room temperature, at -20°C to 40°C or at the boiling point of the solvent used, under atmospheric pressure or in a closed vessel, where appropriate under pressure, and/or in an inert atmosphere, e.g., under argon or nitrogen.

Another aspect of this invention relates to the fact that the compounds of formulae (I) and their pharmaceutically acceptable salts, have beneficial pharmacological activity and, therefore, are useful as pharmaceuticals. In particular, the compounds of formula (I)exhibit human vanilloid antagonistic activity. More particularly, the compounds of formula (I)are active at the TRPVI receptor as demonstrated by their ability to inhibit capsaicin and/or low pH activation of the TRPVI ion channel as follows:
Chinese Hamster Ovary-K1 (CHO-K1) cells, transfected to express either the human, rat or guinea pig TRPV1 receptor, are grown in Minimal Essential Media (MEM) alpha medium without nucleosides supplemented with fetal calf serum (10%), 2 mM L-glutamine, 100 IU/mL penicillin, 100 µg/mL streptomycin and 350-700 µg/mL geneticin. All reagents are supplied by Invitrogen. Cells are grown in T-175 flasks or clear bottom 96- or 384-well plates and maintained at 37°C in a 90% humidified incubator with an atmosphere of 5% CO₂ and 95% air. The cells are passaged twice a week and for experimentation, cells are harvested at approximately 80% confluency and plated onto view plates at 35,000-40,000 cells per well in 100 µL media and grown overnight.

### Calcium mobilisation assay

On the day of the assay, media is aspirated and cells are washed with 10 mM *N*-2-(hydroxyethylpiperazine-*N*'-[2-ethane-sulfonic acid] (HEPES) buffered Hank's Balanced Salt Solution (HBSS), pH 7.4. Cells are then incubated with a fluorescent sensitive-calcium binding dye, typically fluo-4/AM (from Molecular Probes), made up in HEPES buffered HBSS, containing pluronic F-127 with or without probenicid. For the pH assay, HEPES is omitted and the pH of HBSS adjusted to 7.4. After washing, cells are incubated with test compounds (made up in HBSS, pH 7.4), in duplicate. The TRPV1 receptor is stimulated by addition of either capsaicin at an approximate EC₈₀ concentration or a low pH buffered solution [60 mM 2-[*N*-morpholino] ethane sulfonic acid (MES) in HBSS] to give a final pH of 5.5. The cellular responses are followed in fluorescent plate reader, typically a Molecular Devices Flexstation. The response in the presence of the antagonist is calculated as a percentage of the control response to capsaicin or low pH and is plotted against the concentration of antagonist. The IC₅₀ values (concentrations of antagonist that inhibit responses to either pH 5.5 or capsaicin by 50%) is estimated by non-linear regression analysis to sigmoidal-logistic curves. These values are averaged (means and standard error of the mean) for at least three independent experiments.

A specific example of a calcium mobilization assay is as follows:
On the day of the capsaicin assay, media is aspirated and cells are washed with 100 µL 10 mM *N*-2-(hydroxyethylpiperazine-*N*'-[2-ethane-sulfonic acid] (HEPES) buffered Hank's Balanced Salt Solution (HBSS), pH 7.4. Cells are then incubated for 40-60 minutes with 2.3 µM of the calcium binding dye fluo-4/AM (from Molecular Probes), made up in HEPES buffered HBSS, containing 0.01% pluronic F-127 and 2mM probenicid. For the pH assay, HEPES is omitted and the pH of HBSS adjusted to 7.4. After washing twice with 100 µL assay buffer, cells are incubated for 10 minutes with 100 µL of test compounds (made up in HBSS, pH 7.4), in duplicate. The plate is then placed in a Molecular Devices Flexstation. The TRPV1 receptor is stimulated by application of either capsaicin or low pH. For testing the effect of compounds for possible antagonism, capsaicin is used at an approximate EC₈₀ concentration of 0.05 µM. For pH experiments, a low pH buffered solution [60 mM 2-[*N*-morpholino] ethane sulfonic acid (MES) in HBSS] is added to the assay wells to give a final pH of 5.5.

For determinations of antagonist IC₅₀ values (concentrations of antagonist that inhibit responses to either pH 5.5 or capsacin by 50%), at least 10 antagonist concentrations are measured in duplicate. The response in the presence of the antagonist is calculated as a percentage of the control response to capsaicin or low pH and is plotted against the concentration of antagonist. The IC₅₀ is estimated by non-linear regression analysis to sigmoidal-logistic curves by Activity-Base software (v5.0.10) or Microcal Origin (v7.03). These values are averaged (means and standard error of the mean) for at least three independent experiments.
The agents of the invention are useful in the prevention and treatment of diseases and conditions in which human VR1 activation plays a role or is implicated, and therefore susceptible to treatment by the modulation (preferably antagonism) of VR1 receptors. Such conditions include, in particular, acute or chronic pain of somatic or visceral origin, inflammatory or obstructive airways disease, urinary incontinence or overactive bladder, inflammatory skin diseases, inflammatory disorders of the gastrointestinal tract, diabetes, obesity and obesity-related diseases, psychiatric disorders, and treatment of the consequences exposure to VR1 agonists.

The agents of the invention are particularly useful in the treatment or prevention of chronic pain with an inflammatory component such as rheumatoid arthritis; bone and : joint pain (osteoarthritis); post-surgical or trauma pain including dental pain e.g. following third molar extraction, post mastectomy pain and pain associated with sprains or fractures; musculo-skeletal pain such as fibromyalgia; myofascial pain syndromes; headache, including migraine, acute or chronic tension headache, cluster headache, temporomandibular pain, and maxillary sinus pain; ear pain; episiotomy pain; burns, and especially primary hyperalgesia associated therewith; deep and visceral pain, such as heart pain, muscle pain, eye pain, orofacial pain, abdominal pain, gynaecological pain, such as dysmenorrhoea, and labour pain; hemorrhoids; pain associated with the urogenital tract such as cystitis and vulvadynia; chronic pain associated with nerve injury and/or diseases affecting the nervous system, such as neuropathic pain associated with post-herpetic neuralgia, diabetic neuropathy, chemotherapy-induced neuropathy, amputations ("phantom limb pain"), nerve entrapment and brachial plexus avulsions, low back pain, sciatica and ankylosing spondylitis, reflex sympathetic dystrophy and other chronic nerve injuries; complex regional pain syndromes; Glossodynia or burning mouth syndrome; central nervous system pain, such as pain due to spinal cord or brain stem damage, multiple sclerosis or stroke; gout; scar pain; pain associated with carcinoma, often referred to as cancer pain; pain associated with viral (e.g. HIV)-induced neuropathy, alcohol and narcotic abuse; pain and other symptoms associated with sun or UV burn, exposure to VR1 agonist (e.g. capsaicin, acid, tear gas, noxious heat or pepper spray), snake, spider or insect bite and jellyfish sting.

Gastrointestinal disorders to be treated in accordance with the invention include those associated with gastrointestinal hypersensitivity, visceral pain and/ or altered motor responses (including electrolyte/water secretion) such as functional bowel disorders and functional gastrointestinal disorders, including irritable bowel syndrome (IBS), functional dyspepsia, heartburn, non-erosive reflux disease, intestinal pseudoobstruction, functional abdominal bloating, and functional abdominal pain; other conditions associated with visceral hypersensitivity including gastro-oesophageal reflux disease and emesis, oesophagitis, post-operative visceral pain, post-operative ileus, visceral smooth muscle spasms, ulcerative colitis, Crohn's disease, ulcers, chronic constipation, diarrhea, early satiety, epigastric pain, nausea, vomiting, burbulence, anal incontinence, faecal urgency and rectal hypersensitivity, gastroparesis, e. g. diabetic gastroparesis, pancreatitis and Hirschsprung's disease.

Urinary incontinence ("Ul") or overactive bladder to be treated in accordance with the invention is a broad term that covers a range of disorders and symptoms including urge UI, stress Ul, mixed urge/stress Ul, neurogenic Ul, bladder detrusor hyperreflexia (neurogenic detrusor overactivity), detrusor instability (idiopathic detrusor overactivity), decreased bladder compliance, weakness of urethal sphincter, urinary outlet obstruction, interstitial cystitis, nephritis, uveitis, sensory urgency, motor urgency, nocturia, and bladder-related visceral pain.

The agents of the invention are also useful as agents for the therapy of hyperreactive, inflammatory or obstructive airways diseases including asthma, inflammatory airways disease, e.g. chronic obstructive pulmonary or airways disease (COPD or COAD), adult respiratory distress syndrome (ARDS), chronic bronchitis, pneumoconiosis, e.g. aluminosis, anthracosis, asbestosis, chalicosis, ptilosis, siderosis, silicosis, tabacosis, byssinosis; rhinitis including allergic rhinitis such as seasonal and perennial rhinitis, and non-allergic rhinitis; cough, either idiopathic or associated with respiratory diseases such as COPD, asthma, cystic fibrosis, cancer, or gastrointestinal disturbances such as gastro-oesophageal reflux.

The agents of the invention may also have therapeutic benefit in inflammatory skin disorders, for example psoriasis and eczema, or itch of non-specific origin; contact dermatitis and hypersensitivity; autoimmune or inflammatory diseases, including Crohn's disease, ulcerative colitis and Gullian Barre Syndrome; multiple chemical sensitivity, neurological diseases like anxiety, panic disorders, depression, schizophrenia, cognition, Parkinson's Disease and Alzheimer's Disease; hair loss; diabetes; obesity and obesity-related diseases; as anti-spasmodics, e.g. for the treatment of spasm of the gastrointestinal tract or uterus; for the therapy of septic shock, e.g. as anti-hypovolaemic and / or anti hypotensive agents; cerebral oedema.

For the above-mentioned indications, the appropriate dosage will of course vary depending upon, e.g., the compound employed, the host, the mode of administration and the nature and severity of the condition being treated. However, in general, satisfactory results in animals are indicated to be obtained at a daily dosage of from about 0.05 to about 150, preferably from about 0.1 mg/kg to about 100 mg/kg animal body weight. In larger mammals, e.g., humans, an indicated daily dosage is in the range from about 0.5 to about 5,000, preferably from about 1 mg to about 500 mg of a compound of formula (I), conveniently administered, e.g., in divided doses up to four times a day or in sustained-release form.

The agents of the invention can be administered in vivo either alone or in combination with other pharmaceutical agents, e.g. agents effective in the treatment of diseases and conditions in which the human VR1 activation plays a role or is implicated. A suitable combination consists of a compound of the present invention with a compound selected from the class or individuals from the following list:
Dopamine D₂ antagonists, eg domperidone, metoclopramide and itopride;
5HT₄ receptor agonists, eg cisapride, cinitapride, mosapride, renzapride, prucalopride, tegaserod, and compounds described in WO 2005068461 (Aryx), e.g. AT-7505, US 2005228014 and WO 2005080389 (Theravance), e.g. TDI-2749, US 2006100426, US 2006100236, US 2006135764, US 20060183901, WO 200610827, WO 2006094063, WO 2006090224, WO2006090279, US 2005277671, WO 2005092882, WO 2005073222, JP 2005104896, JP 2005082508, WO 2005021539, JP 2004277319, JP 2004277318, WO 2004026869 and EP 1362857;
5HT₃ agonists, eg pumosetrag;
CCK_{A} receptor antagonists, eg loxiglumide and dexloxiglumide;
Motilin receptor agonists, eg motilin, atilmotilin, erythromycin, alemcinal, mitemcinal, KOS-2187 and compounds described in WO 2005060693;
µ-opioid antagonists, eg alvimopan and methylnaltrexone;
Opioid agonists, eg asimadoline, loperamide and codeine;
CRF-1 receptor antagonists, eg GSK876008 and compounds described in WO 2004069257, WO 9940089, US 6844351, WO 2005013997, WO 2005014557, WO 2005023806, WO 2005026126, WO 2005028480, WO 2005044793, WO 2005051954, WO 2005051954, WO 2005115399, WO 2005028480, WO 2005023806, WO 2006044958, US 20060211710 and WO 2006108698;
Glutamate receptor antagonists, eg AZD9272 and compounds described in WO 9902497, WO 2000020001, WO 200304758 and WO 2005030723;
Neurokinin receptor antagonists, eg casopitant, nepadutrent saredutant, DNK-333, SLV-317, SLV321, SLV317 and compounds described in EP 96-810237;
5HT₃ receptor antagonists, eg alosetron, cilansetron, ramosetron, azasetron, ondansetron, granisetron tropisetron and DDP225;
Histamine H₂ antagonists, eg famotidine, cimetidine, rantidine and nizatidine; Histamine H₄ antagonists, eg JNJ7777120, JNJ10191584 and compounds described in US 2006111416, WO 2006050965, WO 2005092066, WO 2005054239 US 2005070550, US 2005070527 and EP 1505064;
Proton pump inhibitors, eg omeprazole, lansoprazole, rabeprazole, tentoprazole, pantoprazole, esomeprazole, revaprazan soraprazan and AGN201904;
Chloride channel activators, eg lubiprostone;
Guanylate cyclase activators, eg linaclotide;
Muscarinic antagonists, eg darifenacin, solifenacin, atropine, dicycloverine, hycosine butyl bromide, propantheline, oxybutinin, cimetropium bromide, pinaverium bromide and otilonium bromide;
Antispasmodics, eg mebeverine, tiropramide, alverine and peppermint oil;
Stimulant laxatives, eg bisacodyl;
Osmotic laxatives, eg activated charcoal with sorbitol, lactulose, magnesium hydroxide and phosphate buffered saline;
Faecal softeners, eg senna concentrate, liquid paraffin and arachis oil;
Absorbents and fibre supplements, eg bulk fibre laxatives such as bran, methycellulose, ispaghula husk and sterculia;
Antacids, eg aluminium, magnesium and calcium antacids, simeticone and alginate containing preparations;
GI relaxants, eg cholestyramine resin;
Bismuth compounds, eg bismuth subsalicylate;
Vanilloid receptor antagonists, eg compounds described in WO 2002076946, WO 2004033435, WO 2005121116 and WO 2005120510;
Anticonvulsants, eg carbamazepine, oxcarbemazepine, lamotrigine, gabapentin, and pregabalin;
NSAIDS, eg aspirin, acetometaphen, ibuprofen, diclofenac, naproxen, flurbiprofen, indomethacin, piricoxam, ketoprofen, sulindac and diflunisal;
COX-2 inhibitors eg celecoxib, rofecoxib, lumiracoxib, valdecoxib, etoricoxib and compounds described in WO 2004048314;
Opiates, eg morphine, buprenorphine, diamorphine, dihydrocodeine, fentanyl and pethidine;
GABA_{b} modulators, eg racemic and (R)-baclofen, AZD3355, XP19986 and compounds described in WO 2006001750 and WO 2004000856;
CB receptor ligands, eg compounds described in WO 2002042248 and WO 2003066603;
Calcium channel blockers, eg ziconotide, AG10-003, PD-217014 and compounds described in WO 2006038594, WO 2006030211 and WO 2005068448;
Sodium channel blockers, eg lamotrigine and compounds described in WO 2006023757, WO 2005097136, JP 2005206590 and WO 2005047270;
Tricyclic antidepressants, e.g. clomipramine, amoxapine, nortripyline, amitriptyline, imipramine, desipramine, doxepin, trimipramine and protripyline;
Selective serotonin reuptake inhibitors, eg fluoxetine, paroxetine, citaprolam, sertaline, fluvoxamine, duloxetine;
Anxiolytic agents, eg milnacipran, tianeptine, MCI-225 and dextofisopam;
CGRP antagonists, eg olcegepant and cizolirtine;
5HT_{1d} antagonists, eg almotriptan, eletriptan, frovatriptan, naratriptan, rizatriptan, sumatriptan and zolmatriptan; and
Bradykinin receptor antagonists, eg compounds described in WO 2000075107, WO 2002092556 and WO 20050851298.

The pharmaceutical compositions for separate administration of the combination partners and for the administration in a fixed combination, i.e., a single galenical composition comprising at least two combination partners, according to the invention can be prepared in a manner known per se and are those suitable for enteral, such as oral or rectal, and parenteral administration to mammals, including man, comprising a therapeutically effective amount of at least one pharmacologically active combination partner alone or in combination with one or more pharmaceutically acceptable carriers, especially suitable for enteral or parenteral application.

Pharmaceutical compositions contain, e.g., from about 0.1 % to about 99.9%, preferably from about 20% to about 60%, of the active ingredients. Pharmaceutical preparations for the combination therapy for enteral or parenteral administration are, e.g., those in unit dosage forms, such as tablets including sugar-coated tablets, capsules, suppositories and ampoules. These are prepared in a manner known, per se, e.g., by means of conventional mixing, granulating, sugar-coating, dissolving or lyophilizing processes. It will be appreciated that the unit content of a combination partner contained in an individual dose of each dosage form need not in itself constitute an effective amount since the necessary effective amount can be reached by administration of a plurality of dosage units.

A further aspect of the instant invention involves the novel compositions comprising a pharmaceutically acceptable carrier or diluent and a therapeutically effective amount of a compound of formula (I), in free or salt form.

In accordance with the foregoing, the present invention also provides:
(1) A compound of formula (I) in free or salt form for use as a vanilloid receptor blocker, e.g., for use in any of the particular indications set forth hereinabove;
(2) A compound of formula (I) in free or salt form for the treatment of a disease or condition in which vanilloid receptor plays a role or is implicated;
(3) A method for the treatment of any of the particular indications set forth hereinabove in a subject in need thereof which comprises administering a therapeutically effective amount of a compound of formula (I) in free or salt form;
(4) A method for treating or preventing a disease or condition in which vanilloid receptor plays a role or is implicated comprising administering to a mammal in need thereof a therapeutically effective amount of a compound of formula (I) in free or salt form;
(5) Use of a compound of formula (I) in free or salt form for the manufacture of a medicament for the treatment or prevention of a disease or condition in which activity of vanilloid receptor plays a role or is implicated;
(6) A method as set forth hereinabove comprising co-administration, e.g., concomitantly or in sequence, of a therapeutically effective amount of a vanilloid receptor antagonist, e.g., a compound of formula (I) in free or salt form and a second drug substance, said second drug substance being, e.g., for use in any of the particular indications set forth hereinabove; and
(7) A combination comprising a therapeutically effective amount of a compound of formula (I) in free or salt form and a second drug substance, said second drug substance being, e.g., for use in any of the particular indications set forth hereinabove.

In the Examples which follow, which are not intended to limit, in any way, the scope of the present invention, the following abbreviations are used:

### The invention is illustrated by the following Examples.

The following examples have been prepared using the process described herein.

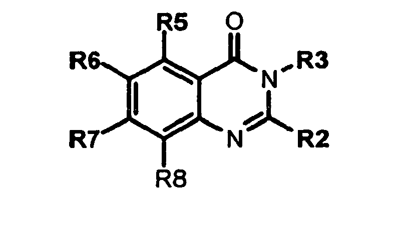

| **Ex** | **R8** | **R7** | **R6** | **R5** | **R3** | **R2** | **[M+H]+** |
|---|---|---|---|---|---|---|---|
| **1** | -H | -OH | -H | -H | 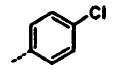 | 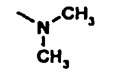 | 344 |
| **2** | -H | -OH | -H | -H | 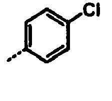 | 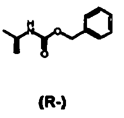 | 450 |
| **3** | -H | -OH | -H | -H | 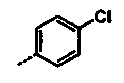 | 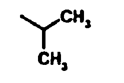 | 315 |
| **4** | -H | -OH | -H | -H | 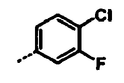 | 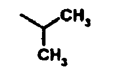 | 333 |
| **5** | -H | -OH | -H | -H | 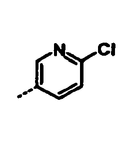 | 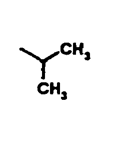 | 316 |
| **6** | -H | -OH | -H | -H | 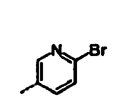 | 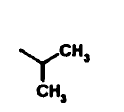 | |
| **7** | -H | -OH | -H | -H | 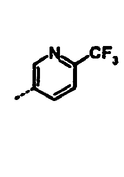 | 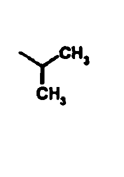 | 350 |
| **8** | -H | -OH | -H | -H | 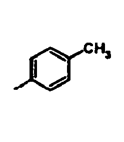 | 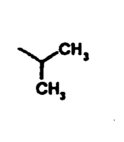 | 295 |
| **9** | -H | -OH | -H | -H | 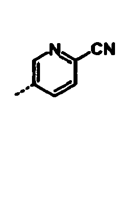 | 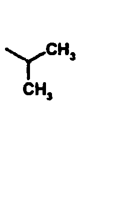 | 307 |
| **10** | -H | -OH | -H | -H | 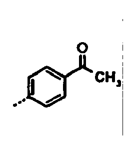 | 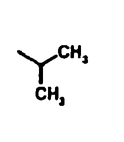 | 323 |
| **11** | -H | -OH | -H | -H | 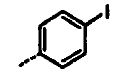 | 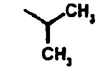 | 407 |
| **12** | -H | -OH | -H | -H | 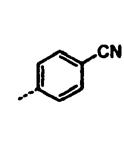 | 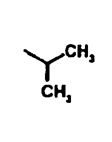 | 306 |
| **13** | -H | -OH | -H | -H | 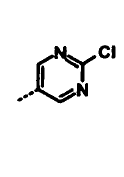 | 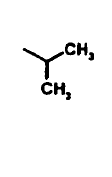 | 317 |
| **14** | -H | -OH | -H | -H | 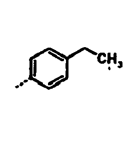 | 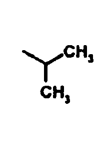 | 309 |
| **15** | -H | -OH | -H | -H | 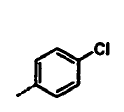 | 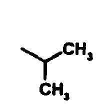 | 315 |
| **16** | -H | -OH | -H | -H | 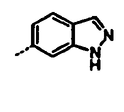 | 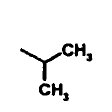 | 321 |
| **17** | -H | -OH | -H | -H | 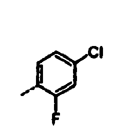 | 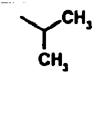 | 333 |
| **18** | -H | -OH | -H | -H | 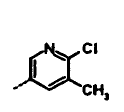 | 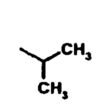 | 330 |
| **19** | -H | -OH | -H | -H | 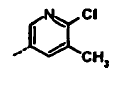 | 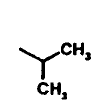 | 346 |
| **20** | -H | -OH | -H | -H | 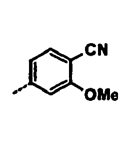 | 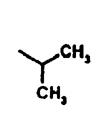 | 336 |
| **21** | -H | -OH | -H | -H | 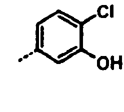 | 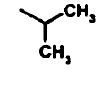 | 331 |
| **22** | -H | -OH | -H | -H | 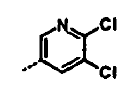 | 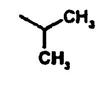 | 350 |
| **23** | -H | -OH | -H | -H | 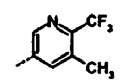 | 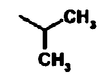 | 364 |
| **24** | -H | -OH | -H | -H | 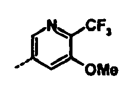 | 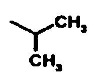 | 380 |
| **25** | -H | -OH | -H | -H | 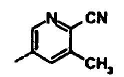 | 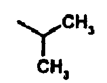 | 321 |
| **26** | -H | -OH | -H | -H | 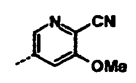 | 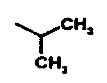 | 337 |
| **27** | -H | -OH | -H | -H | 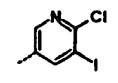 | 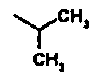 | 442 |
| **28** | -H | -OH | -H | -H | 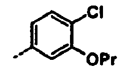 | 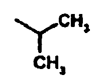 | 372 |
| **29** | -H | -OH | -H | -H | 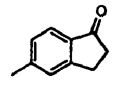 | 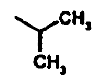 | 335 |
| **30** | -H | -OH | -H | -H | 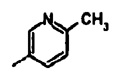 | 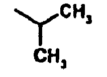 | |
| **31a** | -H | -OH | -H | -H | | | 359 |
| | | | | | 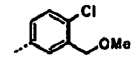 | 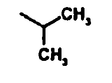 | |
| **31 b** | -H | -OH | -H | -H | 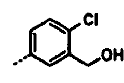 | 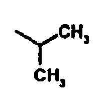 | 345 |
| **32** | 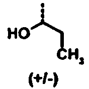 | -OH | -H | -H | 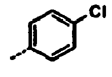 | 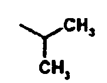 | 364 |
| **33** | 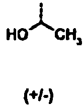 | -OH | -H | -H | 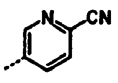 | 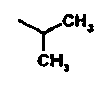 | 351 |
| **34** | 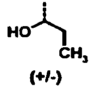 | -OH | -H | -H | 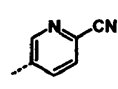 | 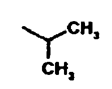 | 365 |
| **35** | 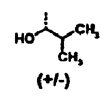 | -OH | -H | -H | 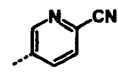 | 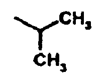 | 379 |
| **36** | 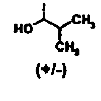 | -OH | -H | -H | 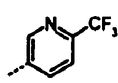 | 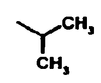 | - |
| **37** | 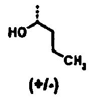 | -OH | -H | -H | 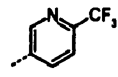 | 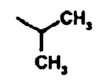 | 422 |
| **38** | 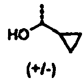 | -OH | -H | -H | 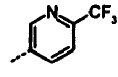 | 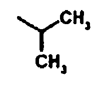 | 420 |
| **39** | 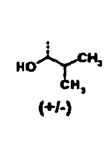 | -OH | -H | -H | 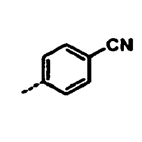 | 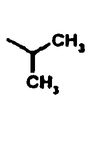 | 378 |
| **40** | 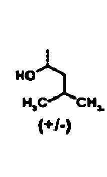 | -OH | -H | -H | 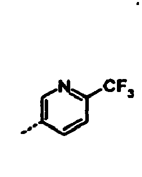 | 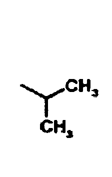 | 436 |
| **41** | 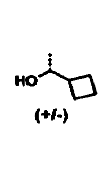 | -OH | -H | -H | 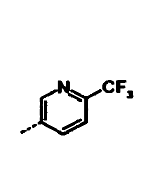 | 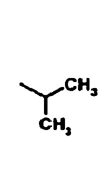 | - |
| **42** | 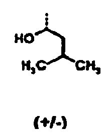 | -OH | -H | -H | 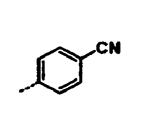 | 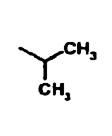 | 392 |
| **43** | 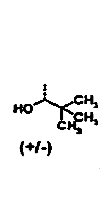 | -OH | -H | -H | 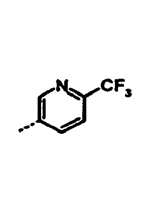 | 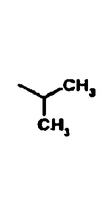 | 436 |
| **44** | 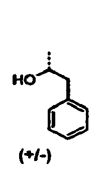 | -OH | -H | -H | 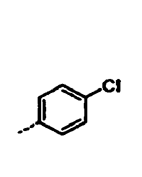 | 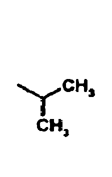 | 435 |
| **45** | 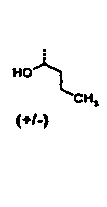 | -OH | -H | -H | 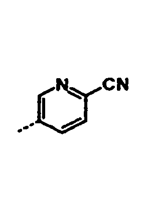 | 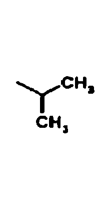 | 379 |
| **46** | 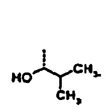 | -OH | -H | -H | | | 393 |
| | (+/-) | | | | 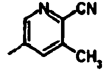 | 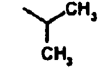 | |
| **47** | 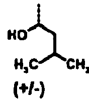 | -OH | -H | -H | 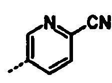 | 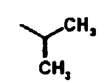 | 393 |
| **48** | 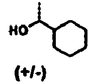 | -OH | -H | -H | 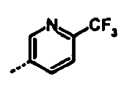 | 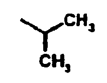 | - |
| **49** | 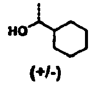 | -OH | -H | -H | 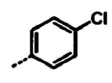 | 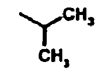 | 427 |
| **50** | 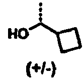 | -OH | -H | -H | 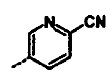 | 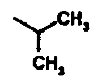 | 391 |
| **51** | 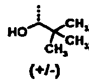 | -OH | -H | -H | 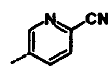 | 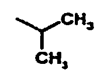 | 393 |
| **52** | 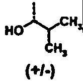 | -OH | -H | -H | 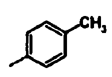 | 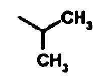 | 367 |
| **53** | 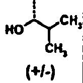 | -OH | -H | -H | 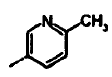 | 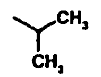 | 368 |
| **54** | 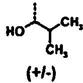 | -OH | -H | -H | 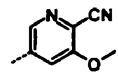 | 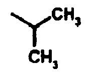 | 409 |
| **55** | 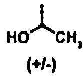 | -OH | -H | -H | 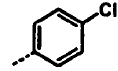 | 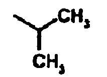 | 359 |
| **56** | 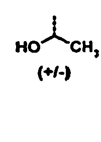 | -OH | -H | -H | 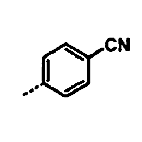 | 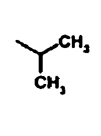 | 350 |
| **57a** | 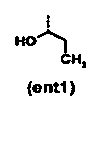 | -OH | -H | -H | 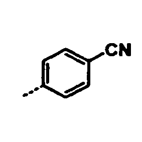 | 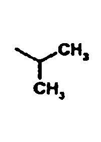 | 364 |
| **57b** | 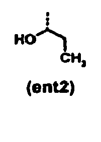 | -OH | -H | -H | 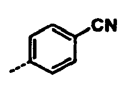 | 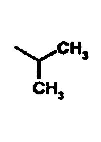 | 364 |
| **58** | 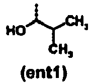 | -OH | -H | -H | 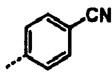 | 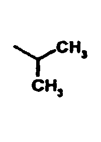 | 378 |
| **59** | 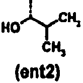 | -OH | -H | -H | 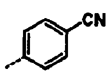 | 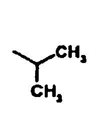 | 378 |
| **60** | 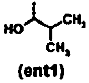 | -OH | -H | -H | 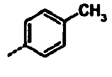 | 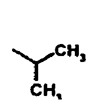 | 367 |
| **61** | 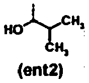 | -OH | -H | -H | 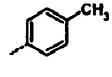 | 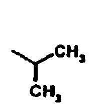 | 367 |
| **62** | 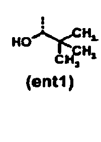 | -OH | -H | -H | 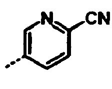 | 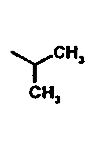 | 393 |
| **63** | 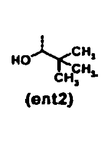 | -OH | -H | -H | 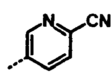 | 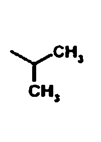 | 393 |
| **64** | 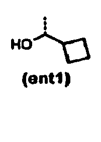 | -OH | -H | -H | 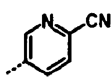 | 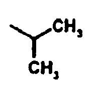 | 391 |
| **65** | 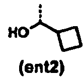 | -OH | -H | -H | 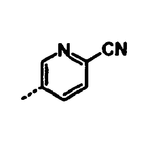 | 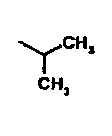 | 391 |
| **66** | 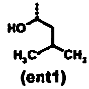 | -OH | -H | -H | 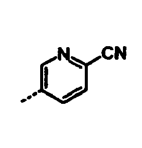 | 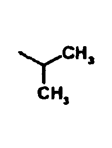 | 393 |
| **67** | 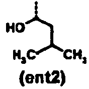 | -OH | -H | -H | 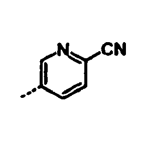 | 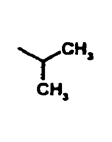 | 393 |
| **68** | 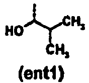 | -OH | -H | -H | 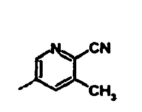 | 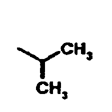 | 393 |
| **69** | 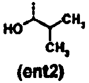 | -OH | -H | -H | 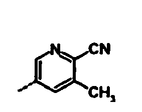 | 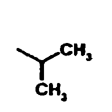 | 393 |
| **70** | 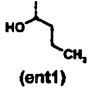 | -OH | -H | -H | 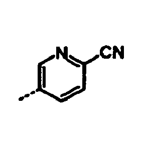 | 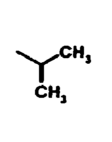 | 379 |
| **71** | 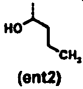 | -OH | -H | -H | 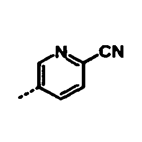 | 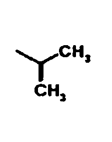 | 379 |
| **72** | 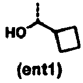 | -OH | -H | -H | 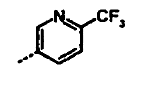 | 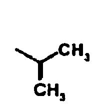 | 434 |
| **73** | 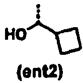 | -OH | -H | -H | 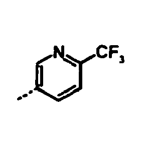 | 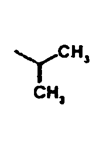 | 434 |
| **74** | 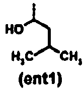 | -OH | -H | -H | 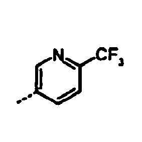 | 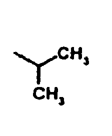 | 436 |
| **75** | 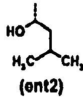 | -OH | -H | -H | 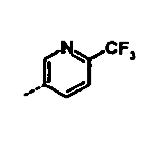 | 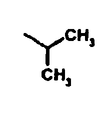 | 436 |
| **76** | 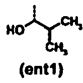 | -OH | -H | -H | 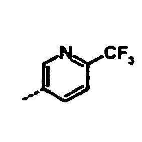 | 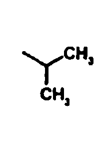 | 422 |
| **77** | 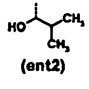 | -OH | -H | -H | 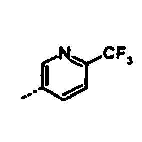 | 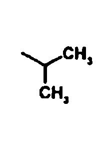 | 422 |
| **78** | 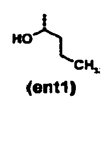 | -OH | -H | -H | 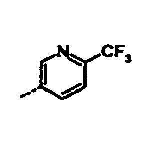 | 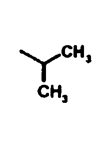 | 422 |
| **79** | 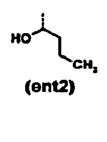 | -OH | -H | -H | 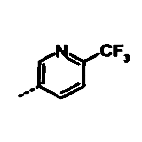 | 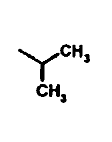 | 422 |
| **80** | 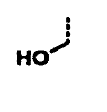 | -OH | -H | -H | 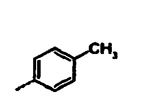 | 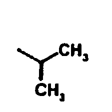 | 325 |
| **81** | 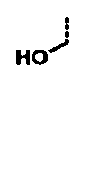 | -OH | -H | -H | 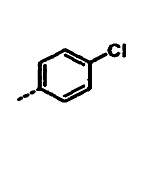 | 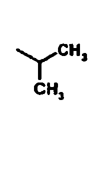 | 345 |
| **82** | 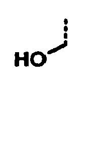 | -OH | -H | -H | 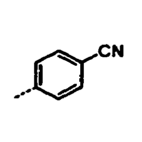 | 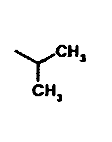 | 336 |
| **83** | 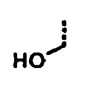 | -OH | -H | -H | 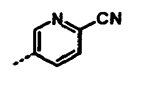 | 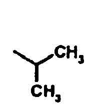 | 337 |
| **84** | 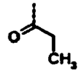 | -OH | -H | -H | 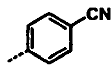 | 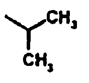 | 362 |
| **85** | 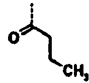 | -OH | -H | -H | 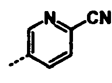 | 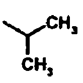 | 377 |
| **86** | 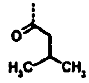 | -OH | -H | -H | 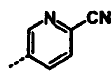 | 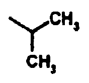 | 391 |
| **87** | 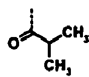 | -OH | -H | -H | 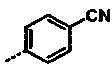 | 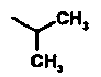 | 376 |
| **88** | 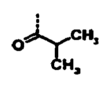 | -OH | -H | -H | 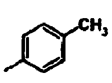 | 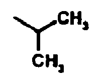 | 365 |
| **89** | 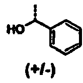 | -OH | -H | -H | 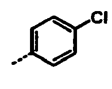 | 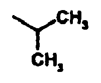 | 421 |
| **90** | 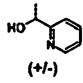 | -OH | -H | -H | 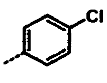 | 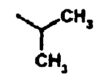 | 422 |
| **91** | 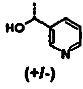 | -OH | -H | -H | 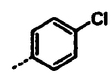 | 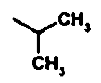 | 433 |
| **92** | 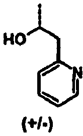 | -OH | -H | -H | 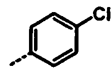 | 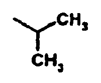 | 435 |
| **93** | 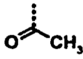 | -OH | -H | -H | | | 348 |
| | | | | | 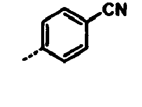 | 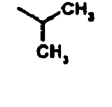 | |
| **94** | 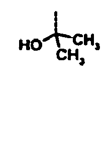 | -OH | -H | -H | 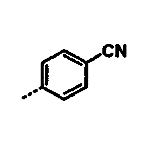 | 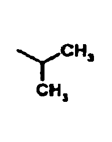 | 364 |
| **95** | 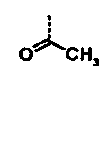 | -OH | -H | -H | 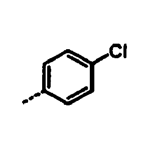 | 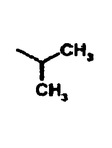 | 357 |
| **96** | 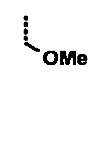 | -OH | -H | -H | 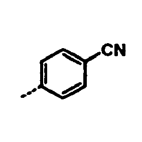 | 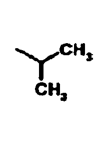 | 350 |
| **97** | 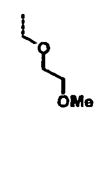 | -OH | -H | -H | 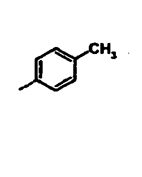 | 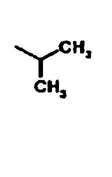 | 383 |
| **98** | 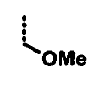 | -OH | -H | -H | 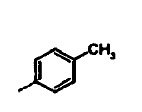 | 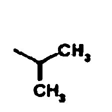 | 339 |
| **99** | 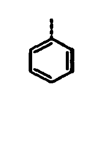 | -OH | -H | -H | 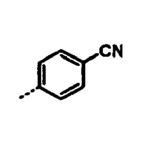 | 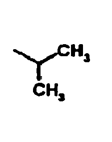 | 382 |
| **100** | 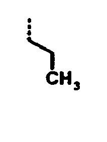 | -OH | -H | -H | 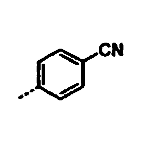 | 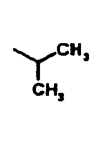 | 348 |
| **101** | 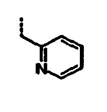 | -OH | -H | -H | 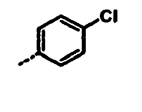 | 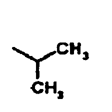 | 406 |
| **102** | -H | -OH | -H | -H | 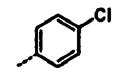 | 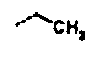 | 301 |
| **103** | -H | -OH | -H | -H | 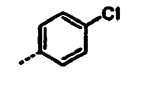 | 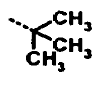 | 329 |
| **104** | -H | -OH | -H | -H | 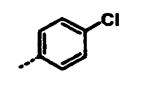 | 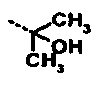 | 331 |
| **105** | -H | -OH | -H | -H | 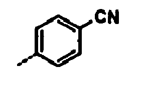 | 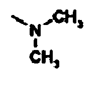 | 335 |
| **106** | -H | -OH | -H | -H | 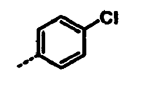 | 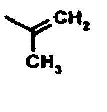 | 313 |
| **107** | -H | -H | -H | -OMe | 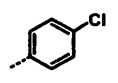 | 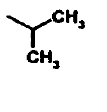 | 329 |
| **108** | -H | -OH | -H | -OH | 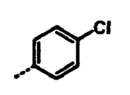 | 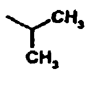 | 331 |
| **109** | -H | -OH | -OMe | -H | 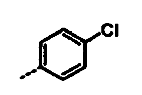 | 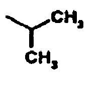 | 345 |
| **110** | -H | -OH | -OH | -H | 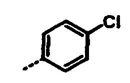 | 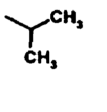 | 331 |
| **111** | -H | -NH₂ | -H | -H | 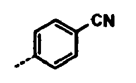 | 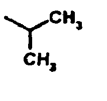 | 305 |

### Abbreviations

BEMP - 2-tert-butylimino-2-diethylamino-1,3- BEMP dimethylperhydro-1,3,2-diaza phophorine; BOP-Cl - bis(2-oxo-3-oxazolidinyl)phosphinic chloride; *n*-BuLi - *n-*butyl lithium; t-BuOH - t-butanol; *t*-BuOK- potassium tert-butoxide; DBU - 1,8-diazabicyclo[5.4.0]undec-7-ene; DCM - dichloromethane; DMAP - 4-dimethylaminopyridine; DMF - dimethylformamide; DMSO - dimethyl sulfoxide; DPPP - 1,3-bis(diphenylphosphine)propane; EtOAc - ethyl acetate; EtOH -ethanol; Et₂O -diethyl ether; EtgSiH - triethylsilane; HMDS - hexamethyl di-siiazane; HOBt - 1 - hydroxybenzotriazole monohydrate; HPLC - high performance liquid chromatography; IPA - isopropyl alcohol; MeOH - methanol; NEt₃ - triethylamine; PS-EDCI - polymer supported N-(3-dimethylamniopropyl)-N'-ethylcarbodiimide hydrochloride; TFA - trifluoroacetic acid; THF - tetrahydrofuran; TIPSCI - tripropylsilylchloride

### Preparation of Specific Examples - General experimental conditions

LCMS are recorded on an Agilent 1100 LC system with a Phenomenex Gemini C18 3.0 x 50 mm, 3 µM analytical column eluting with 5-95% acetonitrile + 0.1% NH₃ in water + 0.1 % NH₃ over 2.5 minutes, with negative ion electrospray ionization or 5-95% acetonitrile + 0.1% TFA in water +0.1% TFA over 2.5 minutes positive ion electrospray ionization. The mobile phase flow rate for LCMS experiments is 1 ml min-¹. [M+H]+ refers to monoisotopic molecular weights. Preparative chiral HPLC is carried out using a Chiralpak-AD, Chiralcel-OD or Chiralcel-OJ 25cm x 20mm, 10 µM semi-preparative column eluting with isocratic n-hexane:EtOH often with addition of 0.1% TFA to the mobile phase to improve selectivity. The separated enantiomeric pairs are arbitrarily assigned *ent1* (shorter retention time) and *ent2* (longer retention time) respectively. Microwave reactions are carried out using a Personal Chemistry Emrys Optimiser™ microwave reactor.

### Preparation of Intermediates:

### Intermediate A 2-Chloro-3-(4-chlorophenyl)-7-methoxy-3H-quinazolin-4-one

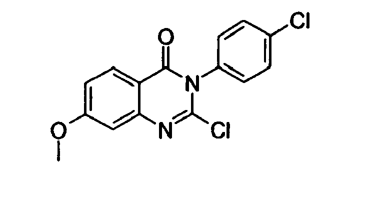

### A1) N-(4-Chloro-phenyl)-4-methoxy-2-nitro-benzamide:

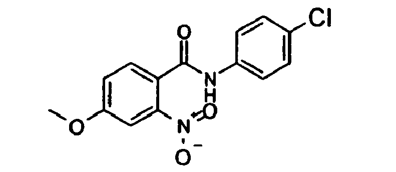

A suspension of 4-methoxy-2-nitro-benzoic acid (2.5 g, 12.68 mmol) and sulfuryl chloride (4.6 ml, 63.2 mmol) in toluene (100 ml) is stirred at 90°C for 1 hour. After cooling to room temperature, the solvent is removed *in vacuo* and the resulting solid is treated with 4-chloro-phenylamine (0.153 g, 1.19 mmol) in THF (100 ml) and then stirred at room temperature overnight. The mixture is partitioned between water and ethyl acetate and the organic portion is separated, dried (MgSO₄) and concentrated *in vacuo* afford the title compound as a brown solid. (MH+ 307)

### A2) 2-Amino-N-(4-chloro-phenyl)-4-methoxy-benzamide:

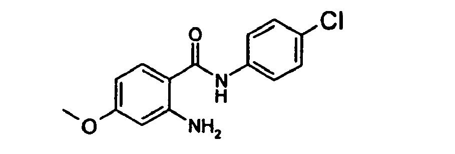

A suspension of N-(4-chloro-phenyl)-4-methoxy-2-nitro-benzamide (A1) (2.5 g, 8.15 mmol) and iron (1.9 g, of 315 mesh, 32.6 mmol) in glacial acetic acid (100 ml) is stirred at 60 °C for 20 minutes. After cooling to room temperature, the mixture is diluted with water and extracted with ethyl acetate (3 times). The combined organic extracts are dried (MgSO₄) and concentrated *in vacuo* to afford the title compound as a pale off white solid. (MH+ 277).

### A3) 3-(4-Chloro-phenyl)-7-methoxy-1H-quinazoline-2,4-dione:

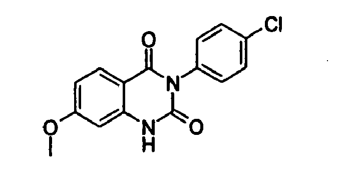

A suspension of 2-amino-N-(4-chloro-phenyl)-4-methoxy-benzamide (A2) (0.1 g, 0.36 mmol) in THF (4 ml) is treated with phosgene (510 □ of 20 %w/v phosgene in toluene, 1.04 mmol) and then heated using microwave radiation in a Personal Chemistry Emrys™ Optimizer microwave reactor at 100°C for 90 minutes. The resulting suspension is filtered to afford the title compound as a white solid. (MH+ 303.2).

### A4) 2-Chloro-3-(4-chlorophenyl)-7-methoxy-3H-quinazolin-4-one:

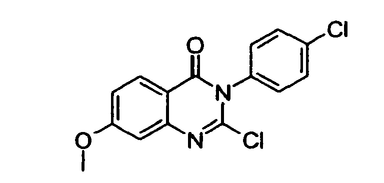

A suspension of 3-(4-chloro-phenyl)-7-methoxy-1H-quinazoline-2,4-dione (A3) (0.4 g, 1.32 mmol) in phosphorus oxychloride (20 ml) is heated using microwave radiation in a Personal Chemistry Emrys™ Optimizer microwave reactor at 120°C for 30 minutes followed by 150 °C for 60 minutes. The resulting mixture is concentrated *in vacuo* and the resulting crude product is triturated with dry diethyl ether to yield the title compound as a beige solid. (MH+ 321.1).

### Intermediate B 2-Methyl-5-triisopropylsilanyloxy-phenylamine

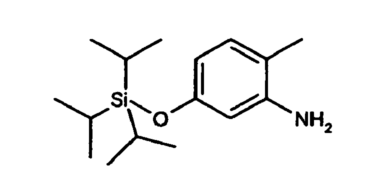

### B1) Triisopropyl-(4-methyl-3-nitro-phenoxy)-silane:

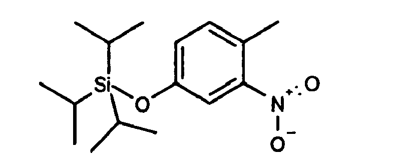

To a solution of 4-methyl-3-nitrophenol (10 g, 65.2 mmol) in DMF (30 ml) is added imidazole (8.89 g, 130 mmol). The solution is cooled (0 °C) and treated with a solution of triisopropylsilyl chloride (13.9 ml, 65.2 mmol) in DMF (10 ml) and stirred at room temperature overnight. The reaction mixture is poured onto water (100 ml) and extracted with diethyl ether (2 x 100 ml). The organic extracts are combined, washed with water (100 ml), citric acid (100 ml), brine (50 ml), dried (MgSO₄) and concentrated *in vacuo* to afford the title compound as a yellow oil. The crude product is carried through to the next step without further purification.

### B2) 2-Methyl-5-triisopropylsilanyloxy-phenylamine

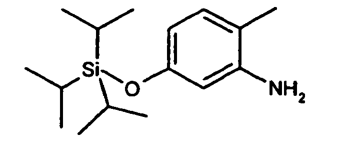

To a solution of triisopropyl-(4-methyl-3-nitro-phenoxy)-silane (B1) (5 g, 16.15 mmol) in ethanol (30 ml) is added tin (II) chloride (18.22 g, 80.7 mmol) and the resulting mixture is stirred at room temperature overnight. The reaction mixture is poured onto water and the pH is adjusted to pH 7-8 by addition of sodium hydrogen carbonate solution. An emulsion forms which is filtered through under vacuum and the product is extracted with ethyl acetate (3 x 100 ml). The organic portions are combined, washed with water (100 ml), brine (100 ml), dried (MgSO₄) and concentrated *in vacuo* to afford the title compound as a brown oil. The crude product is carried through to the next step without further purification.

### Intermediate C 2-Isobutyrylamino-4-triisopropylsilanyloxy-benzoic acid

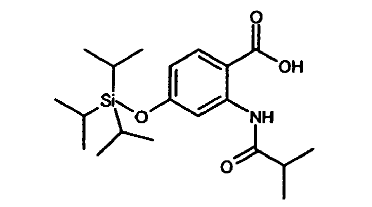

### C1) N-(2-Methyl-5-triisopropylsilanyloxy-phenyl)-isobutyramide:

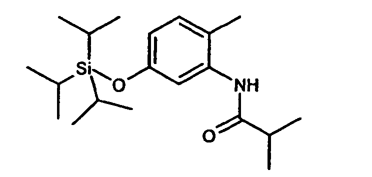

A solution comprising 2-methyl-5-triisopropylsilanyloxy-phenylamine (B2) (80 g, 0.286 mol) in DCM (500 ml) is treated with TEA (43.8 ml, 0.315 mol) and then dropwise with isobutyryl chloride (33 ml, 0.315 mol) over 30 minutes. The resulting mixture is stirred overnight and then washed with water (2 x 200 ml), brine (50 ml), dried (MgSO4) and concentrated *in vacuo.* The crude product is dissolved in a minimum volume of boiling *n*-hexane and then left to stand at room temperature for 2 days. The resulting suspension of product is filtered and washed with cold n-hexane to afford the title product as a colourless crystalline solid.

### C2) 2-isobutyrylamino-4-triisopropylsilanyloxy-benzoic acid:

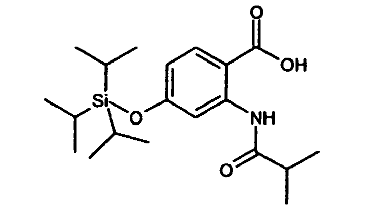

To a hot solution of N-(2-methyl-5-triisopropylsilanyloxy-phenyl)-isobutyramide (C1) (5 g, 14.3 mmol) in 2-methylpropan-2-ol/water (140 ml of a 1:1 mixture) is added carefully and portionwise potassium permanganate (11.3 g, 71.5 mmol) over 1 hour. The reaction mixture is allowed to cool to room temperature and stirred overnight.
The mixture is then partitioned between ethyl acetate (100 ml) and 2M HCl (100 ml) and stirred for 20 minutes before separating. The aqueous is extracted further with ethyl acetate (2 x 70 ml) and the combined organic portions are washed with water (100 ml), brine (100 ml), dried (MgSO₄) and concentrated *in vacuo* to afford the title compound. (MH+380.4)

### Intermediate D

### 4-Chloro-2-dimethylaminomethyl-phenylamine:

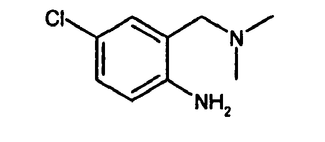

### D1) (5-Chloro-2-nitro-benzyl)-dimethyl-amine:

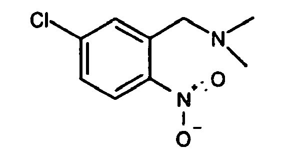

A solution of 5-chloro-2-nitro-benzaldehyde (1 g, 5.38 mmol) in THF (10 ml) is treated with 2M dimethylamine in THF (2.69 ml, 5.38 mmol). The solution is cooled (0 °C) and then sodium triacetoxyborohydride (1.59 g, 7.54 mmol) is carefully added. The reaction mixture is stirred overnight and then diluted with water (50 ml). The product is extracted with ethyl acetate (3 x 50 ml) and the combined organic portions are washed with water (50 ml), brine (50 ml), dried (MgSO₄) and concentrated *in vacuo* to afford the title compound as a yellow oil. (MH+ 215).

### D2) 4-Chloro-2-dimethylaminomethyl-phenylamine:

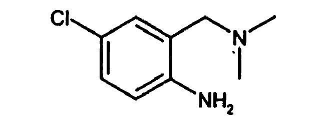

This compound is prepared analogously to 2-methyl-5-triisopropylsilanyloxy-phenylamine (B2) by replacing triisopropyl-(4-methyl-3-nitro-phenoxy)-silane (B1) with (5-chloro-2-nitro-benzyl)-dimethyl-amine (D1).

### Intermediate E

### 4-Chloro-3-dimethylaminomethyl-phenylamine:

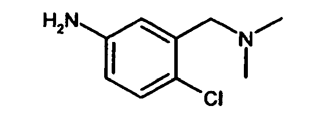

This compound is prepared analogously to 4-chloro-2-dimethylaminomethyl-phenylamine (intermediate D) by replacing 5-chloro-2-nitro-benzaldehyde with 2-chloro-5-nitro-benzaldehyde.

### Intermediate F

### 2-Chloro-3-methyl-5-pyridin-3-ylamine:

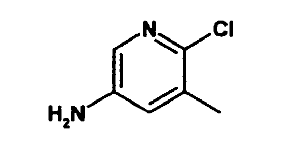

A mixture of 2-chloro-3-methyl-5-nitro-pyridine (1.0 g, 5.8 mmol) and tin (II) chloride dihydrate (6.5 g, 29.0 mmol) are refluxed in EtOH (50 ml) for 2 h. The reaction mixture is concentrated *in vacuo* then partitioned between CH₂Cl₂ and 2M NaOH. The organic phase is washed with water, brine and dried (MgSO₄). The organic phase is concentrated *in vacuo* to afford the title compound. (400MHz CDCl₃) 1 H nmr δ_{H} 7.72 (1H, d), 6.90 (1 H, d), 3.63 (2H, br s), 2.30 (3H, s).

### Intermediate G

### 5-Methyl-6-trifluoromethyl-pyridin-3-ylamine:

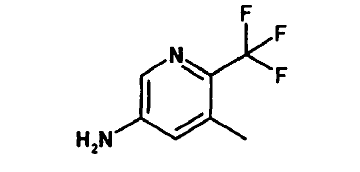

### G1) 3-Methyl-5-nitro-2-trifluoromethyl-pyridine:

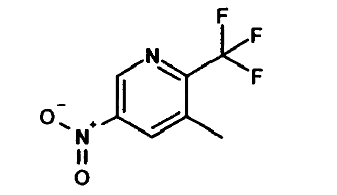

To a mixture of 2-chloro-3-methyl-5-nitropyridine (2.0 g, 11.6 mmol) and copper powder (4.4 g, 69.6 mmol) in dimethylacetamide (20 ml) is added dibromodifluoromethane (5 ml, 54.8 mmol). The reaction mixture is heated at 100 °C for 18 h. The reaction mixture is diluted with EtOAc and filtered. The filtrate is washed with water, brine and dried (MgSO₄) then concentrated *in vacuo*. The crude material is purified by flash chromatography on silica gel using iso-hexane : EtOAc (50: 1 to 25: 1) as the eluent to yield the title compound. (400MHz CDCl₃) 1H nmr δ_{H} 9.32 (1H, d), 8.49 (1 H, d), 2.69 (3H, s).

### G2) 5-Methyl-6-trifluoromethyl-pyridin-3-ylamine:

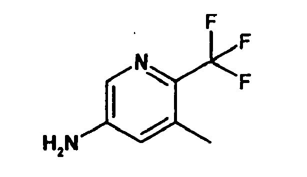

The title compound is prepared analogously to intermediate F by replacing 2-chloro-3-methyl-5-nitropyridine with 3-methyl-5-nitro-2-trifluoromethyl-pyridine (G1). 1 H nmr δ_{H} (400MHz CDCl₃) 7.93 (1H, d), 6.83 (1H, d), 3.95 (1H, bs), 2.40 (3H, s).

### Intermediate H

### 5-Methoxy-6-trifluoromethyl-pyridin-3-ylamine:

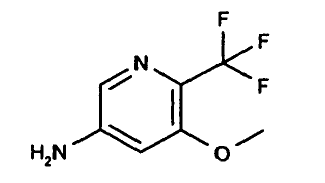

The title intermediate is prepared analogously to 5-methyl-6-trifluoromethyl-pyridin-3-ylamine (intermediate G) by replacing 2-chloro-3-methyl-5-nitropyridine with 2-chloro-3-methoxy-5-nitro-pyridine. 1 H nmr δ_{H} (400MHz CDCl₃) 7.70 (1 H, d), 6.60 (1H, d), 4.04 (1H, br s), 3.89 (3H, s).

### Intermediate I

### 5-Amino-3-methyl-pyridine-2-carbonitrile:

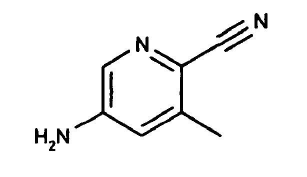

### I1) 3-Methyl-5-nitro-pyridine-2-carbonitrile:

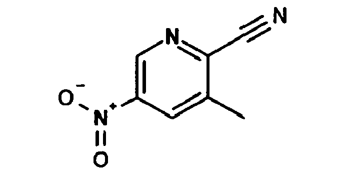

A mixture of 2-chloro-3-methyl-5-nitro-pyridine (1.0 g, 5.8 mmol) and copper (I) cyanide in dimethylacetamide (4 ml) in a microwave reaction vessel is heated in the microwave at 200 °C for 2 h. The reaction mixture is partitioned between EtOAc and water then filtered to remove insoluble material. The organic phase is washed with brine, dried (MgSO₄) and concentrated *in vacuo.* Purified by flash chromatography on silica gel using iso-hexane : EtOAc (10 : 1) as the eluent to yield the title compound. 1H nmr δ_{H} (400MHz CDCl₃) 9.37 (1H, d), 8.51 (1H, d), 2.73 (3H, s).

### I2) 5-Amino-3-methyl-pyridine-2-carbonitrile:

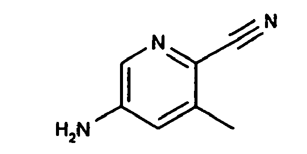

The title compound is prepared analogously to intermediate F by replacing 2-chloro-3-methyl-5-nitropyridine with 3-methyl-5-nitro-pyridine-2-carbonitrile (11). 1 H nmr δ_{H} (400MHz CDCl₃) 7.98 (1 H, d), 6.82 (1 H, d), 4.14 (2H, br s), 2.47 (3H, s).

### Intermediate J

### 5-Amino-3-methoxy-pyridine-2-carbonitrile:

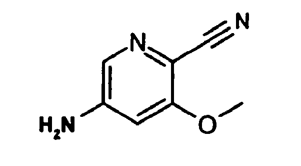

The title intermediate is prepared according to US 2004/0077605 A1 (p281) starting from 3-methoxypyridine. (400MHz CDCl₃) 1H nmr δ_{H} 7.72 (1H, d), 6.50 (1 H, d), 4.27 (2H, br s), 3.92 (3H, s).

### Intermediate K

### 5-Amino-3-methoxy-pyridine-2-carbonitrile:

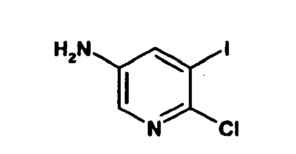

### K1) 2-Chloro-3-iodo-5-nitro-pyridine:

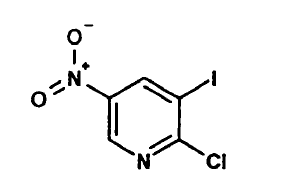

2-hydroxy-3-iodo-5-nitropyridine (2.0 g, 7.52 mmol) is added to a mixture of POCl₃ (0.7 ml, 7.52 mmol) and quinoline (0.44 ml, 3.76 mmol) at room temperature. The reaction mixture is heated at 120 °C for 2 h. The reaction mixture is then cooled to 96 ° C and is carefully quenched by the dropwise addition of water (32 ml). The reaction mixture is cooled to room temperature and filtered. The solid collected is dissolved in EtOAc and dried (MgSO₄) then concentrated *in vacuo* to yield the title compound.

### K2) 5-Amino-3-methoxy-pyridine-2-carbonitrile:

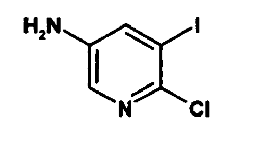

The title intermediate is prepared analogously to intermediate F by replacing 2-chloro-3-methyl-5-nitro-pyridine with 2-chloro-3-iodo-5-nitro-pyridine (K1).

### Intermediate L

### 4-Chloro-3-propoxy-phenylamine:

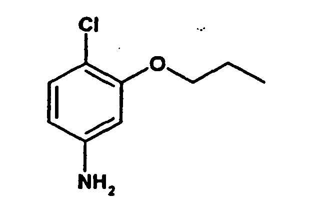

### L1) 1-Chloro-4-nitro-2-propoxy-benzene:

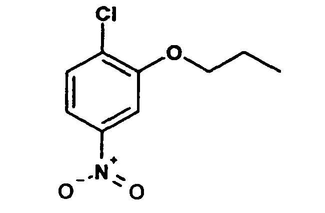

A mixture of 2-chloro-5-nitro-phenol (500 mg, 2.89 mmol), 1-bromopropane (355 mg, 2.89 mmol) and potassium carbonate (593 mg, 4.34 mmol) is heated in 2-butanone (10 ml) at 40°C for 2 h. The reaction mixture is diluted with 1 N NaOH (20 ml) and extracted with CH₂Cl₂ (2 x 40 ml). The organic phase is dried (MgSO₄) and concentrated *in vacuo.* to give the title compound.

### L2) 4-Chloro-3-propoxy-phenylamine:

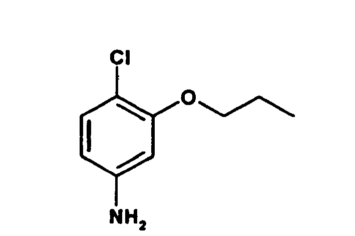

To a solution of 1-chloro-4-nitro-2-propoxy-benzene (L1) (500 mg, 2.33 mmol) in glacial acetic acid (30 ml) is added iron powder (384 mg, 6.98 mmol). The reaction mixture is heated at 60°C for 2 h. The reaction mixture is concentrated *in vacuo,* diluted with water (60 ml) and extracted with EtOAc (3 x 50 ml). The organics are combined, dried (MgSO₄) and concentrated *in vacuo* to yield the title compound. [M+H]+ 186.

### Intermediate M

### 3-(4-Chloro-3-chloromethyl-phenyl)-7-hydroxy-2-isopropyl-3H-quinazolin-4-one:

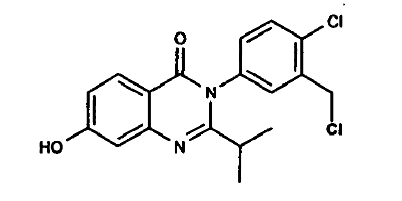

### M1) (5-Amino-2-chloro-phenyl)-methanol:

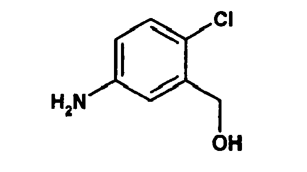

The title intermediate is prepared analogously to intermediate F by replacing 2-chloro-3-methyl-5-nitro-pyridine with (2-chloro-5-nitro-phenyl)-methanol.

### M2) 3-(4-Chloro-3-chloromethyl-phenyl)-7-hydroxy-2-isopropyl-3H-quinazolin-4-one:

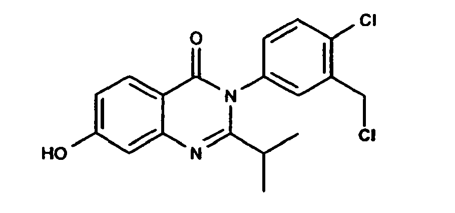

The title intermediate is prepared analogously to example 4 by replacing 4-chloro-3-fluoro-phenylamine with (5-amino-2-chloro-phenyl)-methanol (M1) to give the title compound.

### Intermediate N

### 2-pyridyllithium:

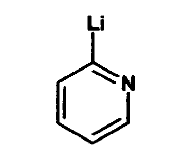

To a solution of 2-bromopyridine (30.5 µl, 0.32 mmol) in dry Et₂O (10 ml) at -78 °C is added dropwise n-BuLi (128 µl, 0.32 mmol; 2.5M in hexane) over 5 mins. The orange solution is stirred at -78 °C for 10 mins then used as a solution for subsequent reaction.

### Intermediate O

### 3-pyridyllithium:

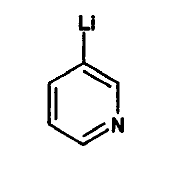

The title compound is prepared analogously to intermediate N by replacing 2-bromopyridine with 3-bromopyridine.

### Intermediate P

### 2-Amino-N-(4-chloro-phenyl)-4-methoxy-benzamide:

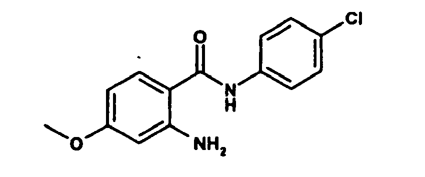

### P1) 4-Methoxy-2-nitro-benzoyl chloride:

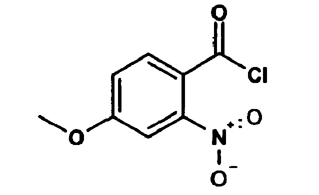

To a suspension of 4-methoxy-2-nitrobenzoic acid (8.0 g, 40.5 mmol) in CH₂Cl₂ (250 ml) containing DMF (2 drops) is added dropwise oxalyl chloride (3.8 ml, 44.6 mmol). The suspension is stirred at room temperature for 2 h. The reaction solvent is removed *in vacuo* to give the title compound as a white solid.

### P2) N-(4-Chloro-phenyl)-4-methoxy-2-nitro-benzamide:

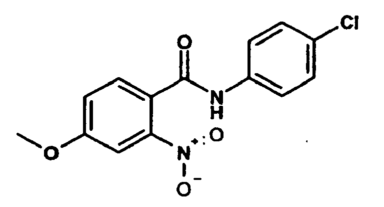

To a solution of 4-methoxy-2-nitro-benzoyl chloride (P1) (8.8 g, 40.8 mmol) in THF (200 ml) at room temperature is added 4-chloroaniline (5.73 g, 44.9 mmol). The reaction mixture is stirred at room temperature for 18 h. The reaction solvent is removed *in vacuo* to yield the title compound.

### P3) 2-Amino-N-(4-chloro-phenyl)-4-methoxy-benzamide:

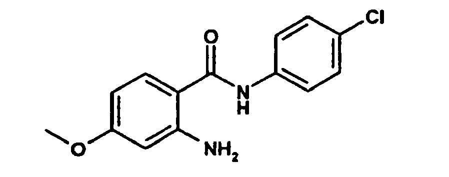

To a suspension of N-(4-chloro-phenyl)-4-methoxy-2-nitro-benzamide (P2) (7.8 g, 25.4 mmol) in EtOH (125 ml) is added tin (II) chloride dihydrate (28.7 g, 127 mmol). The reaction mixture is stirred at room temperature for 16 h. The reaction mixture is poured into water (350 ml) and basified to pH 7 with saturated NaHCO₃ solution. The suspension is left to settle and the supernatant liquid is decanted and extracted with EtOAc (2 x 200 ml). The organics are combined, dried (MgSO₄) and concentrated to dryness *in vacuo* to yield the title compound. [M+H]+ 277.

### Intermediate Q

### 2-(2,2-Dimethyl-propionylamino)-4-methoxy-benzoic acid:

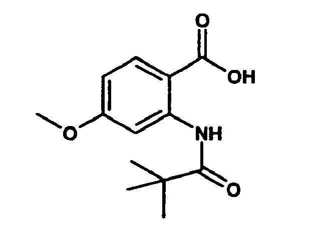

To a solution of 2-amino-4-methoxy-benzoic acid (500 mg, 2.9 mmol) and triethylamine (560 µl, 4 mmol) in CH₂Cl₂ (5 ml) is added dropwise pivaloyl chloride (370 µl, 4 mmol). The reaction mixture is stirred at room temperature for 16 h. The reaction mixture is poured into water (10 ml), the organic layer is separated and dried (MgSO₄). Concentrated *in vacuo.* to yield the title compound. [M+H]+ 252.

### Intermediate R

### 2-(2-Hydroxy-2-methyl-propionylamino)-4-triisopropylsilanyloxy-benzoic acid:

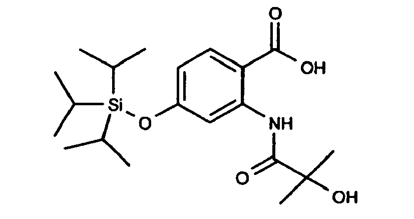

### R1) 2,2,2-Trifluoro-N-(2-methyl-5-triisopropylsilanyloxy-phenyl)-acetamide:

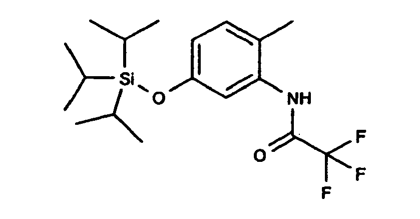

To a solution of 2-methyl-5-triisopropylsilanyloxy-phenylamine (B2) (7.35 g, 26.3 mmol) in CH₂Cl₂ (100 ml) is added pyridine (2.29 g, 29.0 mmol) at room temperature. The reaction mixture is cooled to 0 °C in an ice bath and trifluoroacetic anhydride (6.09 g, 29.0 mmol) is added dropwise. The reaction mixture is allowed to warm to room temperature with stirring over 1 h. The reaction mixture is diluted with water and extracted with CH₂Cl₂. The organic phase is washed with aqueous 0.1 M HCl, water, brine and dried (MgSO₄). Concentrated *in vacuo* then purified by flash chromatography on silica gel using iso-hexane : EtOAc (10 : 1) as the eluent to yield the title compound. (400MHz CDCl₃) 1 H nmr δ_{H} 7.64 (1 H, br s), 7.52 (1H, d), 7.07 (1H, d), 6.72 (1H, d of d), 2.23 (3H, s), 1.29 (3H, m), 1.10 18H, d).

### R2) 2-(2,2,2-Trifluoro-acetylamino)-4-triisopropylsilanyloxy-benzoic acid:

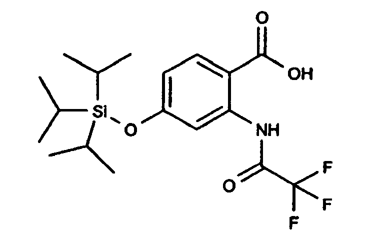

To a solution of 2,2,2-trifluoro-N-(2-methyl-5-triisopropylsilanyloxy-phenyl)-acetamide (8.0 g, 21.3 mmol) (R1) in t-BuOH (125 ml) and water (100 ml) cooled to 0 °C in an ice bath is added portionwise KMnO₄ (16.8 g, 107 mmol). The reaction mixture is allowed to warm up to room temperature with stirring for 16 h. The reaction mixture is treated with 2M HCl (200 ml) and stirred at room temperature for 10 mins. Diluted with EtOAc (400 ml) and filtered through celite™ (filter agent). The organic phase is separated, washed with water, brine and dried (MgSO₄). The solvent is removed *in vacuo* to yield the title compound. (400MHz CDCl₃) 1H nmr δ_{H} 12.15 (1 H, s), 8.29 (1H, d), 8.10 (1H, d), 6.78 (1 H, d of d), 1.35 (3H, m), 1.15 (18H, d).

### R3) 2-Amino-4-triisopropylsilanyloxy-benzoic acid:

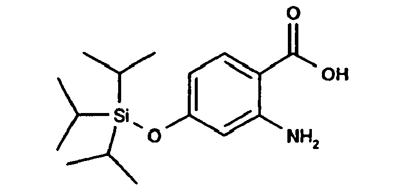

To a solution of 2-(2,2,2-trifluoro-acetylamino)-4-triisopropylsilanyloxy-benzoic acid (6.60 g, 16.3 mmol) (R2) in MeOH (50 ml) at room temperature is added 10% aqueous K₂CO₃ solution (10 ml). The reaction mixture is stirred at room temperature for 16 h. Further aqueous 10% K₂CO₃ solution (20 ml) is added and the reaction mixture is heated at 50 °C for 6 h. The solution is neutralised to pH 7 with 2M HCl and extracted with EtOAc. The organic phase is washed with water, brine and dried (MgSO₄). The solvent is removed *in vacuo* to yield the title compound. (400MHz CDCl₃) 1 H nmr δ_{H} 7.82 (1H, d), 6.24 (1 H, d of d), 6.15 (1H, d), 1.30 (3H, s), 1.12 (18H, d).

### R4) 2-(2-Acetoxy-2-methyl-propionylamino)-4-triisopropylsilanyloxy-benzoic acid:

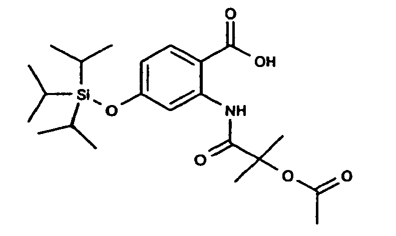

To a solution of 2-amino-4-triisopropylsilanyloxy-benzoic acid (550 mg, 1.78 mmol) (R3) in dry pyridine (5 ml) is added dropwise a solution of 1-chlorocarbonyl-1-methylethyl acetate (293 mg, 1.78 mmol) in CH₂Cl₂ (2 ml). The reaction mixture is stirred at room temperature for 30 min. The reaction mixture is partitioned between CH₂Cl₂ and 1 M HCl. The organic phase is washed with water, brine and dried (MgSO₄). Concentrated *in vacuo* then purified by flash chromatography on silica gel using iso-hexane : EtOAc (2 : 1) as the eluent. (400MHz CDCl₃) 1 H nmr δ_{H} 12.52 (1H, s), 8.40 (1 H, d), 8.00 (1 H, d), 6.61 (1H, d of d), 2.20 (3H, s), 1.75 (6H, s), 1.33 (3H, m), 1.13 (18H, d).

### R5) 2-(2-Hydroxy-2-methyl-propionylamino)-4-triisopropylsilanyloxy-benzoic acid:

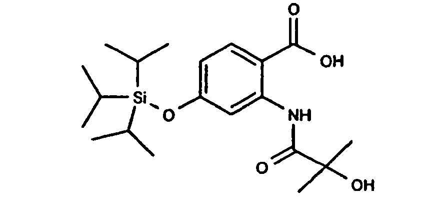

To a solution of 2-(2-acetoxy-2-methyl-propionylamino)-4-triisopropylsilanyloxy-benzoic acid (300 mg, 0.69 mmol) (R4) in MeOH (10 ml) is added K₂CO₃(284 mg, 2.06 mmol) and the reaction mixture is stirred at room temperature for 2 h. The reaction mixture is partitioned between EtOAc and 1M HCl. The organic phase is washed with water, brine and dried (MgSO₄) The solvent is removed *in vacuo* and the crude product is triturated with iso-hexane : EtOAc (3 : 1) to yield the title compound. [M+H]+ 396.

### Intermediate S

### 2-Amino-N-(4-cyano-phenyl)-4-methoxy-benzamide:

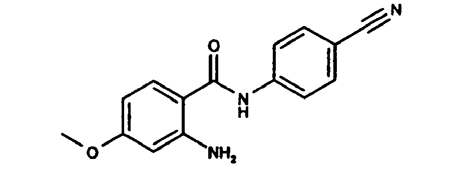

### S1) 4-Methoxy-2-nitro-benzoyl chloride:

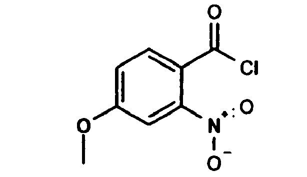

To a solution of 4-methoxy-2-nitro-benzoic acid (10.0 g, 51 mmol) in CH₂Cl₂ (500 ml) is added dropwise oxalyl chloride (4.8 ml, 54 mmol) followed by DMF (1 ml). The reaction mixture is stirred at room temperature for 1 h. The reaction solvent is removed *in vacuo* to yield the title compound.

### S2) N-(4-Cyano-phenyl)-4-methoxy-2-nitro-benzamide:

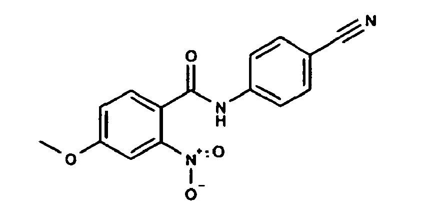

To a solution of 4-methoxy-2-nitro-benzoyl chloride (5.11 g, 23.7 mmol) (S1) in CH₂Cl₂ (200 ml) cooled to 0 °C is added 4-aminobenzonitrile (2.94 g, 24.9 mmol) followed by triethylamine (6.9 ml, 50 mmol). The reaction mixture is stirred at room temperature for 16 h then concentrated *in vacuo.* The residue is dissolved in EtOAc, washed with 2M HCl, water, followed by saturated NaHCO₃ solution.

### S3) 2-Amino-N-(4-cyano-phenyl)-4-methoxy-benzamide:

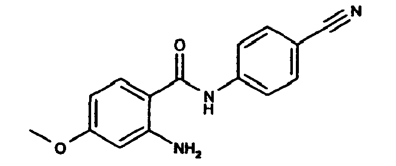

To a solution of N-(4-cyano-phenyl)-4-methoxy-2-nitro-benzamide (S2) (6.57 g, 22 mmol) in MeOH (250 ml) is added ammonium formate (13.86 g, 220 mmol) followed by 10% palladium on carbon. The reaction mixture is heated to reflux then stirred at room temperature for 1 h. The reaction mixture is filtered through Celite™ (filter agent) and concentrated *in vacuo.* The residue is dissolved in EtOAc, washed with water, dried (MgSO₄) and concentrated *in vacuo.* Purification is carried out using an Isolute™ SCX (cation exchange) cartridge eluting with 2N NH₃/MeOH to yield the title compound.

### Intermediate T

### N-(4-Chloro-phenyl)-2-(2-methyl-acryloylamino)-4-triisopropylsilanyloxy-benzamide:

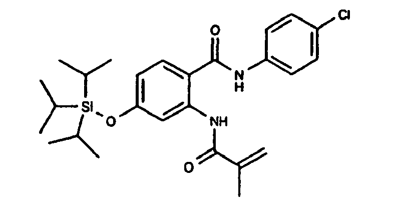

### T1) 4-Hydroxy-2-nitro-benzoic acid:

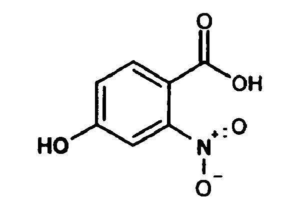

A suspension of 4-methoxy-2-nitro-benzoic acid (10 g, 50.72 mmol) in 48% aqueous HBr (100 ml) and glacial acetic acid (100 ml) is heated at 130°C for 16 h. The reaction mixture is then heated at 150°C for 6 h. The reaction mixture is partially concentrated *in vacuo,* filtered and dissolved in EtOAc. Washed with saturated NaHCO₃ (100 ml), brine (100 ml) and dried (MgSO₄). Concentrated *in vacuo* to give the title compound as a white solid.

### T2) 4-Hydroxy-2-nitro-benzoyl chloride:

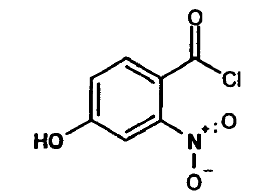

The title compound is prepared analogously to intermediate S1 by replacing 4-methoxy-2-nitro-benzoic acid with -hydroxy-2-nitro-benzoic acid (T1).The reaction mixture is concentrated *in vacuo* and used crude in the next step..

### T3) N-(4-Chloro-phenyl)-4-hydroxy-2-nitro-benzamide: E-11961-127 DB

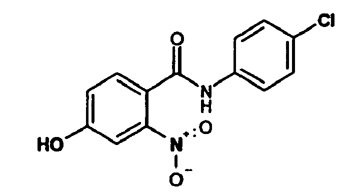

To a solution of 4-chloraniline (2.1 g, 16.4 mmol) in CH₂Cl₂ (25 ml) is added slowly a solution of 4-hydroxy-2-nitro-benzoyl chloride (3.3 g, 16.4 mmol) (T2) in CH₂Cl₂. Triethylamine (1.66 g, 16.4mmol) is added and the reaction mixture is stirred at room temperature for 1 h. The reaction mixture is diluted with CH₂Cl₂ and water, then filtered through Celite™ (filter agent). The organic phase is washed with water, brine and dried (MgSO₄) Concentrated *in vacuo* to give the title compound.

### T4) N-(4-Chloro-phenyl)-2-nitro-4-triisopropylsilanyloxy-benzamide:

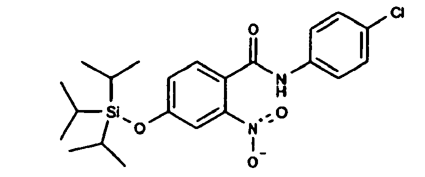

To a solution of N-(4-chloro-phenyl)-4-hydroxy-2-nitro-benzamide (2.4 g, 8.2 mmol) (T3) and imidazole (1.12 g, 16.4 mmol) in dry DMF (20 ml) at room temperature is added slowly TIPSCI (1.58 g, 8.2 mmol). The reaction mixture is stirred at room temperature for 2 h. The reaction mixture is partitioned between Et₂O and 0.5M HCl. The organic phase is washed with water, brine and dried (MgSO₄) Concentrated *in vacuo* and purified by flash chromatography on silica gel using iso-hexane : EtOAc (10 : 1) as the eluent to give the title compound. (400MHz CDCl₃) 1 H nmr δ_{H} 7.58 (2H, d), 7.51 (1H, d), 7.47 (1 H, d), 7.37 (1 H, d), 7.20 (1H, d of d), 1.32 (3H, m), 1.14 (18H, d).

### T5) 2-Amino-N-(4-chloro-phenyl)-4-triisopropylsilanyloxy-benzamide:

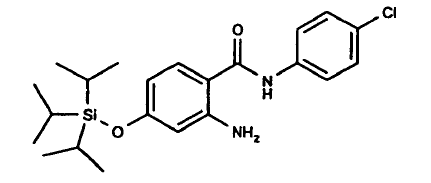

To a solution of N-(4-chloro-phenyl)-2-nitro-4-triisopropylsilanyloxy-benzamide (1.2 g, 2.68 mmol) (T4) in EtOH (50 ml) is added tin (II) chloride dihydrate (3.0 g, 13.4 mmol). The reaction mixture is refluxed for 2 h. The solvent is removed *in vacuo* and the residue is partitioned between CH₂Cl₂ and 2M NaOH. The organic phase is washed with water, brine and dried (MgSO₄). Concentrated *in vacuo* and purified by flash chromatography using iswo-hexane : EtOAc (10: 1) as the eluent. (400MHz CDCl₃) 1 H nmr δ_{H} 7.62 (1H, s), 7.51 (2H, d), 7.34 (3H, m), 6.27 (1H, d of d), 6.22 (1H, d), 5.61 (2h, s), 1.29 (3H, m), 1.13 (18H, d).

### T6) N-(4-Chloro-phenyl)-2-(2-methyl-acryloylamino)-4-triisopropylsilanyloxy-benzamide:

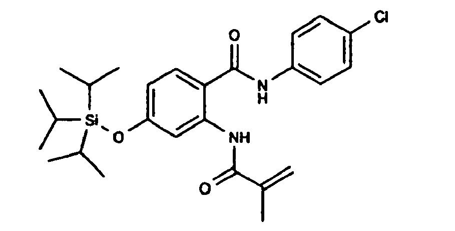

To a solution of 2-amino-N-(4-chloro-phenyl)-4-triisopropylsilanyloxy-benzamide (100 mg, 0.24 mmol) (T5) and triethylamine (48 mg, 0.48 mmol) in CH₂Cl₂ (10 ml) is added dropwise methacryloyl chloride (37 mg, 0.36 mmol). The reaction mixture is stirred at room temperature for 30 min. The reaction mixture is diluted with CH₂Cl₂, washed with 1 M HCl, water, saturated NaHCO₃, brine and dried (MgSO₄). Concentrated *in vacuo* to give the title compound. (400MHz CDCl₃) 1 H nmr δ_{H} 11.60 (1H, s), 8.40 (1 H, d), 7.77 (1 H, s), 7.53 (3H, m), 7.38 (2H, d), 6.64 (1 H, d of d), 6.00 (1H, s), 5.51 (1H, s), 2.10 (3H, s), 1.32 (3H, m), 1.13 (18H, d).

### Intermediate U

### 2-Isobutyrylamino-6-methoxy-benzoic acid:

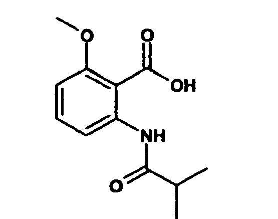

The title intermediate is prepared analogously to intermediate Q by replacing 2-amino-4-methoxy-benzoic acid with 2-amino-6-methoxy-benzoic acid and pivaloyl chloride with isobutyryl chloride respectively.

### Intermediate V

### 2-Amino-N-(4-chloro-phenyl)-4,6-dimethoxy-benzamide:

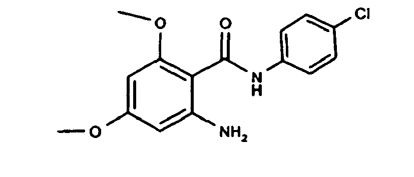

### V1) 2-Amino-4,6-dimethoxy benzoic acid:

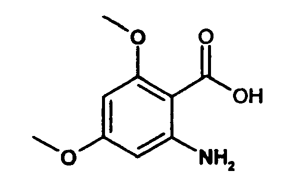

To a solution of 2,4-dimethoxy-6-nitro-benzoic acid (4.0 g, 17.62 mmol) in MeOH (40 ml) and DMF (4 ml) is added 10% palladium on carbon (0.8 g). The reaction mixture is subjected to catalytic hydrogenation (0.35 bar) for 24 h at room temperature. The reaction mixture is filtered through Celite^{™} (filter agent) and the filtrate is concentrated to dryness *in vacuo* to yield the title compound. [M+H] 198.

### V2) 5,7-Dimethoxy-1H-benzo[d][1,3]oxazine-2,4-dione:

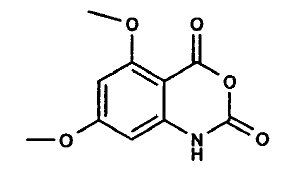

To a solution of 2-amino-4,6-dimethoxy-benzoic acid (4.0 g, 20.3 mmol) (V1) in THF (60 ml) under nitrogen cooled to 0 °C is added triphosgene (1.8 g, 6.1 mmol). The reaction mixture is allowed to warm up to room temperature with stirring for 2 h. The reaction mixture is poured slowly onto an ice-water mixture (70 ml) then filtered and washed with water to yield the title compound. [M+H]+ 224.

### V3) 2-Amino-N-(4-chloro-phenyl)-4,6-dimethoxy-benzamide:

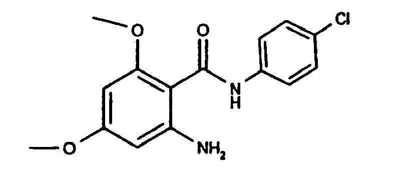

To a solution of 5,7-dimethoxy-1H-benzo[d][1,3]oxazine-2,4-dione (500 mg, 2.2 mmol) (V2) in dimethylacetamide (5 ml) is added DMAP (26.8 mg, 0.22 mmol) followed by 4-chloroaniline (711 mg, 5.6 mmol). The reaction mixture is heated at 110°C for 16 h. The reaction mixture is diluted with water (50 ml), extracted with EtOAc, washed with brine and dried (MgSO₄). Concentrated *in vacuo* and dried under high vacuum for 16 h to yield the title compound. [M+H]+ 307.

### Intermediate W

### 4-Hydroxy-2-isobutyrylamino-5-methoxy-benzoic acid:

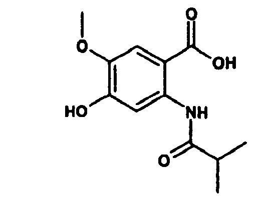

### W1) 2-Amino-4-hydroxy-5-methoxy-benzoic acid:

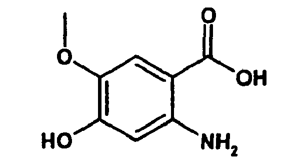

A solution of 4-benzyl-5-methoxy-2-nitro-benzoic acid (5.0 g, 16.48 mmol) in MeOH (100 ml) containing 10% palladium on carbon (0.5 g) is subjected to catalytic hydrogenation (0.35 bar) at room temperature for 24 h. The reaction mixture is filtered through Celite^{™} (filter agent), washing with MeOH. The filtrate is concentrated to dryness *in vacuo* to yield the title compound. [M+H]+ 184.

### W2) 4-Hydroxy-2-isobutyrylamino-5-methoxy-benzoic acid:

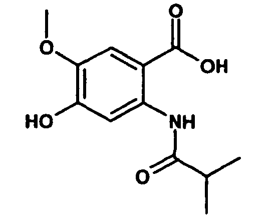

The title intermediate is prepared analogously to intermediate Q by replacing 2-amino-4-methoxy-benzoic acid with 2-amino-4-hydroxy-5-methoxy-benzoic acid (W1).

### PREPARATION OF EXAMPLES

### Example 1

### 3-(4-Chloro-phenyl)-2-diethylamino-7-hydroxy-3H-quinazolin-4-one

### 1a) 3-(4-Chloro-phenyl)-2-diethylamino-7-methoxy-3H-quinazolin-4-one:

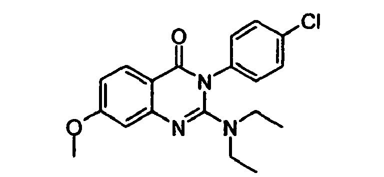

A suspension of 2-chloro-3-(4-chlorophenyl)-7-methoxy-3H-quinazolin-4-one (Intermediate A) (0.113 g, 0.353 mmol) in diethylamine (1.5 ml) and THF (0.5 ml) is heated using microwave radiation in a Personal Chemistry Emrys™ Optimizer microwave reactor at 150°C for 2 hours. The reaction mixture is diluted with saturated sodium hydrogen carbonate solution and ethyl acetate and stirred until all the solid has dissolved. The organic layer is separated and the aqueous portion is extracted with ethyl acetate. The combined organic extracts are dried (MgSO₄) and concentrated *in vacuo* to afford the crude product which is recrystallised from ethyl acetate to remove unreacted starting material. The solid is further purified by flash chromatography on silica eluting with a solvent gradient of dichloromethane:ethyl acetate (100:0, by volume) changing to dichloromethane:ethyl acetate (90:10, by volume), to yield the title compound.

### 1b) 3-(4-Chloro-phenyl)-2-diethylamino-7-hydroxy-3H-quinazolin-4-one:

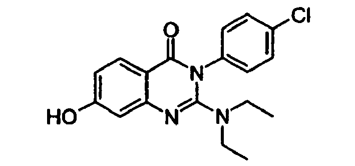

A solution of 3-(4-chloro-phenyl)-2-diethylamino-7-methoxy-3H-quinazolin-4-one (0.057 g, 0.161 mmol) in HBr (4 ml of a 47% aqueous solution) is heated to 130 °C for 5 hours and then allowed to cool to room temperature overnight. The crude suspension is poured onto saturated aqueous sodium hydrogen carbonate solution and extracted twice with ethyl acetate. The combined organic extracts are dried (MgSO₄) and concentrated *in vacuo.* The crude residue is purified by flash chromatography on silica eluting with a solvent gradient of dichloromethane:ethyl acetate (95:5, by volume) changing to dichloromethane:ethyl acetate (50:50, by volume), to yield the title compound as a white crystalline solid. (MH+ 344.2).

### Example 2

### {(R)-1-[3-(4-Chloro-phenyl)-7-hydroxy-4-oxo-3,4-dihydro -quinazolin-2-yl]-ethyl}-carbamic acid benzyl ester

### 2a) [(R)-1-(2-Methyl-5-triisopropylsilanyloxy-phenylcarbamoyl)-ethyl]-carb amic acid benzyl ester:

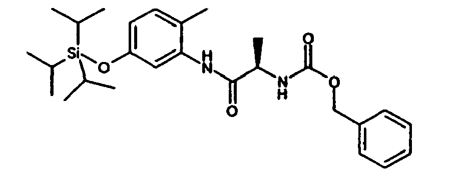

To a solution of (R)-2-benzyloxycarbonylamino-propionic acid (3.19 g, 14.3 mmol) in DCM (200 ml) is added polymer supported EDCl (20.7 g, 28.6 mmol) followed by HOBt (2.18 g, 14.3 mmol). The mixture is stirred gently for 30 minutes and then treated with a solution of 2-methyl-5-triisopropylsilanyloxy-phenylamine (Intermediate B) (4 g, 14.3 mmol) in DCM (10 ml). The reaction mixture is left to stir gently for 3 days and then filtered to remove the poymer supported resin. The resin is washed with DCM (100 ml) and MeOH (100 ml) and the filtrate is concentrated *in vacuo.* The crude residue is dissolved in DCM (100 ml) and washed with water (100 ml), brine (100 ml), dried (MgSO₄) and concentrated *in vacuo* to yield the title compound as a dark red oil. (MH+ 485.5)

### 2b) 2-((R)-2-Benzyloxycarbonylamino-propionylamino)-4-triisopropylsilanyloxy-benzoic acid:

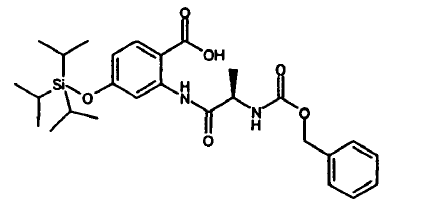

This compound is prepared analogously to 2-isobutyrylamino-4-triisopropylsilanyloxy-benzoic acid (Intermediate C) by replacing N-(2-methyl-5-triisopropylsilanyloxy-phenyl)-isobutyramide (C1) with [(R)-1-(2-methyl-5-triisopropylsilanyloxy-phenylcarbamoyl)-ethyl]-carbamic acid benzyl ester (2a).

### 2c) {(R)-1-[3-(4-Chloro-phenyl)-4-oxo-7-triisopropylsilanyloxy-3,4-dihydro -quinazolin-2-yl]-ethyl}-carbamic acid benzyl ester:

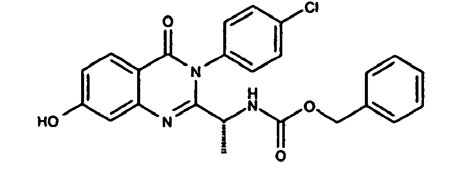

2-((R)-2-Benzyloxycarbonylamino-propionylamino)-4-triisopropylsilanyloxy-benzoic acid (0.105 g, 0.204 mmol) and 4-chloro-phenylamine (31.2 mg, 0.244 mmol) in MeCN (3 ml) is treated with phosphorus trichloride (71.1 □, 0.816 mmol) and then heated using microwave radiation in a Personal Chemistry Emrys™ Optimizer microwave reactor at 100°C for 60 minutes. After standing at room temperature for 2 days, the reaction mixture is poured onto water and extracted with ethyl acetate. The combined organic portions are washed with sodium hydrogencarbonate solution, brine, dried (MgSO₄) and concentrated *in vacuo* to afford a white solid. The solid is washed with hexane and filtered to yield the title compound.

### Example 3

### 3-(4-Chloro-phenyl)-7-hydroxy-2-isopropyl-3H-quinazolin-4-one

### 3a) 2-Isobutyrylamino-4-nitro-benzoic acid:

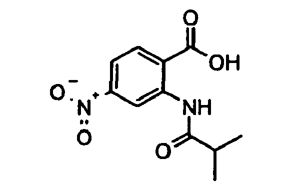

To a cooled (0 °C) solution of 2-amino-4-nitro-benzoic acid (25 g, 0.137 mol) in DCM (500 ml) is added TEA (42.0 ml, 0.302 mol) followed dropwise by isobutyryl chloride (173 ml, 0.166 mol). After stirring at room temperature overnight, the reaction mixture is washed with sodium bicarbonate solution (200 ml), ammonium chloride solution (200 ml) and brine (200 ml). The organic portion is dried (MgSO₄) and concentrated *in vacuo* to afford the crude product which is purified by flash chromatography on silica eluting with a solvent gradient of ethyl acetate:methanol (100:0, by volume) changing to ethyl acetate:methanol (90:10, by volume). The resulting solid is dissolved in ethyl acetate (50 ml) and washed with 1 M HCl (20 ml), dried (MgSO₄) and concentrated *in vacuo* to yield the title compound. (MH+253.0).

### 3b) 3-(4-Chloro-phenyl)-2-isopropyl-7-nitro-3H-quinazolin-4-one:

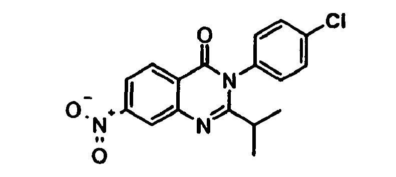

A stirred solution of 2-isobutyrylamino-4-nitro-benzoic acid (21.3 g, 0.085 mol) in MeCN ( 290 ml) is treated with 4-chloro-phenylamine (12.96 g, 0.10 mol) followed by further MeCN (100 ml). After stirring at room temperature for 30 minutes, phosphorus trichloride (22 ml, 0.25 mol) is added dropwise over 15 minutes and then, the reaction mixture is heated to 70°C for 90 minutes. The solvent is removed *in vacuo* and the residue is partitioned between ethyl acetate (300 ml) and saturated sodium bicarbonate solution (200 ml). The organic portion is separated, dried (MgSO₄) and concentrated *in vacuo.* The residue is washed with isopropyl ether (100 ml) and filtered to afford the title compound. (MH+344.20).

### 3c) 7-Amino-3-(4-chloro-phenyl)-2-isopropyl-3H-quinazolin-4-one

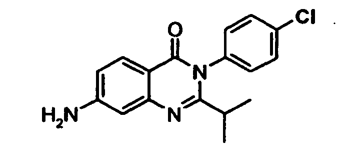

A solution of 3-(4-chloro-phenyl)-2-isopropyl-7-nitro-3H-quinazolin-4-one (26.04 g, 0.076 mol) in glacial acetic acid (775 ml) is treated with iron powder (19.05 g, 0.34 mol) and stirred at 60°C for 1 hour. After cooling to room temperature, 2M HCl is added to quench any remaining iron powder. The solvent is removed *in vacuo* and a mixture is 1: 1 water: 2M HCl (500 ml) is added to the resulting residue. The mixture is extracted with ethyl acetate (3 x 500 ml) and the combined organic extracts are dried (MgSO₄) and concentrated *in vacuo.* The residue is washed with isopropyl ether (100 ml) and filtered to afford the title compound. (MH+ 300.2)

### 3d) 3-(4-Chloro-phenyl)-7-hydroxy-2-isopropyl-3H-quinazolin-4-one

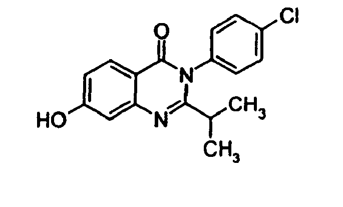

A cooled (0°C) mixture comprising 7-amino-3-(4-chloro-phenyl)-2-isopropyl-3H-quinazolin-4-one (18.57 g, 0.059 mol) in concentrated sulphuric acid/water (59 ml of a 2:3 mixture) is added dropwise a solution of sodium nitrite (6.07 g, 0.089 mol) in water (16.7 ml) ensuring the temperature did not rise above 5°C. The mixture is stirred for 45 minutes and then treated slowly with concentrated sulphuric acid/water (71 ml acid/46 ml water). The reaction mixture is stirred and heated to 150°C for 2 hours and then allowed to cool to room temperature. A solution of NaOH (71 g, in 300 ml water) is added to neutralise the mixture which is extracted with ethyl acetate (3 x 250 ml). The organic extracts are combined, dried (MgSO₄) and concentrated *in vacuo.* The residue is washed with isopropyl ether (100 ml) and filtered to afford the title compound. (MH+ 301).

### Example 4

### 3-(4-Chloro-3-fluoro-phenyl)-7-hydroxy-2-isopropyl-3H-quinazolin-4-one

2-Isobutyrylamino-4-triisopropylsilanyloxy-benzoic acid (Intermediate C) (0.5 g, 1.32 mmol) and 4-chloro-3-fluoro-phenylamine (0.21 g, 1.45 mmol) in MeCN (4 ml) is treated with phosphorus trichloride (0.23 ml, 2.6 mmol) and then heated using microwave radiation in a Personal Chemistry Emrys™ Optimizer microwave reactor at 100°C for 80 minutes. After standing at room temperature for 2 days, the reaction mixture is poured onto water and extracted with ethyl acetate. The combined organic portions are washed with sodium hydrogencarbonate solution, brine, dried (MgSO₄) and concentrated *in vacuo* to afford a white solid. The solid is washed with hexane and filtered to yield the title compound. [MH+ 333.3]

### Examples 5-30

These compounds namely,
3-(6-Chloro-pyridin-3-yl)-7-hydroxy-2-isopropyl-3H-quinazolin-4-one (Example 5),
3-(6-Bromo-pyridin-3-yl)-7-hydroxy-2-isopropyl-3H-quinazolin-4-one (400MHz DMSO) 1 H nmr δ_{H} (400MHz CDCl₃) 10.6 (1H, s), 8.6 (1H, s), 8.1 (1H, d), 7.9 (1 H), 7.75 (1H, d), 7.0-6.9 (2H, m), 2.55-2.4 (1H, m), 1.15-1.05 (6H, m); (Example 6),
7-Hydroxy-2-isopropyl-3-(6-trifluoromethyl-pyridin-3-yl)-3H-quinazolin-4-one (Example 7),
7-Hydroxy-2-isopropyl-3-p-tolyl-3H-quinazolin-4-one(Example 8),
5-(7-Hydroxy-2-isopropyl-4-oxo-4H-quinazolin-3-yl)-pyridine-2-carbonitrile (Example 9),
3-(4-Acetyl-phenyl)-7-hydroxy-2-isopropyl-3H-quinazolin-4-(Example 10),
7-Hydroxy-3-(4-iodo-phenyl)-2-isopropyl-3H-quinazolin-4-one (Example 11)
4-(7-Hydroxy-2-isopropyl-4-oxo-4H-quinazolin-3-yl)-benzonitrile (Example 12),
3-(2-Chloro-pyrimidin-5-yl)-7-hydroxy-2-isopropyl-3H-quinazolin-4-one (Example 13),
3-(4-Ethyl-phenyl)-7-hydroxy-2-isopropyl-3H-quinazolin-4-one (Example 14),
3-(4-Chloro-phenyl)-7-hydroxy-2-isopropyl-3H-quinazolin-4-one (Example 15
Hydroxy-3-(1 H-indazol-6-yl)-2-isopropyl-3H-quinazolin-4-one (Example 16),
3-(4-Chloro-2-fluoro-phenyl)-7-hydroxy-2-isopropyl-3H-quinazolin-4-one (Example 17), 3-(6-Chloro-5-methyl-pyridin-3-yl)-7-hydroxy-2-isopropyl-3H-quinazolin-4-one (Example 18),
3-(6-chloro-5-methoxy-pyridin-3-yl)-7-hydroxy-2-isopropyl-3H-quinazolin-4-one (Example 19),
4-(7-Hydroxy-2-isopropyl-4-oxo-4H-quinazolin-3-yl)-2-methoxy-benzonitrile(4-iodophenyl)-2-isopropyl-3H-quinazolin-4-one (Example 20),
3-(4-Chloro-3-hydroxy-phenyl)-7-hydroxy-2-isopropyl-3H-quinazolin-4-one (Example 21),
3-(5,6-Dichloro-pyridin-3-yl)-7-hydroxy-2-isopropyl-3H-quinazolin-4-one (Example 22),
7-Hydroxy-2-isopropyl-3-(5-methyl-6-trifluoromethyl-pyridin-3-yl)-3H-quinazolin-4-one (Example 23),
7-Hydroxy-2-isopropyl-3-(5-methyoxy-6-trifluoromethyl-pyridin-3-yl)-3H-quinazolin-4-one (Example 24),
5-(7-Hydroxy-2-isopropyl-4-oxo-4H-quinazolin-3-yl)-3-methyl-pyridine-2-carbonitrile (Example 25),
5-(7-Hydroxy-2-isopropyl-4-oxo-4H-quinazolin-3-yl)-3-methoxy-pyridine-2-carbonitrile (Example 26),
3-(6-Chloro-5-iodo-pyridin-yl)-7-hydroxy-2-isopropyl-3H-quinazolin-4-one (Example 27),
3-(4-chloro-3-propoxy-phenyl)-7-hydroxy-2-isopropyl-3H-quinazolin-4-one (Example 28),
7-Hydroxy-2-isopropyl-3-(1-oxo-indan-5-yl)-3H-quinazolin-4-one (Example 29), 7-Hydroxy-2-isopropyl-3-(6-methyl-pyridin-3-yl)-3H-quinazolin-4-one; 1 H nmr δ_{H} (400 MHz DMSO) 10.6 (1H, s), 8.5 (1H, d), 7.95 (1H, d), 7.85 (1H, dd), 7.45 (1H, d), 7.0-6.9 (2H, m), 2.6 (3H, s), 1.2-1.0 (7H, m) (Example 30)
are prepared analogously to example 4 by replacing 4-chloro-3-fluoro-phenylamine with the appropriate amine. Those which are not commercially available are described in the preparation of intermediates section.

### Example 31:

### Example 31a

### 3-(4-Chloro-3-methoxymethyl-phenyl)-7-hydroxy-2-isopropyl-3H-quinazolin-4-one:

To 3-(4-chloro-3-chloromethyl-phenyl)-7-hydroxy-2-isopropyl-3H-quinazolin-4-one (intermediate M) (100 mg, 0.28 mmol) is added 1:1 MeOH: 1 N NaOH (10 ml). The reaction mixture is stirred at room temperature for 16 h to give the title compound as the major product. [M+H]+ 359.

### Example 31b

### 3-(4-Chloro-3-hydroxymethyl-phenyl)-7-hydroxy-2-isopropyl-3H-quinazolin-4-one

The title compound is prepared according to preparation 31 a to give the title compound as the minor product. [M+H]+ 345.

### Example 32

### (+/-)-4-[7-Hydroxy-8-(1-hydroxy-propyl)-2-isopropyl-4-oxo-4H-quinazolin-3-yl]-benzonitrile:

### a) 4-(8-Formyl-7-hydroxy-2-isopropyl-4-oxo-4H-quinazolin-3-yl)-benzonitrile: SAF502-NX-2 E-12588-107 DB

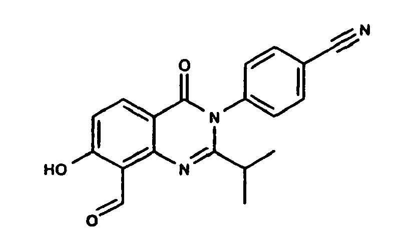

A mixture of 4-(7-hydroxy-2-isopropyl-4-oxo-4H-quinazolin-3-yl)-benzonitrile (example 12) (0.5 g, 1.64 mmol), hexamethylenetetramine (1.63g, 11.6 mmol) and glacial acetic acid (20 ml) is heated at 120°C for 2 h. The reaction mixture is concentrated *in vacuo,* 5M HCl (20 ml) is added and the reaction mixture is refluxed for 1 h. The reaction mixed is cooled to room temperature then poured onto ice. The solution/suspension is extracted with EtOAc, washed with water, followed by saturated NaHCO₃ solution, brine and dried (MgSO₄). Concentrated *in vacuo* to yield the title compound. [M+H]+ 334.

### b) (+/-)-4-[7-Hydroxy-8-(1-hydroxy-propyl)-2-isopropyl-4-oxo-4H-quinazolin-3-yl]-benzonitrile:

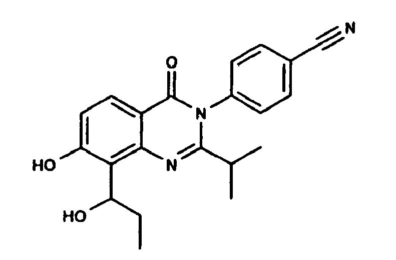

To a solution of 4-(8-formyl-7-hydroxy-2-isopropyl-4-oxo-4H-quinazolin-3-yl)-benzonitrile (32a) (0.3 g, 0.90 mmol) in dry THF (20 ml) at 0 °C is added ethylmagnesium bromide (1.35 ml, 1M in THF). The reaction mixture is quenched with water and extracted with EtOAc. The organic phase is washed with water, brine and dried (MgSO₄). Concentrated *in vacuo* and purified by flash chromatography on silica gel using iso-hexane : EtOAc (4 : 1) as the eluent to yield the title compound. [M+H]+ 364.

### Examples 33-56

These compounds namely,
(+/-)-5-[7-Hydroxy-8-(1-hydroxy-ethyl)-2-isopropyl-4-oxo-4H-quinazolin-3-yl]-pyridine-2-carbonitrile (example 33),
(+/-)-5-[7-Hydroxy-8-(hydroxy-propyl)-2-isopropyl-4-oxo-4H-quinazolin-3-yl]-pyridine-2-carbonitrile (example 34),
(+/-)-5-[7-Hydroxy-8-(1-hydroxy-2-methyl-propyl)-2-isopropyl-4-oxo-4-H-quinazolin-3-yl]-pyridine-2-carbonitrile (example 35),
(+/-)-7-Hydroxy-8-(1-hydroxy-2-methyl-propyl)-2-isopropyl-3-(6-trifluoromethyl-pyridin-3-yl)-3H-quinazolin-4-one (400MHz DMSO) 1 H nmr δ_{H} 10.75 (1 H, s), 9.0 (1H, s), 8.4 (1H, d), 8.2 (1H, d), 7.9 (1H, d), 7.0 (1H, d), 6.5 (1H, m), 5.25 (1H, m), 2.45 (1H, m), 2.15 (1H, m), 1.15 (6H, d), 0.95 (3H, d), 0.9 (3H, d); (example 36),
(+/-)-7-Hydroxy-8-(1-hydroxy-butyl)-2-isopropyl-3-(6-trifluoromethyl-pyridin-3-yl)-3H-quinazolin-4-one (example 37),
(+/-)-8-(Cyclopropyl-hydroxy-methyl)-7-hydroxy-2-isopropyl-3-(6-trifluoromethyl-pyridin-3-yl)-3H-quinazolin-4-one (example 38),
(+/-)-4-[7-Hydroxy-8-(1-hydroxy-2-methyl-propyl)-2-isopropyl-4-oxo-4H-quinazolin-3-yl]-benzonitrile (Example 39),
(+/-)-7-Hydroxy-8-(1-hydroxy-3-methyl-butyl)-2-isopropyl-3-(6-trifluoromethyl-pyridin-3-yl)-3H-quinazolin-4-one (Example 40),
(+/-)-8-(Cyclobutyl-hydroxy-methyl)-7-hydroxy-2-isopropyl-3-(6-trifluoromethyl-pyridin-3-yl)-3H-quinazolin-4-one (500MHz DMSO) 1 H nmr δ_{H} 10.65 (1 H, br), 8.96 (1H, m), 8.38 (1 H, m), 8.20 (1H, d), 7.89 (1H, d), 7.01 1H, 8.70), 6.44 (1 H, br), 5.46 (1 H, m), 2.86 (1 H, m), 2.48 (1 H, m), 1.99 (2H, m), 1.80 (2H, m), 1.99-1.75 (2H, m), 1.15 (6H, m); (Example 41),
(+/-)-4-[7-Hydroxy-8-(1-hydroxy-3-methyl-butyl)-2-isopropyl-4-oxo-4H-quinazolin-3-yl]-benzonitrile (Example 42),
(+/-)-7-Hydroxy-8-(1-hydroxy-2,2-dimethyl-propyl)-2-isopropyl-3-(6-trifluoromethyl-pyridin-3-yl)-3H-quinazolin-4-one (Example 43),
(+/-)-3-(4-Chloro-phenyl)-7-hydroxy-8-(1-hydroxy-2-phenyl-ethyl)-2-isopropyl-3H-quinazolin-4-one (Example 44),
(+/-)-5-[7-Hydroxy-8-(1-hydroxy-butyl)-2-isopropyl-4-oxo-4H-quinazolin-3-y]-pyridine-2-carbonitrile (Example 45),
(+/-)-5-[7-Hydroxy-8-(1-hydroxy-2-methyl-propyl)-2-isopropyl-4-oxo-4H-quinazolin-3-yl]-3-methyl-pyridine-2-carbonitrile (Example 46), (+/-)-5-[7-Hydroxy-8-(1-hydroxy-3-methyl-butyl)-2-isopropyl-4-oxo-4H-quinazolin-3-yl]-pyridine-2-carbonitrile (Example 47),
(+/-)-8-(Cyclohexyl-hydroxy-methyl)-7-hydroxy-2-isopropyl-3-(6-trifluoromethyl-pyridin-3-yl)-3H-quinazolin-4-one 400MHz (DMSO) 10.7 (1H, s), 9.0 (1 H, q), 8.4 (1 H, m), 8.2 (1H, d), 7.9 (1H, d), 7.0 (1H, d), 6.45 (1H, bs), 5.3 (1H, s), 2.45 (1H, m), 1.9 (2H, m), 1.7 (4H, m), 1.45 (1H, m), 1.25 (3H, m), 1.1 (6H, d); (Example 48), (+/-)-3-(4-Chloro-phenyl)-8-(cyclohexyl-hydroxy-methy)-7-hydroxy-2-isopropyl-3H-quinazolin-4-one (Example 49),
(+/-)-5-[8-(Cyclobutyl-hydroxy-methyl)-7-hydroxy-2-isopropyl-4-oxo-4H-quinazolin-3-yl]-pyridine-2-carbonitrile (Example 50),
(+/-)-5-[7-Hydroxy-8-(1-hydroxy-2,2-dimethyl-propyl)-2-isopropyl-4-oxo-4H-quinazolin-3-yl]-pyridine-2-carbonitrile (Example 51), (+/-)-7-Hydroxy-8-(1-hydroxy-2-methyl-propyl)-2-isopropyl-3-p-tolyl-3H-quinazolin-4-one (Example 52), (+/-)-7-Hydroxy-8-(1-hydroxy-2-methyl-propyl)-2-isopropyl-3-(6-methyl-pyridin-3-yl)-3H-quinazolin-4-one (Example 53),
(+/-)-5-[7-Hydroxy-8-(1-hydroxy-2-methyl-propyl)-2-isopropyl-4-oxo-4H-quinazolin-3-yl]-3-methoxy-pyridine-2-carbonitrile (Example 54),
(+/-)-3-(4-Chloro-phenyl)-7-hydroxy-8-(1-hydroxy-ethyl)-2-isopropyl-3H-quinazolin-4-one; (Example 55),
(+/-)-4-[7-Hydroxy-8-(1-hydroxy-ethyl)-2-isopropyl-4-oxo-4H-quinazolin-3-yl]-benzonitrile (Example 56),
are prepared analogously to example 32 by replacing 4-(7-hydroxy-2-isopropyl-4-oxo-4H-quinazolin-3-yl)-benzonitrile (example 12) with the appropriate quinazolinone, followed by treatment of the aldehyde formed with the appropriate Grignard reagent under similar conditions.

### Example 57:

### Example 57a

### 4-[7-Hydroxy-8-(1-hydroxy-propyl)-2-isopropyl-4-oxo-4H-quinazolin-3-yl]-benzonitrile: (ent1)

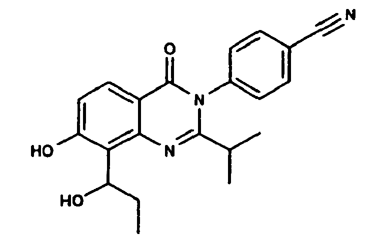

The title compound is prepared by preparative chiral HPLC of (+/-)-4-[7-hydroxy-8-(1-hydroxy-propyl)-2-isopropyl-4-oxo-4H-quinazolin-3-yl]-benzonitrile (32) to yield the title compound. [M+H]+ 364.

### Example 57b

### 4-[7-Hydroxy-8-(1-hydroxy-propyl)-2-isopropyl-4-oxo-4H-quinazolin-3-yl]-benzonitrile: (ent 2)

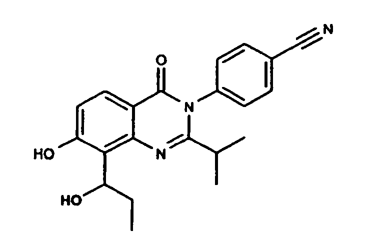

The title compound is prepared by preparative chiral HPLC of (+/-)-4-[7-hydroxy-8-(1-hydroxy-propyl)-2-isopropyl-4-oxo-4H-quinazolin-3-yl]-benzonitrile (30). [M+H]+ 364.

### Examples 58-79

These compounds namely,
4-[7-Hydroxy-8-(-hydroxy-2-methyl-propyl)-2-isopropyl4-oxo-4H-quinazolin-3-yl]-benzonitrile (*ent 1*) (Example 58),
4-[7-Hydroxy-8-(1-hydroxy-2-methyl-propyl)-2-isopropyl-4-oxo-4H-quinazolin-3-yl]-benzonitrile (*ent 2*) (Example 59),
7-Hydroxy-8-(1-hydroxy-2-methyl-propyl)-2-isopropyl-3-p-totyl-3H-quinazolin-4-one *(ent 1)* (Example 60),
7-Hydroxy-8-(1-hydroxy-2-methyl-propyl)-2-isopropyl-3-p-tolyl-3H-quinazolin-4-one *(ent 2*) (Example 61),
5-[7-Hydroxy-8-(1-hydroxy-2,2-dimethyl-propyl)-2-isopropyl-4-oxo-4H-quinazolin-3-yl]-pyridine-2-carbonitrile (*ent 1*) (Example 62), 5-[7-Hydroxy-8-(1-hydroxy-2,2-dimethyl-propyl)-2-isopropyl-4-oxo-4H-quinazolin-3-yl]-pyridine-2-carbonitrile (*ent 2*) (Example 63),
5-[8-(Cyclobutyl-hydroxy-methyl)-7-hydroxy-2-isopropyl-4-oxo-4H-quinazolin-3-yl]-pyridine-2-carbonitrile *(ent 1)* (Example 64),
5-[8-(Cyclobutyl-hydroxy-methyl)-7-hydroxy-2-isopropyl-4-oxo-4H-quinazolin-3-yl]-pyridine-2-carbonitrile *(ent* 2) (Example 65),
5-[7-Hydroxy-8-(1-hydroxy-3-methyl-butyl)-2-isopropyl-4-oxo-4H-quinazolin-3-yl]-pyridine-2-carbonitrile *(ent 1)* (Example 66), 5-[7-Hydroxy-8-(1-hydroxy-3-methyl-butyl)-2-isopropyl-4-oxo-4H-quinazolin-3-yl]-pyridine-2-carbonitrile *(ent 2*) (Example 67),
5-[7-Hydroxy-8-(1-hydroxy-2-methyl-propyl)-2-isopropyl-4-oxo-4H-quinazolin-3-yl]-3-methyl-pyridine-2-carbonitrile *(ent 1)* (Example 68),
5-[7-Hydroxy-8-(1-hydroxy-2-methyl-propyl)-2-isopropyl-4-oxo-4H-quinazolin-3-yl]-3-methyl-pyridine-2-carbonitrile *(ent* 2) (Example 69),
5-[7-Hydroxy-8-(1-hydroxy-butyl)-2-isopropyl-4-oxo-4H-quinazolin-3-yl]-pyridine-2-carbonitrile (*ent 1*) (Example 70),
5-[7-Hydroxy-8-(1-hydroxy-butyl)-2-isopropyl-4-oxo-4H-quinazolin-3-yl]-pyridine-2-carbonitrile *(ent* 2) (Example 71), 8-(Cyclobutyl-hydroxy-methyl)-7-hydroxy-2-isopropyl-3-(6-trifluoromethyl-pyridin-3-yl)-3H-quinazolin-4-one *(ent 1)* (Example 72),
8-(Cyclobutyl-hydroxy-methyl)-7-hydroxy-2-isopropyl-3-(6-trifluoromethyl-pyridin-3-yl)-3H-quinazolin-4-one *(ent* 2) (Example 73),
7-Hydroxy-8-(1-hydroxy-3-methyl-butyl)-2-isopropyl-3-(6-trifluoromethyl-pyridin-3-yl)-3H-quinazolin-4-one (*ent 1*) (Example 74),
7-Hydroxy-8-(1-hydroxy-3-methyl-butyl)-2-isopropyl-3-(6-trifluoromethyl-pyridin-3-yl)-3H-quinazolin-4-one *(ent* 2) (Example 75),
7-Hydroxy-8-(1-hydroxy-2-methyl-propyl)-2-isopropyl-3-(6-trifluoromethyl-pyridin-3-yl)-3H-quinazolin-4-one (*ent 1*) (example 76),
7-Hydroxy-8-(1-hydroxy-2-methyl-propyl)-2-isopropyl-3-(6-trifluoromethyl-pyridin-3-yl)-3H-quinazolin-4-one *(ent 2*) (example 77),
7-Hydroxy-8-(1-hydroxy-butyl)-2-isopropyl-3-(6-trifluoromethyl-pyridin-3-yl)-3H-quinazolin-4-one (*ent 1*) (example 78),
7-Hydroxy-8-(1-hydroxy-butyl)-2-isopropyl-3-(6-trifluoromethyl-pyridin-3-yl)-3H-quinazolin-4-one (*ent 2*) (example 79),
are prepared analogously to example 57.

### Example 80

### 7-Hydroxy-8-hydroxymethyl-2-isopropyl-3-p-tolyl-3H-quinazolin-4-one:

### a) 7-Hydroxy-2-isopropyl-4-oxo-3-p-tolyl-3,4-dihydro-quinazoline-8-carbaldehyde:

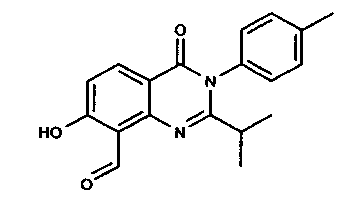

The title compound is prepared analogously to example 32a by replacing 4-(7-hydroxy-2-isopropyl-4-oxo-4H-quinazolin-3-yl)-benzonitrile (example 12) with 7-hydroxy-2-isopropyl-3-p-tolyl-3H-quinazolin-4-one (example 8). [M+H]+ 323.

### b) 7-Hydroxy-8-hydroxymethyl-2-isopropyl-3-p-tolyl-3H-quinazolin-4-one

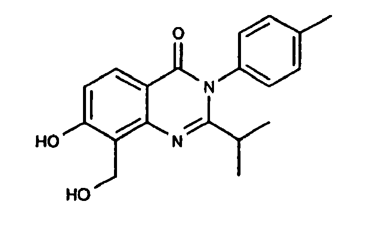

To a suspension of 7-hydroxy-2-isopropyl-4-oxo-3-p-tolyl-3,4-dihydro-quinazoline-8-carbaldehyde (100 mg, 0.29 mmol) (80a) in MeOH (5 ml) is added NaBH₄ (11 mg, 0.29 mmol). The reaction mixture is stirred at room temperature for 16 h. The reaction mixture is quenched with 10% citric acid solution and extracted with CH₂Cl₂, dried (MgSO₄) then concentrated *in vacuo.* Purified by flash chromatography on silica gel using iso-hexane : EtOAc (2: 1 to 4: 1) as the eluent to yield the title compound. [M+H]+ 325.

### Example 81

### 3-(4-Chloro-phenyl)-7-hydroxy-8-hydroxymethyl-2-isopropyl-3H-quinazolin-one:

The title compound is prepared analogously to preparation 80 by replacing 7-hydroxy-2-isopropyl-3-p-tolyl-3H-quinazolin-4-one (example 8) with 3-(4-chlorophenyl)-7-hydroxy-2-isopropyl-3H-quinazolin-4-one (example 15). [M+H]+ 345.

### Example 82

### 4-(7-Hydroxy-8-hydroxymethyl-2-isopropyl-4-oxo-4H-quinazolin-3-yl)-benzonitrile:

The title compound is prepared analogously to example 80 by replacing 7-hydroxy-2-isopropyl-3-p-tolyl-3H-quinazolin-4-one (example 8) with 4-(7-hydroxy-2-isopropyl-4-oxo-4H-quinazolin-3-yl)-benzonitrile (example 12). [M+H]+ 336.

### Example 83

### 5-(7-Hydroxy-8-hydroxymethyl-2-isopropyl-4-oxo-4H-quinazolin-3-yl)-pyridine-2-carbonitrile:

The title compound is prepared analogously to example 80 by replacing 7-hydroxy-2-isopropyl-3-p-tolyl-3H-quinazolin-4-one (example 8) with 5-(7-hydroxy-2-isopropyl-4-oxo-4H-quinazolin-3-yl)-pyridine-2-carbonitrile (Example 9). [M+H]+ 337.

### Example 84

### 4-(7-Hydroxy-2-isopropyl-4-oxo-8-propionyl-4H-quinazolin-3-yl)-benzonitrile:

To a solution of 4-[7-hydroxy-8-(1-hydroxy-propyl)-2-isopropyl-4-oxo-4H-quinazolin-3-yl]-benzonitrile (32b) (118 mg, 0.32 mmol) in CH₂Cl₂ (5 ml) is added pyridinium chlorochromate (104 mg, 0.49 mmol). The reaction mixture is stirred at room temperature for 16 h. The reaction mixture is diluted with Et₂O (20 ml) and stirred for 5 mins. The mixture is filtered and concentrated *in vacuo.* Purified by flash chromatography on silica gel using iso-hexane : EtOAc (3 : 1) as the eluent to yield the title compound. [M+H]+ 362.

### Examples 85-88

These compounds namely,
5-(8-Butyryl-7-hydroxy-2-isopropyl-4-oxo-4H-quinazolin-3-yl)-pyridine-2-carbonitrile (Example 85),
5-[7-Hydroxy-2-isopropyl-8-(3-methyl-butyryl)-4-oxo-4H-quinazolin-3-yl]-pyridine-2-carbonitrile (Example 86),
4-(7-Hydroxy-8-isobutyryl-2-isopropyl-4-oxo-4H-quinazolin-3-yl)-benzonitrile (Example 87),
7-Hydroxy-8-isobutyryl-2-isopropyl-3-p-tolyl-3H-quinazolin-4-one (Example 88), are prepared analogously to example 84 by replacing 4-[7-hydroxy-8-(1-hydroxypropyl)-2-isopropyl-4-oxo-4H-quinazolin-3-yl]-benzonitrile (32b) with the appropriate 8-(hydroxyalkyl)-substituted quinazolinone.

### Example 89

### (+/-)-3-(4-Chloro-phenyl)-7-hydroxy-8-(hydroxyl-phenyl-methyl)-2-isopropyl-3H-quinazolin-4-one:

### a) 3-(4-Chloro-phenyl)-7-hydroxy-2-isopropyl-4-oxo-3,4-dihydroquinazoline-8-carbaldehyde:

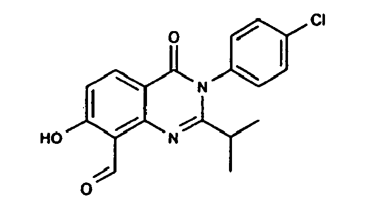

The title compound is prepared analogously to example 32a by replacing 4-(7-hydroxy-2-isopropyl-4-oxo-4H-quinazolin-3-yl)-benzonitrile (example 12) with 3-(4-chloro-phenyl)-7-hydroxy-2-isopropyl-3H-quinazolin-4-one (example 15). [M+H]+ 343.

### b) (+/-)-3-(4-Chloro-phenyl)-7-hydroxy-8-(hydroxyl-phenyl-methyl)-2-isopropyl-3H-quinazolin-4-one: QBA906-NX-1 E-15985-082 KB

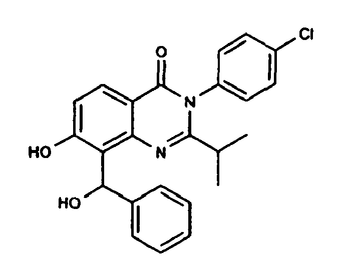

To a solution of 3-(4-chloro-phenyl)-7-hydroxy-2-isopropyl-4-oxo-3,4-dihydroquinazoline-8-carbaldehyde (89a) (70 mg, 0.20 mmol) in dry THF : Et₂O (5 ml : 10 ml) at -78 °C under nitrogen is added dropwise phenyllithium (0.22 ml, 0.45 mmol; 2M in Et₂O). The reaction mixture is warmed to room temperature and extracted with EtOAc (2 x 20 ml). The organics are combined, washed with water (10 ml), brine (10 ml) and dried (MgSO₄). Concentrated *in vacuo* then purified by flash chromatography on silica gel using iso-hexane : EtOAc (5 : 2) as the eluent to yield the title compound. [M+H]+ 421.

### Example 90

### (+/-)-3-(4-Chloro-phenyl)-7-hydroxy-8-(hydroxy-pyridin-2-yl-methyl)-2-isopropyl-3H-quinazolin-4-one:

The title compound is prepared analogously to example 89b by replacing phenyllithium with 2-pyridyllithium (intermediate N). [M+H]+ 422.

### Example 91

### (+/-)-3-(4-Chloro-phenyl)-7-hydroxy-8-(hydroxyl-pyridin-3-yl-methyl)-2-isopropyl-3H-quinazolin-4-one:

The title compound is prepared analogously to example 89b by replacing phenyllithium with 3-pyridyllithium (intermediate O). [M+H]+ 433.

### Example 92

### (+/-)-3-(4-Chloro-phenyl)-7-hydroxy-8-(1-hydroxy-2-pyridin-2-yl-ethyl)-2-isopropyl-3H-quinazolin-one:

To a solution of diisopropylamine (94 µl, 0.67 mmol) in dry Et₂O (10 ml) at 0 °C is added dropwise n-BuLi (268 µl, 0.67 mmol; 2.5M in hexane). The reaction mixture is stirred at 0 °C for 20 mins, then a solution of 2-picoline (63 µl, 0.64 mmol) in dry Et₂O (3 ml) is added dropwise. The reaction mixture is stirred at 0 °C for 1 h then added to a solution of 3-(4-chloro-phenyl)-7-hydroxy-2-isopropyl-4-oxo-3,4-dihydroquinazoline-8-carbaldehyde (89a) (100 mg, 0.29 mmol) in dry THF (5 ml) under nitrogen at -78 °C. The reaction mixture is stirred at -78 °C for 2 h then treated with saturated NH₄Cl solution (20 ml) and warmed to room temperature. The product is extracted with EtOAc (3 x 20 ml), the organics are combined, washed with water (20 ml), brine (20ml) and dried (MgSO₄). Concentrated *in vacuo* then purified by flash chromatography on silica gel using iso-hexane : EtOAc (2 : 1) as the eluent to yield the title compound. [M+H]+ 435.

### Example 93

### 4-(8-Acetyl-7-hydroxy-2-isopropyl-4-oxo-4H-quinazolin-3-yl)-benzonitrile:

### a) 4-(7-Hydroxy-8-iodo-2-isopropyl-4-oxo-4H-quinazolin-3-yl)-benzonitrile:

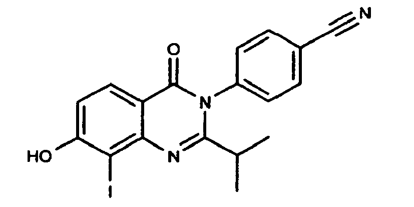

To a suspension of 4-(7-hydroxy-2-isopropyl-4-oxo-4H-quinazolin-3-yl)-benzonitrile (example 12) (5.0 g, 16.4 mmol) in CH₂Cl₂ (300 ml) cooled to 0° C is added N-iodosuccinimide (4.42 g, 19.7 mmol). The reaction mixture is stirred at 0 °C for 2 h. The reaction mixture is poured into water and the lower organic phase is separated, washed with water, brine and dried (MgSO₄). The organic phase is concentrated *in vacuo* to yield the title compound. [M+H]+ 432.

### b) 4-(8-Acetyl-7-hydroxy-2-isopropyl-4-oxo-4H-quinazolin-3-yl)-benzonitrile:

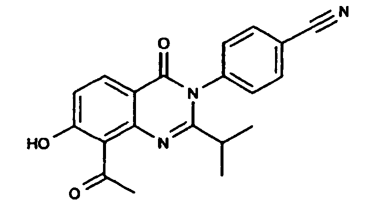

To a solution of 4-(7-hydroxy-8-iodo-2-isopropyl-4-oxo-4H-quinazolin-3-yl)-benzonitrile (93a) (1.0 g, 2.3 mmol) in DMF (13 ml) is added DPPP (210 mg, 0.5 mmol), potassium carbonate (388 mg, 2.80 mmol), followed by butyl vinyl ether (1.5 ml) and water (2 ml). Palladium (II) acetate (52 mg, 0.23 mmol) is added and the reaction mixture is heated in the microwave at 80 °C for 1 h. Further DPPP (210 mg, 0.5 mmol), potassium carbonate (368 mg, 2.80 mmol), butyl vinyl ether (1.5 ml) and palladium (II) acetate (52 mg, 0.23 mmol) are added. The reaction mixture is heated in the microwave at 80 °C for 1 h. The reaction mixture is poured into water (100 ml) and extracted with EtOAc (2 x 100 ml). The extracts are combined, washed with brine and dried (MgSO₄). Concentrated *in vacuo* then purified by flash chromatography on silica gel using iso-hexane : EtOAc (4 : 1) as the eluent to yield the title compound. [M+H]+ 348.

### Example 94

### 3-(4-Chloro-phenyl)-7-hydroxy-8-iodo-2-isopropyl-3H-quinazolin-4-one:

To a solution of 4-(8-acetyl-7-hydroxy-2-isopropyl-4-oxo-4H-quinazolin-3-yl)-benzonitrile (93b) (100 mg, 0.29 mmol) in dry THF (5 ml) under nitrogen at 0 °C is added methylmagnesium bromide (192 µl, 0.58 mmol; 3M in Et₂O). The reaction mixture is stirred at 0 °C for 2 h. The reaction mixture is quenched with a solution of saturated NH₄Cl, diluted with Et₂O and filtered. The organic layer is separated, washed with brine and dried (MgSO₄). The crude material is recrystallised from MeOH to yield the title compound. [M+H]+ 364.

### Example 95

### 8-Acetyl-3-(4-chloro-phenyl)-7-hydroxy-2-isopropyl-3H-quinazolin-4-one:

The title compound is prepared analogously to example 93 by replacing 4-(7-hydroxy-2-isopropyl-4-oxo-4H-quinazolin-3-yl)-benzonitrile (example 12) with 3-(4-chloro-phenyl)-7-hydroxy-2-isopropyl-3H-quinazolin-4-one (example 15). [M+H]+ 35

### Example 96

### 4-(7-Hydroxy-2-isopropyl-8-methoxymethyl-4-oxo-4H-quinazolin-3-yl)benzonitrile:

### a) 4-[8-Formyl-2-isopropyl-7-(4-methoxy-benzyloxy)-4-oxo-4H-quinazolin-3-yl]-benzonitrile:

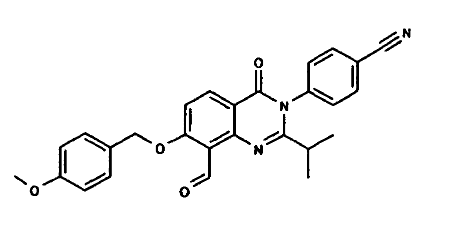

To a solution of 4-(8-formyl-7-hydroxy-2-isopropyl-4-oxo-4H-quinazolin-3-yl)-benzonitrile (1.90 g, 5.7 mmol) (32a) in DMF (40 ml) under nitrogen at room temperature is added portionwise K₂CO₃ (2.4 g, 17.1 mmol) followed by 4-methoxybenzyl bromide (1.64 ml, 11.4 mmol). The reaction mixture is stirred at room temperature for 16 h. The reaction mixture is diluted with water (150 ml) and extracted with CH₂Cl₂ (2 x 80 ml). The organics are combined, washed with brine, dried (MgSO₄) and concentrated *in vacuo.* Triturated with EtOAc (40 ml) and filtered to yield the title compound. [M+H]+ 454.

### b) 4-[8-Hydroxymethyl-2-isopropyl-7-(4-methoxy-benryloxy)-4-oxo-4H-quinazolin-3-yl]-benzonitrile:

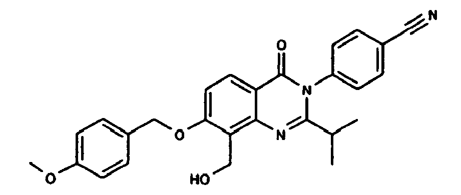

The title compound is prepared analogously to (80b) replacing 7-hydroxy-2-isopropyl-4-oxo-3-p-tolyl-3,4-dihydro-quinazoline-8-carbaldehyde (80a) with 4-[8-formyl-2-isopropyl-7-(4-methoxy-benzyloxy)-4-oxo-4H-quinazolin-3-yl]-benzonitrile (96a). [M+H]+ 456.

### c) 4-[2-Isopropyl-7-(4-methoxy-benzyloxy)-8-methoxymethyl-4-oxo-4H-quinazolin-3-yl]-benzonitrile:

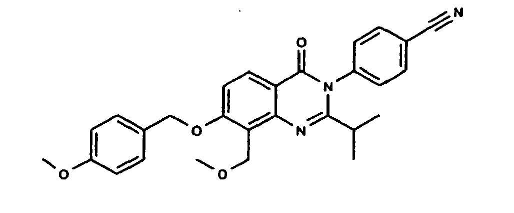

To a solution of 4-[8-hydroxymethyl-2-isopropyl-7-(4-methoxy-benzyloxy)-4-oxo-4H-quinazolin-3-yl]-benzonitrile (96b) (200 mg, 0.44 mmol) in dry THF (10 ml) is added NaH (26 mg, 0.66 mmol; 60% dispersion in mineral oil). The reaction mixture is stirred at room temperature for 1 h. The reaction mixture is cooled to 0 °C and Mel (60µl, 0.97 mmol) is added. The reaction mixture is allowed to warm up to room temperature with stirring for 16 h. Further NaH (13.2 mg, 0.33 mmol) is added and the reaction mixture is stirred at room temperature for 15 minutes before adding Mel (30µl, 0.49 mmol) at room temperature. The reaction mixture is stirred at room temperature for 5 h. The reaction mixture is quenched with MeOH (10 ml), concentrated *in vacuo* and partitioned between EtOAc (20 ml) and water (20 ml). The organic layer is separated and dried (MgSO₄). Concentrated *in vacuo* then purified by flash chromatography on silica gel using CH₂Cl₂: MeOH (99 : 1) as the eluent to yield the title compound. [M+H]+ 470.

### d) 4-(7-Hydroxy-2-isopropyl-8-methoxymethyl-4-oxo-4H-quinazolin-3-yl)benzonitrile:

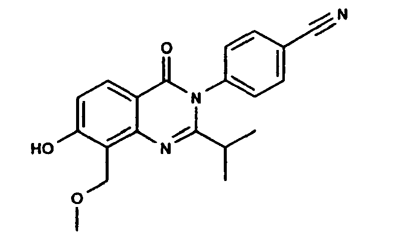

4-[2-isopropyl-7-(4-methoxy-benzyloxy)-8-methoxymethyl-4-oxo-4H-quinazolin-3-yl]-benzonitrile (148 mg, 0.32 mmol) (96c) is dissolved in 5% TFA/CH₂Cl₂ (5 ml) and the reaction mixture is stirred under nitrogen at room temperature for 72 h. The reaction mixture is neutralised with a saturated solution of NaHCO₃ (10 ml) and extracted with CH₂Cl₂ (2 x 20 ml). The organic extracts are combined, washed with brine and dried (MgSO₄). Concentrated *in vacuo* then purified by flash chromatography on silica gel using iso-hexane : EtOAc (3 : 1) as the eluent to yield the title compound. [M+H]+ 350.

### Example 97

### 7-Hydroxy-2-isopropyl-8-(2-methoxy-ethoxymethyl)-3-p-tolyl-3H-quinazolin-4-one:

A microwave vial is charged with 7-hydroxy-8-hydroxymethyl-2-isopropyl-3-p-tolyl-3H-quinazolin-4-one (100 mg, 0.31 mmol) (80b) and 2-methoxyethanol (3 ml). The reaction mixture is heated at 130 °C in a microwave for 15 mins. The reaction mixture is concentrated *in vacuo* and purified by flash chromatography on silica gel using iso-hexane : EtOAc (5 : 1) as the eluent to yield the title compound. [M+H]+ 383.

### Example 98

### 7-Hydroxy-2-isopropyl-8-methoxymethyl-3-p-tolyl-3H-quinazolin-4-one:

A microwave vial is charged with 7-hydroxy-8-hydroxymethyl-2-isopropyl-3-p-tolyl-3H-quinazolin-4-one (100 mg, 0.31 mmol) (80b) and dry MeOH (3 ml). The reaction mixture is heated at 130 °C in a microwave for 10 mins. The reaction mixture is concentrated *in vacuo* and purified by flash chromatography on silica gel using iso-hexane : EtOAc (5 : 1) as the eluent to yield the title compound. [M+H]+ 339.

### Example 99

### 4-(7-Hydroxy-2-isopropyl-4-oxo-8-phenyl-4H-quinazolin-3-yl)-benzonitrile:

To a solution of 4-(7-hydroxy-8-iodo-2-isopropyl-4-oxo-4H-quinazolin-3-yl)-benzonitrile (50 mg, 0.12 mmol) (93a) in toluene (1 ml) is added K₂CO₃ (48 mg, 0.35 mmol) in water (1 ml). Phenylboronic acid (21.2 mg, 0.17 mmol) is added followed by tetrakis(triphenylphosphine)palladium(0) (6.7 mg, 0.0058 mmol). The reaction mixture is heated in a microwave at 100 °C for 30 min. The reaction mixture is diluted with EtOAc and water. The organic phase is separated, washed with water and dried (MgSO₄). Concentrated *in vacuo* then purified by flash chromatography on silica gel using iso-hexane : EtOAc (4 : 1 to 2 : 1) as the eluent. Triturated with MeOH and the solid formed is filtered off to yield the title compound. (400 MHz CDCl₃) 1 H nmr δ_{H} 8.22 (1H,d) ; 7.87 (2H,d) ; 7.38-7.57 (5H,m) ; 7.19 (1H,d) ; 5.93 (1 H,s) ; 2.50 (1H,m) ; 1.02 (6H,d).

### Example 100

### 4-(7-Hydroxy-2-isopropyl-4-oxo-8-propyl-4H-quinazolin-3-yl)-benzonitrile:

To a solution of (+/-)-4-[7-hydroxy-8-(1-hydroxy-propyl)-2-isopropyl-4-oxo-4H-quinazolin-3-yl]-benzonitrile (243 mg, 0.69 mmol) (32b) in dry CH₂Cl₂ (3 ml) is added Et₃SiH (170 µl, 1.06 mmol). The solution is cooled to 0 °C and treated with TFA (1.24 ml, 16.7 mmol). The reaction mixture is heated in a microwave at 100 °C for 10 min.
The reaction mixture is neutralised with a saturated solution of NaHCO₃ and extracted with CH₂Cl₂ (2 x 20 ml). The organics are combined, washed with water (20 ml), brine (20 ml) and dried (MgSO₄). The reaction mixture is concentrated *in vacuo* and purified by flash chromatography on silica gel using iso-hexane : EtOAc (3 : 1) as the eluent to yield the title compound. [M+H]+ 348.

### Example 101

3-(4-Chloro-phenyl)-7-hydroxy-2-isopropyl-8-pyridin-2-ylmethyl-3H-quinazolin-4-one: The title compound is prepared analogously to preparation 100 by replacing (+/-)-4-[7-hydroxy-8-(1-hydroxy-propyl)-2-isopropyl-4-oxo-4H-quinazolin-3-yl]-benzonitrile (32b) with (+/-)-3-(4-chloro-phenyl)-7-hydroxy-8-(hydroxy-pyridin-2-yl-methyl)-2-isopropyl-3H-quinazolin-4-one (90). [M+H]+ 406

### Example 102

### 3-(4-Chloro-phenyl)-2-ethyl-7-hvdroxy-3H-quinazolin-4-one:

### a) 3-(4-Chloro-phenyl)-2-ethyl-7-methoxy-3H-quinazolin-4-one:

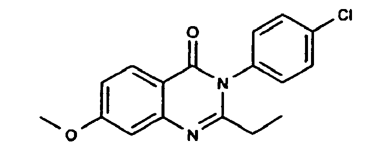

2-amino-N-(4-chloro-phenyl)-4-methoxy-benzamide (intermediate P) (500 mg, 0.36 mmol) is suspended in triethyl orthopropionate (7 ml) and heated at 100 °C in a microwave for 3 h. The reaction mixture is heated further at 140 °C in a microwave for 2 h. The reaction mixture is left standing at room temperature for 72 h. The crystalline compound is isolated by filtration. [M+H] 315.

### b) 3-(4-Chloro-phenyl)-2-ethyl-7-hydroxy-3H-quinazolin-4-one:

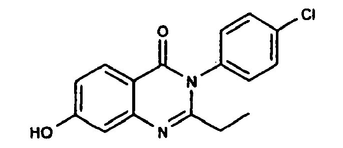

A suspension of 3-(4-chloro-phenyl)-2-ethyl-7-methoxy-3H-quinazolin-4-one (102a) (50 mg, 0.16 mmol) in 48% aqueous HBr (3 ml) is heated at 120 °C in a microwave for 1 h. The reaction mixture is cooled to room temperature, filtered and washed with water (2 x 5ml) to afford the title compound. [M+H]+ 301.

### Examples 103

### 2-tert-Butyl-3-(4-chloro-phenyl)-7-hydroxy-3H-quinazolin-4-one:

### a) 2-tert-Butyl-3-(4-chloro-phenyl)-7-methoxy-3H-quinazolin-4-one:

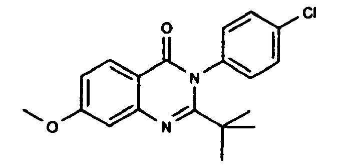

The title intermediate is prepared analogously to preparation 4 by replacing intermediate C2 with 2-(2,2-dimethyl-propionylamino)-4-methoxy-benzoic acid (intermediate Q) and 4-chloro-3-fluoro-phenylamine with 4-chloroaniline respectively. [M+H]+ 343.

### b) 2-tert-Butyl-3-(4-chloro-phenyl)-7-hydroxy-3H-quinazolin-4-one:

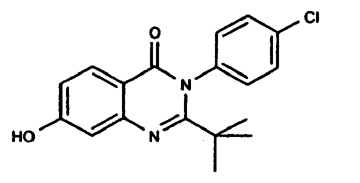

The title compound is prepared analogously to preparation 102b by replacing 3-(4-chloro-phenyl)-2-ethyl-7-methoxy-3H-quinazolin-4-one (102a) with 2-tert-butyl-3-(4-chloro-phenyl)-7-methoxy-3H-quinazolin-4-one (103a). [M+H]+ 329.

### Example 104

### 3-(4-Chloro-phenyl)-7-hydroxy-2-(1-hydroxy-1-methyl-ethyl)-3H-quinazolin-4-one:

The title compound is prepared analogously to preparation 4 by replacing 2-isobutyrylamino-4-triisopropylsilanyloxy-benzoic acid (intermediate C) with 2-(2-hydroxy-2-methyl-propionylamino)-4-triisopropylsilanyloxy-benzoic acid (intermediate R) and 4-chloro-3-fluoro-phenylamine with 4-chloroaniline respectively. [M+H]+ 331.

### Example 105

### 4-(2-Diethylamino-7-hydroxy-4-oxo-4H-quinazolin-3-yl)-benzonitrile:

### a) 4-(7-Methoxy-2,4-dioxo-1,4-dihydro-2H-quinazolin-3-yl)-benzonitrile:

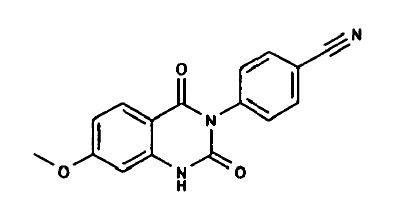

To a solution of 2-amino-N-(4-cyano-phenyl)-4-methoxy-benzamide (500 mg, 1.87 mmol) (intermediate S) in CH₂Cl₂ is added triphosgene (556 mg, 1.87 mmol). The reaction mixture is heated for 1h at 100 °C in a microwave. The reaction mixture is cooled, filtered and the solid is washed with CH₂Cl₂ and dried to yield the title compound. No mass ion.

### b) 4-(2-Diethylamino-7-methoxy-4-oxo-4H-quinazolin-3-yl)-benzonitrile:

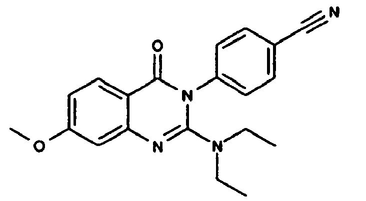

To a suspension of 4-(7-methoxy-2,4-dioxo-1,4-dihydro-2H-quinazolin-3-yl)-benzonitrile (293 mg, 1 mmol) (105a) in MeCN (10 ml) is added BOP-Cl (331 mg, 1.3 mmol) followed by DBU (226µl, 1.5 mmol) and diethylamine (522µl, 5 mmol). The reaction mixture is heated in a microwave for 2 h at 80 °C. The reaction mixture is cooled, concentrated *in vacuo* and the residue is suspended in EtOAc. Filtered, washed with EtOAc and the filtrate is dried (MgSO₄) and concentrated *in vacuo.* Purified by flash chromatography on silica gel using iso-hexane : EtOAc (10 : 1 to 4 : 1) as the eluent to yield the title compound. No mass ion.

### c) 4-(2-Diethylamino-7-hydroxy-4-oxo-4H-quinazolin-3-yl)-benzonitrile:

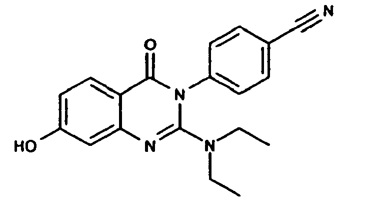

To a solution of 4-(2-diethylamino-7-methoxy-4-oxo-4H-quinazolin-3-yl)-benzonitrile (260 mg, 0.75 mmol) (105b) in DMF (7 ml) is added sodium methoxide (122 mg, 2.25 mmol) and 1-dodecanethiol (612 µl, 2.55 mmol). The reaction mixture is heated at 100 °C for 1 h then cooled and diluted with water. Extracted with EtOAc, dried (MgSO₄) and concentrated *in vacuo.* Purified by flash chromatography on silica gel using iso-hexane : EtOAc (10 : 1 to 4 1) as the eluent to yield the title compound. [M+H]+ 335.

### Example 106

### 3-(4-Chloro-phenyl)-7-hydroxy-2-isopropenyl-3H-quinazolin-4-one:

To a suspension of N-(4-Chloro-phenyl)-2-(2-methyl-acryloylamino)-4-triisopropylsilanyloxy-benzamide (100 mg, 0.21 mmol) (intermediate T) in EtOH (1 ml) is added conc. H₂SO₄ (~ 4 drops) and the reaction mixture is heated in a microwave at 100 °C for 30 min. The reaction mixture is partitioned between CH₂Cl₂ and water then passed through an Isolute^{™} phase separator. Concentrated *in vacuo* and purified by flash chromatography on silica gel using iso-hexane : EtOAc (2:1) to give the title compound. [M+H]+ 313.

### Example 107

### 3-(4-Chloro-phenyl)-2-isopropyl-5-methoxy-3H-quinazolin-4-one:

The title compound is prepared analogously to preparation 4, replacing intermediate C2 with 2-isobutyrylamino-6-methoxy-benzoic acid (intermediate U) and 4-chloro-3-fluoro-phenylamine with 4-chloroaniline respectively. [M+H] 329.

### Example 108

### 3-(4-Chloro-phenyl)-5,7-dihydroxy-2-isopropyl-3H-quinazolin-4-one:

### a) 3-(4-Chloro-phenyl)-2-isopropyl-5,7-dimethoxy-3H-quinazolin-4-one:

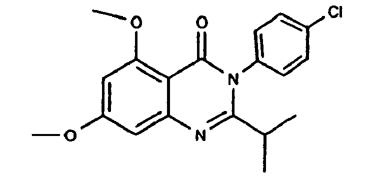

A solution of 2-amino-N-(4-chloro-phenyl)-4,6-dimethoxy-benzamide (700 mg, 2.29 mmol) (intermediate V) in 1,1,1-trimethoxy-2-methylpropane (5 ml) is heated at reflux for 4 h. The reaction mixture is evaporated *in vacuo* and purified by reverse phase (C18) chromatography using MeCN : H₂O containing 0.1% TFA as the eluent.

Purified further by flash chromatography on silica gel using iso-hexane : EtOAc (2 : 1) as the eluent to yield the title compound. [M+H]+ 359.

### b) 3-(4-Chloro-phenyl)-5,7-dihydroxy-2-isopropyl-3H-quinazolin-4-one:

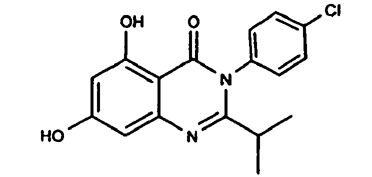

To a solution of 3-(4-chloro-phenyl)-2-isopropyl-5,7-dimethoxy-3H-quinazolin-4-one (84 mg, 0.24 mmol) (108a) in dry CH₂Cl₂ (5 ml) under nitrogen at 0 °C is added BBr₃. The reaction mixture is allowed to warm up to room temperature with stirring for 4 days. The reaction mixture is cooled to 0 °C and a solution of saturated NaHCO₃ is added. Extracted with CH₂Cl₂, washed with brine and dried (MgSO₄). Concentrated *in vacuo* and purified by reverse phase chromatography (C18) using MeCN : H₂O containing 0.1 % TFA as the eluent to yield the title compound. [M+H]+ 331.

### Example 109

### 3-4-Chloro-phenyl)-7-hydroxy-2-isopropyl-6-methoxy-3H-quinazolin-4-one:

The title compound is prepared analogously to example 4, replacing intermediate C2 with 4-hydroxy-2-isobutyrylamino-5-methoxy-benzoic acid (intermediate W) and 4-chloro-3-fluoro-phenylamine with 4-chloroaniline respectively to yield the title compound. [M+H]+ 345.

### Example 110

### 3-(4-Chloro-phenyl)-6,7-dihydroxy-2-isopropyl-3H-quinazolin-4-one:

A solution of 3-(4-chloro-phenyl)-7-hydroxy-2-isopropyl-6-methoxy-3H-quinazolin-4-one (109) (100 mg, 0.29 mmol) in 48% aqueous HBr (1.5 ml) and glacial acetic acid (1.5 ml) is heated in the microwave at 100° C for 1 h, followed by 130 °C for 2 h. The reaction mixture is cooled to room temperature then treated with 1 M NaOH solution to pH 6-7. Extracted with EtOAc (3 x 10 ml), the organics are combined, washed with brine (20 ml), dried (MgSO₄) and concentrated *in vacuo.* Recrystallised from (1:1) MeCN : H₂O (4 ml) to yield the title compound. [M+H]+ 331.

### Example 111

### 4-(7-Amino-2-isopropyl-4-oxo-4H-quinazolin-3-yl)-benzonitrile:

The title compound is prepared analogously to example 3 by replacing 4-chloroaniline with 4-aminobenzonitrile. [M+H]+ 305.

The following compounds namely,
2-amino-3-(4-chloro-phenyl)-7-hydroxy-3H-quinazolin-4-one, (Example 111)
3-(4-chloro-phenyl)-7-hydroxy-2-pyrrolidin-1-yl-3H-quinazolin-4-one, (Example 112)
3-(4-chloro-phenyl)-7-hydroxy-2-morpholin-4-yl-3H-quinazolin-4-one, (Example 113)
3-(4-chloro-phenyl)-7-hydroxy-2-(2-hydroxy-ethylamino)-3H-quinazolin-4 -one and (Example 114)
2-(2-amino-ethylamino)-3-(4-chloro-phenyl)-7-hydroxy-3H-quinazolin-4-o ne, (Example 115)
5-(2-dimethylamino-7-hydroxy-4-oxo-4H-quinazolin-3-yl)-pyridine-2-carb onitrile, (Example 116)
3-(4-chloro-benzyl)-2-dimethylamino-7-hydroxy-3H-quinazolin-4-one, (Example 117) 2-dimethylamino-7-hydroxy-3-isopropyl-3H-quinazolin-4-one,
can be prepared analogously to 3-(4-chloro-phenyl)-2-diethylamino-7-hydroxy-3H-quinazolin-4-one (Example 1) by replacing diethylamine with the appropriate amine. Some of these examples may also require replacing 2-chloro-3-(4-chlorophenyl)-7-methoxy-3H-quinazolin-4-one (Intermediate A) with an alternative starting material prepared analogously to intermediate A with the appropriate aniline/alkylamine in step A1.

The following compounds namely,
3-(4-chloro-phenyl)-7-hydroxy-2-(2,2,2-trifluoro-1-trifluoromethyl-ethyl)-3H-quinazolin-4-one, (Example 118)
3-(4-chloro-phenyl)-7-hydroxy-2-(1-hydroxy-ethyl)-3H-quinazolin-4-one, (Example 119)
3-(4-chloro-phenyl)-7-hydroxy-2-(2-hydroxy-1,1-dimethyl-ethyl)-3H-quinazolin-4-one, (Example 120)
3-(4-chloro-phenyl)-7-hydroxy-2-(2,2,2-trifluoro-1-hydroxymethyl-ethyl)-3H-quinazolin-4-one, (Example 121)
[3-(4-chloro-phenyl)-7-hydroxy-4-oxo-3,4-dihydro-quinazolin-2-yl]-acetonitrile, (Example 122)
3-(4-chloro-phenyl)-7-hydroxy-2-methoxymethyl-3H-quinazolin-4-one,
3-(4-chloro-phenyl)-2-dimethylaminomethyl-7-hydroxy-3H-quinazolin-4-one, (Example 123)
2-(1-amino-ethyl)-3-(4-chloro-phenyl)-7-hydroxy-3H-quinazolin-4-one, (Example 124)
3-(4-chloro-phenyl)-2-furan-2-ylmethyl-7-hydroxy-3H-quinazolin-4-one, (Example 125)
3-(4-chloro-phenyl)-7-hydroxy-2-(1-methyl-1H-imidazol-2-ylmethyl)-3H-quinazolin-4-one, (Example 126)
2,3-bis-(4-chloro-phenyl)-7-hydroxy-3H-quinazolin-4-one (Example 127) can be prepared analogously to {(R)-1-[3-(4-Chloro-phenyl)-7-hydroxy-4-oxo-3,4-dihydro-quinazolin-2-yl]-ethyl}-carbamic acid benzyl ester (Example 2) by replacing (R)-2-benzyloxycarbonylamino-propionic acid (3.19 g, 14.3 mmol) with the appropriate acid. Compounds containing free hydroxyl group should be protected using, for example, a reagent such as triisopropylsilyl chloride as described in the preparation of triisopropyl-(4-methyl-3-nitro-phenoxy)-silane (B1).

The following compounds namely,
3-(4-Chloro-3-dimethylaminomethyl-phenyl)-7-hydroxy-2-isopropyl-3H-quinazolin-4-one, (Example 128)
3-(4-Chloro-2-fluoro-5-hydroxy-phenyl)-7-hydroxy-2-isopropyl-3H-quinazolin-4-one, (Example 129)
3-(4-Chloro-2,5-dimethoxy-phenyl)-7-hydroxy-2-isopropyl-3H-quinazolin-4-one, (Example 130)
3-(4-Chloro-2-fluoro-3-methyl-6-trifluoromethyl-phenyl)-7-hydroxy-2-isopropyl-3H-quinazolin-4-one, (Example 131)
3-[4-(4-Chloro-phenyl)-thiazol-2-yl]-7-hydroxy-2-isopropyl-3H-quinazolin-4-one, (Example 132)
7-Hydroxy-2-isopropyl-3-(3-methyl-isoxazol-5-yl)-3H-quinazolin-4-one, (Example 133)
3-(5-Chloro-benzothiazol-2-yl)-7-hydroxy-2-isopropyl-3H-quinazolin-4-one, (Example 134)
3-(5-Chloro-thiazol-2-yl)-7-hydroxy-2-isopropyl-3H-quinazolin-4-one, (Example 135)
7-Hydroxy-2-isopropyl-3-(1-methyl-piperidin-4-yl)-3H-quinazolin-4-one, (Example 136)
7-Hydroxy-2-isopropyl-3-propyl-3H-quinazolin-4-one, (Example 137)
7-Hydroxy-2,3-diisopropyl-3H-quinazolin-4-one, (Example 138)
7-Hydroxy-3-(2-hydroxy-ethyl)-2-isopropyl-3H-quinazolin-4-one, (Example 139)
7-Hydroxy-3-[2-(2-hydroxy-ethoxy)-ethyl]-2-isopropyl-3H-quinazolin-4-one, (Example 140)
7-Hydroxy-2-isopropyl-3-(2-methylamino-ethyl)-3H-quinazolin-4-one, (Example 141)
3-Furan-3-ylmethyl-7-hydroxy-2-isopropyl-3H-quinazolin-4-one, (Example 142)
7-Hydroxy-2-isopropyl-3-pyridin-4-ylmethyl-3H-quinazolin-4-one, (Example 143)
7-Hydroxy-2-isopropyl-3-pyridin-3-ylmethyl-3H-quinazolin-4-one, (Example 144)
7-Hydroxy-2-isopropyl-3-pyrrolidin-1-ylmethyl-3H-quinazolin-4-one, (Example 145)
7-Hydroxy-2-isopropyl-3-(1-phenyl-ethyl)-3H-quinazolin-4-one, (Example 146)
7-Hydroxy-2-isopropyl-3-[2-(3-methyl-3H-imidazol-4-yl)-ethyl]-3H-quinazolin-4-one, (Example 147)
7-Hydroxy-2-isopropyl-3-(2-oxo-2-phenyl-ethyl)-3H-quinazolin-4-one, (Example 148)
1-[2-(7-Hydroxy-2-isopropyl-4-oxo-4H-quinazolin-3-yl)-ethyl]-pyrrole-2,5-dione, (Example 149)
3-(2-Chloro-benzyl)-7-hydroxy-2-isopropyl-3H-quinazolin-4-one, (Example 150)
3-Benzo[1,3]dioxol-4-ylmethyl-7-hydroxy-2-isopropyl-3H-quinazolin-4-one, (Example 151)
7-Hydroxy-2-isopropyl-3-naphthalen-1-ylmethyl-3H-quinazolin-4-one (Example 152)
3-(4-tert-Butyl-benzyl)-7-hydroxy-2-isopropyl-3H-quinazolin-4-one, (Example 153)
3-Benzothiazol-2-ylmethyl-7-hydroxy-2-isopropyl-3H-quinazolin-4-one, (Example 154)
3-Cyclopropylmethyl-7-hydroxy-2-isopropyl-3H-quinazolin-4-one, (Example 155)
3-Cyclobutylmethyl-7-hydroxy-2-isopropyl-3H-quinazolin-4-one, (Example 156)
7-Hydroxy-2-isopropyl-3-isoxazol-3-yl-3H-quinazolin-4-one, (Example 157)
3-Cyclopentyl-7-hydroxy-2-isopropyl-3H-quinazolin-4-one, (Example 158)
7-Hydroxy-2-isoproryl-3-pyridin-4-yl-3H-quinazolin-4-one, (Example 159)
7-Hydroxy-2-isopropyl-3-pyridin-2-yl-3H-quinazolin-4-one, (Example 160)
7-Hydroxy-2-isopropyl-3-pyrazin-2-yl-3H-quinazolin-4-one, (Example 161)
7-Hydroxy-2-isopropyl-3-(3-methyl-isoxazol-5-yl)-3H-quinazolin-4-one, (Example 162)
3-Cyclohexyl-7-hydroxy-2-isopropyl-3H-quinazolin-4-one, (Example 163)
7-Hydroxy-2-isopropyl-3-(4-methoxy-phenyl)-3H-quinazolin-4-one, (Example 164)
7-Hydroxy-2-isopropyl-3-(3-methoxy-phenyl)-3H-quinazolin-4-one, (Example 165)
7-Hydroxy-2-isopropyl-3-(2-methoxy-phenyl)-3H-quinazolin-4-one, (Example 166)
7-Hydroxy-2-isopropyl-3-(5-methyl-pyrazin-2-ylmethyl)-3H-quinazolin-4-one, (Example 167)
3-Benzo[1,3]dioxol-5-yl-7-hydroxy-2-isopropyl-3H-quinazolin-4-one, (Example 168)
7-Hydroxy-3-(3-hydroxy-naphthalen-2-yl)-2-isopropyl-3H-quinazolin-4-one, (Example 169)
7-Hydroxy-2-isopropyl-3-naphthalen-1-yl-3H-quinazolin-4-one, (Example 170)
7-Hydroxy-2-isopropyl-3-isoquinolin-1-yl-3H-quinazolin-4-one, (Example 171)
7-Hydroxy-2-isopropyl-3-quinolin-8-yl-3H-quinazolin-4-one, (Example 172)
3-Benzothiazol-6-yl-7-hydroxy-2-isopropyl-3H-quinazolin-4-one, (Example 173)
4-(7-Hydroxy-2-isopropyl-4-oxo-4H-quinazolin-3-yl)-benzoic acid methyl ester, (Example 174)
2-(7-Hydroxy-2-isopropyl-4-oxo-4H-quinazolin-3-yl)-thiophene-3-carboxylic acid methyl ester, (Example 175) and
2-(7-Hydroxy-2-isopropyl-4-oxo-4H-quinazolin-3-yl)-cyclopentanecarboxylic acid ethyl ester, (Example 176)
can be prepared analogously to 3-(4-chloro-3-fluoro-phenyl)-7-hydroxy-2-isopropyl-3H-quinazolin-4-one (Example 4) by replacing 4-chloro-3-fluoro-phenylamine with the appropriate aniline. Some of the compounds may also be prepared analogously to 3-(4-chloro-phenyl)-7-hydroxy-2-isopropyl-3H-quinazolin-4-one (Example 3) by replacing 4-chloro-phenylamine (step 1 b) with the appropriate aniline. Those which are not commercially available are described in the preparation of the intermediates section.

## Claims

1. A quinazolinone compound of the formula 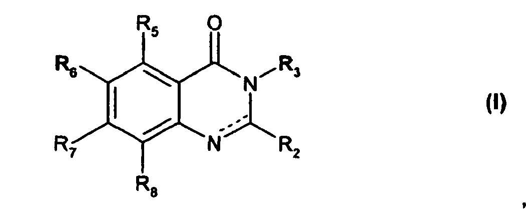 wherein
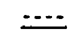 is a single bond or a double bond;
R₂ is selected from
(a) C₁-C₈alkyl, C₃-C₆cycloalkyl, (C₁-C₆alkyl)amino or di-(C₁-C₆alkyl)amino;
or
(b) NH₂, hydroxyC₁-C₆alkylamino-, aminoC₁-C₆alkylamino, C₂-C₆alkenyl, di(trifluoromethyl)C₁-C₆alkyl, R₉-O-(C₁-C₆alkyl)- in which the alkyl chain is optionally substituted by trifluoromethyl, (NC)-C₁-C₆alkyl-, (R₁₀R₁₁N-)C₁-C₆alkyl-, (C₁-C₆alkyl)-SO₂-(C₁-C₆alkyl)-, wherein R₉, R₁₀ and R₁₁ are each independently H or C₁-C₆ alkyl; phenyl optionally substituted by one, two or three substituents each independently selected from the group consisting of halogen, C₁-C₆alkyl, halogen-substituted C₁-C₆alkyl, hydroxy C₁-C₆alkyl, cyano or a group -(C=O)-R₂ₐ, where R₂ₐ is C₁-C₆alkyl; or 5, 6, or 7-membered, saturated or unsaturated, heterocyclic ring, directly attached to the quinazolinone ring or attached through -C₁-C₆ alkyl-, containing one, two, or three heteroatoms selected from N, O and S, and optionally substituted with one, two or three substitutents selected from C₁-C₆alkyl, C₁-C₆alkoxy, hydroxy, cyano, halo, R₁₀R₁₁N-, R₉-O-(C=O)-, -(C=O)-N-R₁₀R₁₁, =O and phenyl ;
R₃ is selected from
(a'):
phenyl substituted by one, two or three substituents each independently selected
from the group consisting of halogen, C₁-C₆alkyl, halogen-substituted C₁-C₆alkyl, hydroxyC₁-C₆alkyl, cyano or a group -C(=O)-R₃ₐ, where R₃ₐ is C₁-C₆alkyl; or
(b'):
C₁-C₆alkyl, (NC)-C₁-C₆alkyl-, R₉-O-(C₁-C₆alkyl)-, R₉-O-(C₁-C₆alkyl)-O-(C₁-C₆alkyl)-,R₁₀R₁₁N-(C₁-C₆alkyl)-, R₁₀R₁₁N-(C=O)-(C₁-C₆alkyl)-, or (C₁-C₆alkyl)-SO₂-(C₁-C₆alkyl)-, wherein R₉, R₁₀ and R₁₁ are each independently H or C₁-C₆ alkyl; or
unsubstituted phenyl, phenyl substituted with one or two substituents selected from -(C₁-C₆alkoxy)-, R₁₀R₁₁N-, R₁₀R₁₁N-(C₁-C₆alkyl)-, -SO₂-(C₁-C₆alkyl), R₉-O-(C=O)-, wherein R₉, R₁₀ and R₁₁ are as defined above, or with halo-substituted phenyl or a 5- or 6-membered saturated or unsaturated heterocyclic ring having one, two or three heteroatoms selected from N, O and S and optionally including a further substituent selected from halo, or phenyl substituted with three or four substituents selected from halo, hydroxyl, and C₁-C₆alkyl; or
a cycloalkyl ring having 3, 4, 5 or 6 carbon atoms, directly attached to the quinazolinone ring or attached through -C₁-C₆alkyl-, and which is optionally substituted with one or two substituents selected from C₁-C₆alkyl, C₁-C₆alkoxy, hydroxy, cyano, halo, R₁₀R₁₁N-, R₉-O-(C=O)-, -(C=O)-N-R₁₀R₁₁, and phenyl; or
benzyl, or phenyl(C₁-C₆alkyl)-, phenoxy-(C₁-C₆alkyl)- or phenyl(C=O)-(C₁-C₆alkyl)-, optionally substituted with one, two, or three substituents selected from C₁-C₆alkyl, C₁-C₆alkoxy, hydroxy, cyano, halo, R₁₀R₁₁N-, R₉-O-(C=O)-, -(C=O)-N-R₁₀R₁₁, and phenyl; or
a 5, 6, or 7- membered, saturated or unsaturated, heterocyclic ring, directly attached to the quinazolinone ring or attached through -C₁-C₆ alkyl-, containing one, two, or three heteroatoms selected from N, O and S, and optionally substituted with one, two or three substitutents selected from C₁-C₆alkyl, C₁-C₆alkoxy, hydroxy, cyano, halo, R₁₀R₁₁N-, R₉-O-(C=O)-, -(C=O)-N-R₁₀R₁₁, =O and phenyl; or
a 9- or 10- membered aromatic or heterocyclic fused ring, directly attached to the quinazolinone ring or attached through -C₁-C₆ alkyl-, containing zero, one, two or three heteroatoms selected from N, O and S, and optionally substituted with one, two, three or four substitutents selected from C₁-C₆alkyl, C₁-C₆alkoxy, hydroxy, cyano, halo, R₁₀R₁₁N-, R₉-O-(C=O)-, -(C=O)-N-R₁₀R₁₁, and phenyl;
R₇ is hydroxy, esterified hydroxy, etherified hydroxy, amino, (C₁-C₆alkyl)amino, a group 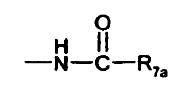 or a group 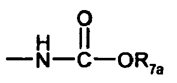 ,where R₇ₐ is C₁-C₆alkyl or halogen-substituted C₁-C₆alkyl, or a group 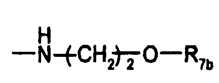 where R_{7b} is benzyl or phenylethyl; and
R₅, R₆ and R₈ are each independently hydrogen, halogen, C₁-C₆alkyl, C₂-C₆alkenyl, C₂-C₆alkynyl, hydroxy, hydroxy-substituted C₁-C₆alkyl, C₁-C₆alkoxy, C₃-C₆cycloalkyl, cyano, -C(=O)H, phenyl, (C₃-C₆cycloalkyl)C₁-C₆alkyl, (C₃-C₆cycloalkyl)C₁-C₆alkoxy, (C₁-C₆alkoxycarbonylamino)C₁-C₆alkoxy or (C₁-C₆alkylcarbonylamino)C₁-C₆alkoxy, (amino) C₁-C₆alkoxy, (dimethylamino)C₁-C₆alkoxy, or (C₁-C₆alkoxycarbonyl) C₁-C₆alkoxy, and R₈ is further suitably hydroxy-substituted (C₃-C₆cycloalkyl)C₁-C₆alkyl, hydroxy-substituted phenylC₁-C₆alkyl, hydroxy-substituted heteroarylC₁-C₆alkyl, C₁-C₆alkylcarbonyl, C₁-C₆alkoxyC₁-C₆alkoxy or heteroarylC₁-C₆alkyl,
in free form or in salt form , provided that, in formula (I), when R₂ is selected from group (a), R₃ is selected from group (b') and when R₃ is selected from group (b), R₃ is selected from group (a') and excluding the compounds in which R₇ is hydroxyl and R₅, R₆ and R₈ are each independently hydrogen and R₂ is isopropyl and R₃ is pyridin-5-yl substituted in the 2-position by Cl or CN.

2. A compound according to claim 1 where R₂ is isopropyl, ethyl, t-butyl, hydroxyisopropyl, dimethylamino or 2-isopropenyl.

3. A compound according to any one of claims 1-2 where R₃ is phenyl, pyridyl or pyrimidyl, where each ring is substituted by one or two halo, trifluoromethyl, C₁-C₆alkyl, C₁-C₆alkoxy, C₁-C₆alkoxyC₁-C₆alkyl, C₁-C₆hydroxyalkyl,C₁-C₆alkylcarbonyl, cyano or hydroxyl, or R₃ is indazolyl or 1-oxo-indan-5-yl,

4. A compound according to any one of claims 1 to 3 where R₅ and R₆ are hydrogen.

5. A compound according to any one of claims 1 to 4 where R₇ is hydroxyl or amino.

6. A compound according to any one of claims 1 to 5 where R₈ is hydrogen or hydroxy-substituted C₁-C₆alkyl.

7. The use of a compound of the formula (I) as defined in any one of claims 1 to 6, for the manufacture of a medicament for the treatment or prevention of a disease or condition, in which vanilloid receptor activation plays a role or is implicated.

8. A method for treating or preventing a disease or condition, in which vanilloid receptor activation plays a role or is implicated, comprising administering to a mammal in need thereof a therapeutically effective amount of a quinazolinone compound of the formula(I) as defined in any one of claims 1 to 6.

9. A pharmaceutical composition comprising a compound as defined in any one of claims 1 to 6 of the formula I, in free form or in pharmaceutically acceptable salt form, in association with a pharmaceutical carrier or diluent.

10. A process for the manufacture of a compound of formula (II) 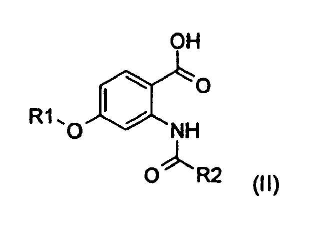 where R¹ is H or a suitable protecting group and R₂ is selected from
(a) C₁-C₈alkyl, C₃-Cycloalkyl, (C₁-C₆alkyl)amino or di-(C₁-C₆alkyl)amino; or
(b) NH₂, hydroxyC₁-C₆alkylamino-, aminoC₁-C₆alkylamino, C₂-C₆alkenyl, di(trifluoromethyl)C₁-C₆alkyl, R₉-O-(C₁-C₆alkyl)- in which the alkyl chain is optionally substituted by trifluoromethyl, (NC)-C₁-C₆alkyl-, (R₁₀R₁₁N-)C₁-C₆alkyl-, (C₁-C₆alkyl)-SO₂-(C₁-C₆alkyl)-, wherein R₉, R₁₀ and R₁₁ are each independently H or C₁-C₆ alkyl; phenyl optionally substituted by one, two or three substituents each independently selected from the group consisting of halogen, C₁-C₆alkyl, halogen-substituted C₁-C₆alkyl, hydroxy C₁-C₆alkyl, cyano or a group -(C=O)-R₂ₐ, where R₂ₐ is C₁-C₆alkyl; or 5, 6, or 7-membered, saturated or unsaturated, heterocyclic ring, directly attached to the quinazolinone ring or attached through -C₁-C₆ alkyl-, containing one, two, or three heteroatoms selected from N, O and S, and optionally substituted with one, two or three substitutents selected from C₁-C₆alkyl, C₁-C₆alkoxy, hydroxy, cyano, halo, R₁₀R₁₁N-, R₉-O-(C=O)-, -(C=O)-N-R₁₀R₁₁, =O and phenyl ;
from a compound of formula (III) 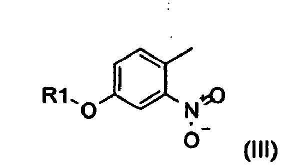 by one of the following sequential steps:
a) oxidation using a suitable oxidizing agent, reduction using a suitable reducing agent and acylation with a suitable acylating agent; or
b) reduction using a suitable reducing agent, acylation with a suitable acylating agent and oxidation using a suitable oxidizing agent; or
c) conversion of the methyl group to a dialkylaminovinyl group using a suitable agent, oxidation using a suitable oxidizing agent, reduction using a suitable reducing agent and acylation with a suitable acylating agent; or
d) reduction using a suitable reducing agent, acylation with a suitable acylating agent, conversion of the methyl group to a dialkylaminovinyl group using a suitable agent, and oxidation using a suitable oxidizing agent, followed by optional deprotection of the protecting group under standard conditions.
